# EUROPEAN PATENT SPECIFICATION

(11) **EP 1 436 267 B1**
(45) Date of publication and mention of the grant of the patent: **27.12.2006**
(21) Application number: 02801693.9
(22) Date of filing: 15.10.2002
(51) Int. Cl.: C07D 257/12, C07D 239/91, C07D 239/96, C07D 495/04, C07D 209/48, A61K 31/517, A61P 29/00

(54) **LACTAM DERIVATIVES AS ANTAGONISTS FOR HUMAN 11CBY RECEPTORS**
LACTAMDERIVATEN ZUR VERWENDUNG ALS HUMANE 11CBY REZEPTORANTAGONISTEN
DERIVES DE LACTAM COMME ANTAGONISTES DE RECEPTEURS 11CBY HUMAINS

(30) Priority: 15.10.2001 GB 0124627
(43) Date of publication of application: 14.07.2004
(73) Proprietor: SMITHKLINE BEECHAM PLC, Brentford, Middlesex TW8 9GS (GB)
(72) Inventor: ARMSTRONG, Sula, Anne, Royal Society of Chemistry, Cambridge, Cambridgeshire TW9 9EP (GB); HAMPRECHT, Dieter, Wolfgang, GlaxoSmithKline, 37135 Verona (IT); JONES, Martin, GlaxoSmithKline, Harlow, Essex CM 19 5AW (GB); WITTY, David, Richard, GlaxoSmithKline, Harlow, Essex CM 19 5AW (GB); AL-BARAZANJI, Kamal, A., GlaxoSmithKline, Research Triangle Park, NC 27709 (US); TADAYYON, Mohammad, St. Andrew Street, Hertford, SG14 1HQ (GB)
(74) Representative: Learoyd, Stephanie Anne
(86) International application number: PCT/US2002/032740
(87) International publication number: WO 2003/033480

(56) References cited:
- SAXENA S ET AL: "ANTI-INFLAMMATORY QUINAZOLINONES" INDIAN JOURNAL OF PHARMACEUTICAL SCIENCES, XX, XX, vol. 53, no. 2, March 1991 (1991-03), pages 48-52, XP008012951 ISSN: 0250-474X
- DATABASE CAPLUS [Online] CHEMICAL ABSTRACTS SERVICE, COLUMBUS, OHIO, US; Database accession no. 1991:122049 XP002233183 -& PL 137 227 B (UNIWERSYTET LÓDZKI, LÓDZ (POLSKA)) 31 March 1987 (1987-03-31)

## Description

This invention relates to novel lactam derivatives which are antagonists at the human 11CBy receptors, to processes for their preparation, to pharmaceutical compositions containing them and to their uses in therapy.

WO01/21577 (Takeda) relates to a compound of the formula 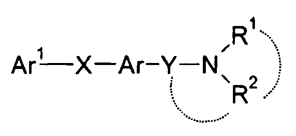 wherein Ar¹ is a cyclic group which may have substituents, X is a spacer having a main chain of 1 to 6 atoms, Y is a bond or a spacer having a main chain of 1 to 6 atoms, Ar is a monocyclic aromatic ring which may be condensed with a 4 to 8 membered non-aromatic ring, and may have further substituents; R¹ and R² are independently hydrogen or a hydrocarbon group which may have substituents; R¹ and R² together with the adjacent nitrogen atom may form a nitrogen containing hetero ring which may have substituents; R² may form a spiro ring together with Ar; or R² together with the adjacent nitrogen atom may form a nitrogen containing hetero ring which may have substituents; or a salt thereof; and which compounds are antagonists of a melanin concentrating hormone. Such compounds are suggested as being useful for preventing or treating obesity.

We have now found a novel group of compounds that exhibit a useful profile of activity as antagonists of the human 11CBy receptor (also referred to as MCHR1) disclosed in Nature 400, 261-265 (1999).

### SUMMARY OF THE INVENTION

The invention thus provides compounds of formula (I) 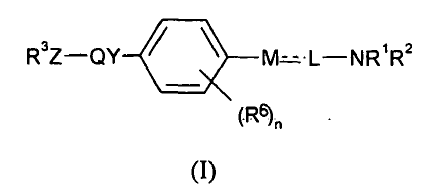 a salt or solvate thereof , wherein
M is O or CH₂;
L is a 2 or 3 membered alkylene chain which may be linked to the phenyl ring of formula (I) via an R⁶ substituent located in the ortho position relative to the group M to form a bicyclic structure ;
wherein together M-L may be optionally substituted by at least one moiety selected from the group consisting of methyl, ethyl, hydroxy and C₁₋₃ alkoxy;
(i) R¹ and R² are each independently selected from the group consisting of hydrogen,
   C₁₋₆ straight or branched alkyl which may be optionally substituted by phenyl, and C₃₋₆ cycloalkyl optionally substituted by one or more C₁₋₆ alkyl groups;
or (ii) R¹ and R² together with the nitrogen atom to which they are bonded form a 4-8 membered heterocyclic ring or a 7-10 membered bicyclic heterocyclic ring containing 1, 2, or 3 heteroatoms selected from N, O, and S, wherein said 4 to 8 membered heterocyclic ring and said 7 to 10 membered bicyclic heterocyclic ring are optionally substituted with a substituent selected from the group consisting of phenyl and from one to four C₁₋₃ alkyl;
each R⁶ is the same or different and is independently selected from the group consisting of hydroxy, C₁₋₂alkyl, C₁₋₃alkoxy, halo, C₂₋₃alkenyl, benzyl, and -C(R³)NOR^{b}, wherein R^{a} and R^{b} are each independently selected from the group consisting of hydrogen, methyl, methoxymethyl, methoxymethoxyl, and methoxyethoxyl; and n is 1, 2, 3, or 4;
QY is a bicyclic fused heterocyclic ring wherein Q and Y are each one ring of said bicyclic fused heterocyclic group, wherein said Y ring contains from 1 to 3 nitrogens and is bound to the phenyl ring of formula (I) via a nitrogen atom, and said Q ring is a 5 or 6 membered aryl or heterocyclic ring having a group ZR³; Z is bound to the Q ring;
Z is selected from the group consisting of a direct bond, NH, NCH₃, O, S or CH₂; and
R³ is a group selected from the group consisting of aryl, alk-2-en-1-yl, cycloalkyl and cycloalk-2-en-1-yl and wherein said aryl, alk-2-en-1-yl, cycloalkyl, and cycloalk-2-en-1-yl are optionally substituted from 1 to 3 times with a subsittuent selected from the group consisting of C₁₋₃ alkyl, halo, amino, alkylamino, dialkylamino, hydroxy, C₁₋₃ alkoxy, cyano, trifluoromethyl, and methylthio groups.

The invention also includes embodiments to (i) a pharmaceutical formulation comprising a compound of formula (I), (ii) the use of a compound of formula (I) or a pharmaceutical formulation comprising the formula (I) in therapy, and (iii) processes for the preparation of a compound of formula (I).

### DETAILED DESCRIPTION OF THE INVENTION

In the present invention, unless otherwise stated, references to salts, include both physiologically acceptable salts and non-physiologically acceptable salts of compounds of formula (I). The compounds of formula (I) and salts thereof may form solvates (e.g. hydrates) and the invention includes all such solvates.

As used herein, "a compound of the invention" or a compound of formula (I) means a compound of formula (I) or a pharmaceutically acceptable salt, or solvate thereof.

As used herein, the term "optionally" means that the subsequently described event(s) may or may not occur, and includes both event(s) that occur and events that do not occur.

The term "alkyl" as a group or part of a group (e.g., alkoxy, alkylamino, etc.) refers to a straight or branched alkyl group. Said alkyl contains from 1 to 6 carbon atoms unless otherwise specified. Examples of such C₁₋₆ alkyl groups include methyl, ethyl, propyl, isopropyl, butyl, isobutyl, t-butyl, pentyl, neopentyl or hexyl.

The term "alk-2-en-1-yl" refers to a straight or branched C₃₋₆ alk-2-en-1-yl group.

The term "cycloalkyl" refers to non-aromatic monocyclic carbocyclic rings having 3 to 7 carbon atoms which may be substituted by a moiety selected from the group consisting of hydroxy, C₁₋₃ alkoxy, and halo.

The term "halo" or "halogen" refers to the elements fluorine, chlorine, bromine, and iodine, unless otherwise specified.

The term "cycloalk-2-en-1-yl" refers to a C₅₋₇ cycloalk-2-en-1-yl group.

The term "aryl" preferably refers to phenyl, but also includes fused aryl rings such as naphthyl.

The terms "heterocycle" and "heterocyclic" refer to a ring system composed of C and at least one other atom selected from the group consisting of N, O, and S. Heterocycles may or may not be heteroaromatic as defined below. In other words, heteroaromatics are heterocycles, but all heterocycles are not heteroaromatic.

The term "heteroaryl" and "heteroaromatic" refer to a monocyclic or bicylic aromatic ring system composed of C and at least one other atom selected from the group consisting of N, O, and S.

The terms "members" (and variants thereof, e.g., "membered") in the context of heterocyclic and heteroaryl groups refers to the total atoms, carbon and heteroatoms (N, O, and/or S) which form the ring. Thus, an example of a 6-membered heterocyclic ring is piperidine and an example of a 6-membered heteroaryl ring is pyridine.

In formula (I), M is O or CH₂. Most preferably, M is O.

L of formula (I) is a 2 to 3 membered alkylene chain (that is, (CH₂)₂ or (CH₂)₃). Preferaby, L is (CH₂)₂. When the group L is linked to the phenyl ring of formula (I) via a R⁶ substituent, said R⁶ is located in the ortho position to the group M. Examples of the resultant bicyclic structure include 1,4-benzodioxan; benzopyran; 1,2-dihydrobezopyran; 1,2,3,4-tetrahydronaphthalene; and 1,2-dihydronaphthalene.

In formula (I), in (i) R¹ and R² are each independently selected from the group consisting of hydrogen, C₁₋₆ straight or branched alkyl which may be optionally substituted by phenyl, and C₃₋₆ cycloalkyl optionally substituted by one to four C₁₋₆ alkyl groups. When R¹ and R² independently represent C₁₋₆ alkyl, then preferred examples of such R¹ and R² groups include methyl and isopropyl.

Alternatively, in (ii) R¹ and R² together with the nitrogen atom to which they are bonded form a 4-8 membered heterocyclic ring or a 7-10 membered bicyclic heterocyclic ring (containing 1, 2, or 3 heteroatoms selected from N, O and S), wherein said 4 to 8 membered heterocyclic ring and said 7 to 10 membered bicyclic heterocyclic ring are optionally substituted with a substituent selected from the group consisting of phenyl and from one to four C₁₋₃ alkyl.

When R₁ and R₂ together with the nitrogen atom to which they are bonded or attached form a 4-8 membered heterocyclic ring, examples of such rings include azetendinyl, pyrrolidinyl or piperidinyl, which rings may be substituted by 1-4 methyl groups or a phenyl group (e.g. pyrrolidinyl, 2-methylpyrrolidinyl, 2,5-dimethyl-pyrrolidinyl, piperidyl, 2-methylpiperidinyl, 2,6-dimethylpiperidinyl, 2,2,6,6, tetramethylpiperidinyl or 4-phenylpiperidinyl).

When R¹ and R² together with the nitrogen atom form a bridged heterocyclic ring, examples of such groups include 7-aza-bicyclo [2.2.1] hept-7-yl or 2-aza-bicyclic [2.2.2] oct-8-yl.

Preferably, the group NR¹R² is a tertiary amino group; more preferably a 4-8 membered heterocyclic ring which may be substituted by 1-4 methyl groups; or an optionally substituted 5- or 6- membered heterocycle. In the most preferred embodiment of the invention, NR¹R² is a pyrrolidinyl group.

Each R⁶ is the same or different and is independently selected from the group consisting of hydroxy, C₁₋₂alkyl, C₁₋₃alkoxy, halo, C₂₋₃alkenyl, benzyl, and -C(R^{a})NOR^{b}, wherein R^{a} and R^{b} are each independently selected from the group consisting of hydrogen, methyl, methoxymethyl, methoxymethoxy, and methoxyethoxy. Typically, R⁶ is a substituent of the phenyl ring of formula (I) that is preferably located in the ortho position relative to the M substituent. Preferred examples of R⁶ include hydroxy, methoxy, methoxymethoxy, methoxyethoxy, and methoxymethyl. Most preferably, R⁶ is methoxyl. In the formula, n is 1, 2, 3, or 4.

QY is a bicyclic fused heterocyclic ring wherein Q and Y are each one ring of said bicyclic fused heterocyclic group, wherein said Y ring contains from 1 to 3 nitrogens and is bound to the phenyl ring of formula (I) through a nitrogen, and said Q ring is a 5 or 6 membered aryl or heteroaryl having a group ZR³ bound to the Q ring.

The fused bicyclic heterocyclic group QY is preferably a 5,5; 5,6; 6,5; or 6,6 bicyclic heterocyclic system.

In QYgroup, Q is conveniently an aromatic ring such as benzene, thiophene, furan or pyridine. Y, in QY, is linked via a nitrogen atom therein to the rest of the molecule in formula (I) and optionally contains a further 1 or 2 nitrogen atoms. Preferably, the ring also contains a carbonyl group adjacent to the linking nitrogen atom.

When Y is a 5- membered ring, it contains either two carbonyl groups or a carbonyl and a methylene group, in addition to the nitrogen atom. When Y is a 5-membered ring, then preferably Q is a benzene ring, and more preferably QY is an isoindole-1,3-dione.

When Y is a 6- membered ring, it preferably contains a carbonyl group and either two additional nitrogen atoms or a single additional nitrogen and a moiety selected from the group consisting of carbonyl, thiocarbonyl, and methylidene. Said moiety can be optionally substituted by a member selected from the group consisting of methyl, methoxy ,methylthio, and S(O)CH₃. When Y is a 6- membered ring, Q is preferably a benzene or thiophene ring.

Examples of suitable QY groups include isoindole-1,3-dione, 2,3-dihydro-isoindole-1-one, 3-H-benzo[d](1,2,3)triazin-4-one, 3-H-quinazolin-4-one, 1-H-quinazoline-2,4-dione, 3-H-thieno[2,3-d]-1-triazine-4-one, 3-H-thieno[2,3d]pyrimidin-4-one, 3-H-thieno[3,2-d]pyrimidin-4-one, 2-thioxo-2,3-dihydro-1-H-thieno[3,2-d]pyrimidine-4-one, 2-methylsulphanyl-3H-thieno[3,2-d] pyrimidin-4-one.

Z is selected from the group consisting of a direct bond, NH, OCH₃, O, S, and CH₂. Preferably, examples of suitable groups Z include a bond, oxygen, sulphur, CH₂ or NH. Most preferably, the group Z is a bond or an oxygen atom.

R³ is a group selected from the group consisting of aryl, alk-2-en-1-yl, cycloalkyl and cycloalk-2-en-1-yl and wherein said aryl, alk-2-en-1-yl, cycloalkyl, and cycloalk-2-en-1-yl are optionally substituted from 1 to 3 times with a substituent selected from the group consisting of C₁₋₃ alkyl, halo, amino, alkylamino, dialkylamino, hydroxy, C₁₋₃ alkoxy, cyano, trifluoromethyl, and methylthio groups. Examples of suitable R³ groups include phenyl optionally substituted by one or two groups selected from halogen (e.g. fluorine, chlorine or bromine), hydroxy, C₁₋₃ alkoxy (e.g. methoxy), C₁₋₄ alkyl (e.g., methyl, ethyl, t-butyl, and haloalkyls such as trifluoromethyl), and cyano. Also, R³ can be C₅₋₇ cycloalkyl (e.g., cyclohexyl), C₅₋₇ cycloalk-2-en-1-yl (e.g., cyclohex-2-en-1-yl or cyclopent-2-en-1-yl), or C₃₋₆ alk-2-en-1-yl (e.g., 3-methyl-prop-2-en-1-yl or 2-methyl-prop-2-en-yl). Preferably, the group R³ is phenyl or a substituted phenyl group; and most preferably R³ is phenyl.

Examples of suitable preferred compounds according to the invention include those described in the Examples herein.

Specifically, most preferred compounds of the invention include: 3-[3-methoxy-4-(2-pyrrolidin-1-yl-ethyl)-phenyl]-7-phenyl-3H-benzo[d] [1,2,3]triazin-4-one; 3-[3-methoxy-4-(2-pyrrolidin-1-yl-ethoxy)-phenyl]-6-phenyl-3H-thieno[3,2-d]pyrimidin-4-one; and physiologically acceptable acid addition salts thereof.

The compounds of formula (I) form acid addition salts and can be dosed as such. For use in medicine acid addition salts of a compound of formula (I) will be those formed with physiologically acceptable inorganic and organic acids. Examples of such suitable acids include hydrochloric, hydrobromic, phosphoric, acetic, ascorbic, benzoic, citric, fumaric, lactic, maleic, mandelic, methanesulphonic, salicylic, succinic, and tartaric.

Other acid addition salts of the compounds of formula (I) may however be useful in the preparation and/or isolation of a compound of formula (I) or a physiologically acceptable salt thereof and are contemplated as being part of the invention herein.

Compounds of the present invention are antagonists of a human 11CBy receptor disclosed in Nature, 400, 261-265 (1999). Accordingly, these compounds are believed to have a role in the treatment (preventing, prophylaxis, ameliorating, or correcting) of dysfunctions or diseases, including, but not limited to, infections such as bacterial, fungal, protozoan and viral infections, particularly infection caused by HIV-1 or HIV-2; pain; cancers; diabetes; obesity; feeding abnormalities, such as anorexia and bulimia; drinking abnormalities (too much or too little liquid consumption); asthma; Parkinson's disease; both acute and congestive heart failure; hypertension; urinary retention; osteoporosis; angina pectoris; myocardial infarction; ulcers; allergies; benign prostatic hypertrophy; psychotic and neurological disorders, including anxiety, schizophrenia, depression (major), manic depression (bi-polar depression), delirium, dementia, and/or severe mental retardation; and dyskinesias (such as Huntington's disease or Gilles de la Tourette's syndrome, among others), hereinafter all of the above conditions (diseases, dysfunctions, abnormalities, etc.) are referred to collectively herein as "the Disorders". Preferably, this aspect of the invention provides for the treatment (including prophylaxis) of one or more of the Disorders selected from diabetes, major depression, manic depression (bipolar disorder), anxiety, schizophrenia and sleep disorders. Preferred among these disorders are obesity, diabetes (especially diabetes mellitus), major depression, and anxiety.

It has been reported that over-expression of MCH gene in mouse is associated with obesity and insulin resistance (Ludwig DS et al. Melanin-concentrating hormone overexpression in transgenic mice leads to obesity and insulin resistance. J Clin Invest 2001 107(3): 379-386). In vitro data revealed that MCH stimulates insulin secretion in both CRI-G1 and RINm5F cell-lines (Tadayyon M et al. Expression of melanin-concentrating hormone receptors in insulin-producing cells: MCH stimulates insulin release in RINm5F and CRI-G1 cell-lines. BBRC 2000 275: 709-712).

In this patent we also disclose that surprisingly MCH contributes to blood glucose regulation by increasing plasma glucagon levels in conscious instrumented rats. In addition, we claim the use of 11CBy antagonists to inhibit MCH-induced hyperglycemia by reducing MCH-induced hyperglucagonemia. Thus, in addition to other important impications of 11CBy antagonists in obesity and neurological indications, such antagonists will have therapeutic implication for the treatment of diabetes mellitus.

The administration of such compounds to a mammal may be by way of oral (including sub-lingual), parenteral, nasal, rectal or transdermal administration. An amount effective to treat the Disorders herein before described depends on the usual factors such as the nature and severity of the disorders being treated and the weight of the mammal. However, a unit dose will normally contain 1 to 1000 mg, suitably 1 to 500 mg, for example an amount in the range of from 2 to 400 mg such as 2, 5, 10, 20, 30, 40, 50, 100, 200, 300 and 400 mg of the active compound. Unit doses will normally be administered once or more than once per day, for example 1, 2, 3, 4, 5 or 6 times a day, more usually 1 to 4 times a day, such that the total daily dose is normally in the range, for a 70 kg adult of 1 to 1000 mg, for example 1 to 500 mg, that is in the range of approximately 0.01 to 15 mg/kg/day, more usually 0.1 to 6 mg/kg/day, for example 1 to 6 mg/kg/day.

It is greatly preferred that compounds of formula (I) and or physiologically acceptable salts or solvates thereof are administered in the form of a unit-dose composition, such as a unit dose oral (including sub-lingual), nasal, rectal, topical or parenteral (especially intravenous) composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well-known methods in the art. Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats; emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

Oral formulations also include conventional sustained release formulations, such as tablets or granules having an enteric coating.

For parenteral administration, fluid unit dose forms are prepared containing the compound and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and is sterilised by exposure to ethylene oxide before suspending in the sterile vehicle. Advantageously, a surfactant or wetting agent is included in the composition to facilitate uniform distribution of the compound of the invention.

As is common practice, the compositions will usually be accompanied by written or printed directions for use in the medical treatment concerned.

Compounds of the present invention may be employed alone or in conjunction with other compounds, such as therapeutic compounds.

No adverse toxicological effects are expected for the compounds of the invention, when administered in accordance with the invention.

Accordingly, in a further aspect, the present invention provides a pharmaceutical composition for use in the treatment and/or prophylaxis of one or more of the Disorders which comprises a compound of this invention, or a physiologically acceptable salt or solvate thereof, and a pharmaceutically acceptable carrier.

In a further aspect the invention provides the use of a compound of this invention, or a physiologically acceptable salt or solvate thereof, for the manufacture of a medicament for the treatment and/or prophylaxis of one or more of the Disorders. In a still further aspect the invention provides a compound of formula (I), or a physiologically acceptable salt or solvate, thereof as a therapeutic agent, in particular for the treatment and/or prophylaxis of one or more of the Disorders.

A further aspect of the invention provides processes for the preparation of compounds of formula (I). Unless stated otherwise, R¹, R², R³, R⁶, Z, Q, Y, M, and L are as defined in formula (I).

Thus compounds of formula (I) wherein Y is a 5 membered ring containing two carbonyl groups may be prepared by reaction of an aniline of formula (II) 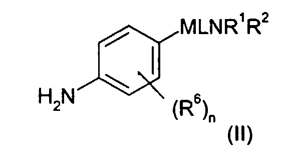 wherein R⁶, n, M , L, R¹ and R² have the meanings defined in formula (I) or are protected derivatives thereof with appropriate anhydride (III), orthodicarboxylic acid (IV) or imide (V) set forth below 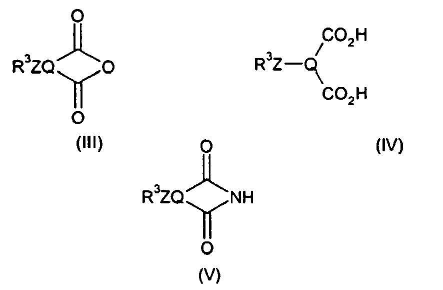 wherein R³, Z and Q have the meanings defined in formula (I), under conventional conditions for the formation of the required imide group Y.

The reaction is carried out using a coupling agent, e.g., dicyclohexylcarbodiimide, conveniently on a resin support, in an appropriate solvent such as a halohydrocarbon, e.g., dichloromethane or an amide such as N, N-dimethylformamide or a mixture thereof and optionally in the presence of a tertiary organic base such as 4-dimethylaminopyridine or diethylisopropylamine, which is also conveniently on a resin support.

Compounds of formula (I) where Y is a 5-membered ring containing a single carbonyl group may be prepared by reduction of the corresponding compound of formula (I) wherein Y is a 5 membered ring containing 2 carbonyl groups, using a conventional two step reduction process. For example, reduction with a suitable hydride such as a borohydride followed by treatment with a hydrogen donor such as triethylsilane in the presence of a suitable acid, e.g., trifluoroacetic acid.

Compounds of formula (I) wherein Y is a 6-membered heterocycle may be prepared by cyclisation of the compound of formula (VI) 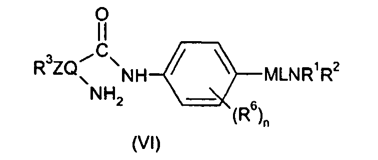 wherein R³ Z, Q, R⁶, n, M, L, R¹ and R² have the meanings defined in formula (I). Thus compounds of formula (I) wherein Y is a pyrimidin-4-one moiety may be prepared by heating a compound of formula (VI) with formic acid or an appropriate orthoester e.g. triethylorthoformate in a suitable solvent such as a halohydrocarbon e.g. dichloroethane followed by treatment with a strong base such as 1, 8-diazabicyclo [5.4.0] undec-7-one (DBU).

Compounds of formula (I) wherein Y is a pyrimidine-2,4-dione may be prepared by treating the amine (VI) with a carboxylating agent such as carbonyldiimidazole and a strong base such as DBU in a suitable aprotic solvent such as dichloroethane.

Compounds of formula (I) wherein Y is a 1,2,3-triazin-4-one may be prepared by diazotisation of the compound of formula (VI) followed by treatment with a strong base, e.g. DBU. Thus, the reaction may be carried out by reacting a solution of amine (VI) in a solvent such as dichloromethane with an alkyl nitrite, e.g., butyl or amyl nitrite, in the presence of a suitable organic carboxylic acid, e.g., trifluoroacetic acid followed by addition of the strong base.

Compounds of formula (I) wherein Y is a pyrimidine ring containing a thiocarbonyl group or an alkylthio substituent at the 2 position may be prepared from the thiourea (VII) 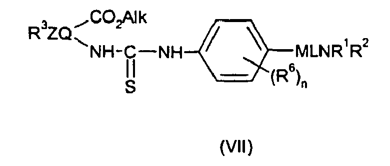 wherein R³, Z, Q, R⁶, n, M, L, R¹ and R² have the meanings defined in formula (I) or are protected derivatives thereof and Alk is C₁₋₄alkyl. Thus, compounds of formula (I) wherein Y contains a 2-thiocarbonyl group may be prepared by heating the thiourea (VII) in a suitable high boiling solvent such as an aromatic hydrocarbon, e.g., toluene.

Compounds of formula (I) wherein Y is a pyrimidinone having an alkylthio group at the 2 position may be prepared by reaction of compound (VII) with the appropriate alkyl halide in an aprotic polar solvent such as DMF and in the presence of a suitable base such as a tertiary amine or an alkali or alkaline metal earth carbonate, e.g., sodium carbonate or potassium carbonate.

Compounds of formula (I) wherein Y is a pyridmidinone derivative, unsubstituted at the 2-position may be prepared from the corresponding pyrimidinone having an alkylthio group at the 2-position by reaction with Raney nickel in a solvent such as an alkanol, e.g., ethanol.

Compounds of formula (I) may also be prepared by reaction of a compound of formula (VIII) 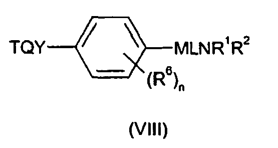 wherein Q, Y, R⁶, n, M, L, R¹ and R² have the meanings defined in formula (I) or a group convertible thereto and T is a leaving or displacable group, e.g., halogen, with a compound capable of introducing the group R³Z.

Thus, compounds of formula (I) wherein Z is a bond and R³ is an aryl group may be prepared from the halide of formula (VIII) with the appropriate arylboronic acid using a Suzuki coupling reaction or with the appropriate aryl-tin reagent using a Stille displacement reaction.

Compounds of formula (I) wherein Z is a methylene group may be prepared by treating a compound of formula (VIII) with an appropriate organo zinc or organo magnesium compound in the presence of a palladium catalyst. The reaction is carried out in an aprotic solvent such as an ether (e.g., THF) and the organo zinc or organo magnesium compound is one capable of introducing the group R³CH₂.

Compounds of formula (I) wherein Z is NH or NCH₃ may be prepared by reacting the halide of formula (VIII) with the appropriate amine R³NH₂ or R³NHCH₃ in the presence of a palladium catalyst under standard Buchwald conditions.

Compounds of formula (I) wherein Z is sulphur may be prepared by reaction of a compound of formula (VIII) with the thiol R³SH in the presence of a suitable base such as an alkali metal hydride in an aprotic solvent such as an ether, e.g., THF. Similarly, compounds of formula (I) wherein Z is oxygen may be prepared by the reaction of the compound of formula (VIII) with the compound R³OH in the presence of an alkali metal hydride and an aprotic solvent, e.g., DMF.

The anilines of formula (II) are either known compounds or may be prepared by analogous methods described for preparing the known compounds. Thus, compounds of formula (II) wherein L and (R⁶)ₙ are to form a cyclic group as defined in formula (I) may be prepared using the procedures described in WO 01/2577 A2.

Compounds of formula (II) wherein M is O or S may be prepared by reaction of the nitrochlorobenzene (X) 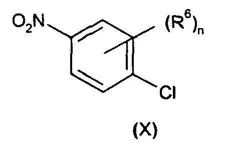 wherein R⁶ and n has the meanings defined in formula (I) or is a group convertible thereto, with the compound (XI)

HMLNR¹R² (XI)

wherein M is O or S and L, R¹ and R² have the meaning defined in formula (I) or R¹ and R² are protected derivatives thereof, in the presence of a strong base such as sodium hydride in a polar aprotic solvent such as DMF, followed by reduction of the nitro group to amino using conventional means such as hydrogen and a palladium catalyst or iron and ammonium chloride.

Compounds of formula (II) wherein M is CH₂ may be prepared by reacting an activated derivative of the acid (XII) 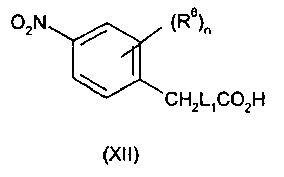 wherein R⁶ and n have the meanings as defined in formula (I) and L₁ is a 1 or 2 membered alkylene chain with the amine HNR¹R² wherein R¹ and R² have the meanings defined in formula (I) followed by reduction of the resultant amide with borane in an aprotic solvent such as tetrahydrofuran and then reduction of the nitro group using conventional procedures as documented above.

Alternatively compounds of formula (I) may be prepared by alkylation of the amino R¹R²NH wherein R¹ and R² have the meaning defined in formula (I) or are protected derivatives thereof by reaction with a compound of formula (XIII) 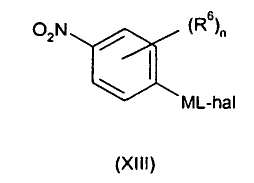 wherein R⁶, n, M and L have the meanings defined in formula (I) and hal is a halogen in the presence of a suitable base and a polar solvent such as DMF, and subsequent generation of the required primary amino group by reaction of the corresponding nitro group using a conventional procedure.

The compounds of formula (X), (XI), (XII) and (XIII) are either known compounds or may be prepared by analogous methods to those used to prepare the known compounds.

The anhydride (III) is either a known compound or may be prepared by analogous methods to those described for preparing known compounds. Thus the compounds may be prepared from the anhydride (IX) 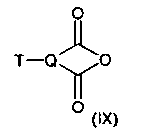 wherein Q has the meaning in formula (I) and T is a leaving group, using the same reaction conditions to introduce the group R³Z as described above for the preparation of compounds of formula (I) from the compounds of formula (VIII).

The ortho dicarboxylic acid (IV) and the imide (IV) may also be prepared in an analogous manner to that for preparing the anhydride (III) from the compound of formula (IX).

Compounds of formula (VI) may be prepared by the process outlined below wherein

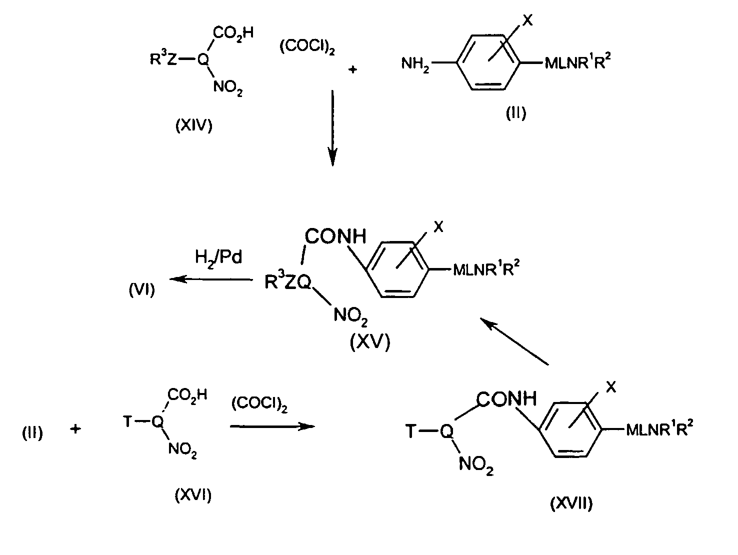

In the above formulae the groups M, L, R¹, R², R³, R⁶, n, Z and Q have the meaning defined in formula (I) and T is a displaceable group as defined in formula (VIII).

The condensation of an activated derivative, e.g., acid chloride of the acids (XIV) or (XVI) with the amine (II) to give amides (XV) and (XVII) may be carried out under conventional conditions for preparing amides from anilines and a carboxylic acid.

Compounds of formula (XVII) may be converted into the required compound (XV) using the conditions described above for converting a compound of formula (VIII) into a compound of formula (I).

The carboxylic acid (XIV) may be prepared from the corresponding carboxylic acid (XVI) or a protected derivative thereof by the conditions previously described above for introducing the group R³Z by displacement of the group T.

Alternatively, compounds of formula (VI) may be prepared by the process outlined below wherein R¹, R², R³, R⁶, Z, Q, M, and L are as defined in formula (I).

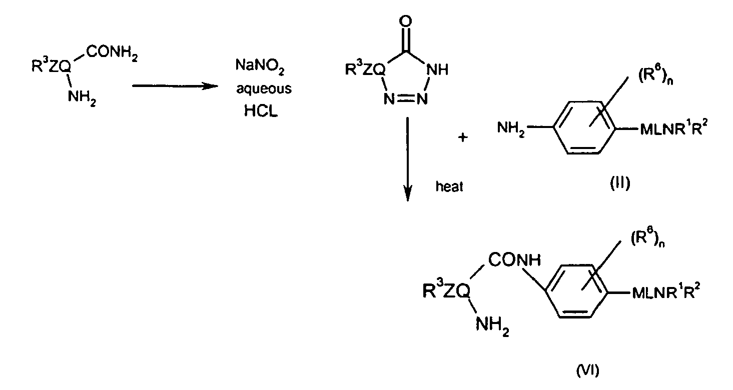

Compounds of formula (VII) may be prepared by the process as outlined below wherein R¹, R², R³, R⁶, Z, Q, M, and L are as defined in formula (I), and Alk is C₁₋₄alkyl.

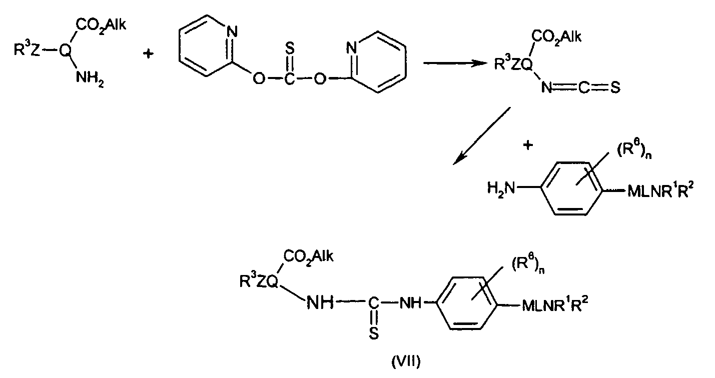

Compounds of formula (VIII) may be prepared from the aniline (II) and the anhydride (IX) or carboxylic acid (XVI) using the general processes described above for preparing the corresponding compounds of formula (I) from the aniline (II).

The acids of formula (XIV) are either known compounds or can be prepared by analogous processes to those used for preparing the known compounds. Alternatively, they may be prepared by the process outlined below wherein wherein R³, Z, and Q are as defined in formula (I). 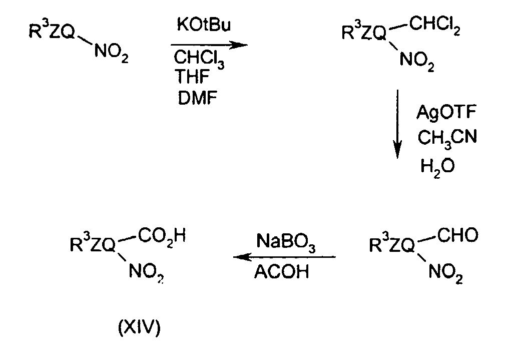 In the above processes, it will be appreciated that it may be necessary to carry out the reactions wherein one or more of the groups R³, Z, Q, X, M, L, R⁶, R¹ or R² may need to be present in a protected form and then the protecting group(s) removed. The need for such protection will be well understood by those skilled in the art and such protection is clearly within the scope of the present invention.

Acid addition salts of compounds of formula (I) may be prepared in a conventional manner by treating the compound of formula (I) with the appropriate inorganic or organic acid. For example, by addition of the acid, optionally as a solution in an organic solvent to a solution of the free base in a suitable solvent.

Compounds for use in this invention and their preparation are illustrated in the following Examples and Tables.

These Examples illustrate general procedures and sources of chemicals utilised to prepare compounds whose structures are shown in the Tables of data which follow the Examples. In the case of Examples prepared as members of a coupled array, the synthetic origin of all starting componants of the array are shown in the Examples. Rather than detailing the experimental procedure for each case, the method by which individual members of the array were prepared is indicated in a Table by reference to a related Example. Mass spectral characterisation of all Examples is provided in the tables of data. Additional characterisation is provided for selected representative Examples with full experimental procedures.

### Example A1

### 2-[4-(2-Diisopropylamino-ethoxy)-3-methoxy-phenyl]-5-phenyl-isoindole-1,3-dione

4-Bromophthalic anhydride [Apin] (2.27 g, 10mmol) was dissolved in 1,2-dichloromethane (8ml), and pyridine (10mmol, 0.81ml) was added, followed by 4-(2-diisopropylamino-ethoxy)-3-methoxy-phenylamine **[Patent WO-9901127]** (2.44 g, 10mmol) and a catalytic amount of 4-dimethylaminopyridine [Aldrich]. The mixture was heated for 16 h at 85°C. The mixture was evaporated *in vacuo* and applied to a Biotage Flash40M cartridge, (eluting with dichloromethane - methanol - aqueous ammonia) to give the 5-bromo-2-[4-(2-diisopropylamino-ethoxy)-3-methoxy-phenyl]-isoindole-1,3-dione 3.05g, 68%, as the trifluoroacetate salt;
¹H NMR (CDCl₃): δ 1.06 (12H, bd), 2.92 (2H, bt), 3.05 (2H, bm), 3.88 (3H, s), 3.99 (2H, bt), 6.89-7.03 (3H, m), 7.78 (1H, d), 7.91 (1H, dd) 8.06 (1H, d); *m*/*z* [AP+] 475, 477 (M+H⁺, 100%).
A solution of this material (331mg, 0.7mmol) in benzene (46ml), ethanol (9ml) and aqueous sodium carbonate (2M, 9ml) was degassed and treated with phenyl boronic acid (85mg, 0.7mmol) and tetrakis(triphenylphosphine) palladium[0] (20mg). The mixture was brought to reflux (80°C) for 16 hours then cooled and filtered through filter-aid, washing with dichloromethane. The filtrate was evaporated and the residue subjected to flash chromatography on silica gel (eluting with methanol - dichloromethane - aqueous ammonia) to obtain the title compound;
¹H NMR (CDCl₃): δ 1.06 (12H, d), 2.94 (2H, t), 3.07 (2H, m), 3.89 (3H, s), 4.00 (2H, t), 6.95-7.00 (3H, m), 7.35-7.52 (3H, m), 7.67 (2H, dd), 7.99 (2H, s), 8.15 (1H, s), which crystallised on treatment with 1M HCl in ether to a white solid.

### Example A2

### 5-Benzyl-2-[4-(2-diisopropylamino-ethoxy)-3-methoxy-phenyl]-isoindole-1,3-dione

A mechanically agitated piece of zinc foil [Aldrich] (99.99%, 0.1 mm thick, 0.5g), in dry THF under argon was treated with trimethylsilyl chloride (20ul), then after ten minutes, 1,2-dibromoethane (80ul). The mixture was stirred for 20 minutes then a solution of benzyl bromide (5mmol, 0.59ml) and THF (4.4ml) introduced. The mixture was stirred at 0°C for 2h then warmed to RT for 1h. A 2.6ml portion of the resulting solution was added to a mixture of 5-bromo-2-[4-(2-diisopropylamino-ethoxy)-3-methoxy-phenyl]-isoindole-1,3-dione **[Example A1],** tetrakis(triphenylphosphine) palladium[0] (5mol%, 120mg), and THF (3ml). The mixture was heated at 60°C for 16h. The resulting solution was evaporated and partially purified by flash chromatography (dichloromethane - methanol - aqueous ammonia) on silica gel before being further purified by reverse phase chromatography (aq. MeCN / TFA) to give the title compound as the trifluoroacetate salt;
¹H NMR (CDCl₃): δ 1.45 (12H, m), 3.52 (2H, t), 3.84 (2H, s), 3.85 (3H, sm), 4.15 (2H, m), 4.74 (2H, t), 5.80 (1H, bs), 6.92-7.04 (3H, m), 7.19-7.35 (5H, m), 7.60 (1H, dd), 7.75 (1H, s), 7.83 (1H, d).

### Example A3

### 2-[4-(2-Diisopropylamino-ethoxy)-3-methoxy-phenyl]-5-phenylamino-isoindole-1,3-dione

A mixture of palladium II acetate (7.5mg, 0.04mmol), 2-(dicyclohexylphosphino)biphenyl [Digital] (0.06mmol, 21mg) and cesium carbonate (205mg, 0.63mmol) in 1,4-dioxane (5ml) was sonicated under argon for 40 minutes. To this suspension was added a mixture of 5-bromo-2-[4-(2-diisopropylamino-ethoxy)-3-methoxy-phenyl]-isoindole-1,3-dione **[Example A1]** (200mg, 0.42mmol) and aniline (0.42mmol, 39mg) in 1,4-dioxane (1ml). The mixture was heated at 85°C for 16h then the cooled mixture filtered through filter-aid and the filtrate evaporated. The residue was subjected to purification by preparative reverse phase chromatography (aq. MeCN, TFA) to give the title compound as the trifluoroacetate salt;
¹H NMR (CDCl₃): δ 1.46 (12H, m), 3.51 (2H, t), 3.80-3.87 (2H, m), 3.86 (3H, s), 4.50 (2H, t), 6.4 (1H, bs), 6.94-7.03 (3H, m), 7.17-7.24 (4H, m), 7.38-7.44 (3H, m), 7.75 (1H, d).

### Example A4

### 2-[3-Methoxy-4-(2-pyrrolidin-1-yl-ethoxy)-phenyl]-5-phenoxy-isoindole-1,3-dione hydrochloride

To a solution of the 1-(2-hydroxyethyl)-pyrrolidine [Aldrich], (1.87ml, 16mmol) in dimethylformamide was added portionwise sodium hydride [60% dispersion in oil, (544mg, 16mmol). After stirring at room temperature for 10 minutes a solution of 1-chloro-2-methoxy-4-nitro-benzene [Avocado] (3g, 16mmol) in dimethylformamide (10ml) was added dropwise. The reaction mixture was left stirring at room temperature for 16hrs then concentrated. The residue was dissolved in ethyl acetate (200ml) and washed with water (3 x 50ml). The organic phase was dried with magnesium sulphate, evaporated and the residue purified by flash chromatography on silica gel using dichloromethane - aq. ammonia - methanol as eluent to afford 1-[2-(2-methoxy-4-nitro-phenoxy)-ethyl]-pyrrolidine as a brown oil.
¹H NMR (CDCl₃): δ1.82 (4H, m), 2.65 (4H, m), 3.01 (2H, t), 3.94 (3H, s), 4.24 (2H, t), 6.92 (1H, d), 7.74 (1H, d), and 7.89(1H, dd); MS (AP +ve): *m*/*z* 267 [M+H]⁺. To a solution of 1-[2-(2-methoxy-4-nitro-phenoxy)-ethyl]-pyrrolidine (2.3g, 8.6mmol) in ethanol (100ml) was added 10% Pd/C (50mg). The mixture was stirred at room temperature under an atmosphere of hydrogen at atmospheric pressure for 16h, then filtered through celite and the filtrate concentrated to give the corresponding aniline; 3-methoxy-4-(2-pyrrolidin-1-yl-ethoxy)-phenylamine, as a brown solid.
¹H NMR (CDCl₃): δ 1.80 (4H, m), 2.62 (4H, m), 2.89 (2H, t), 3.5 (2H, brs), 3.80 (3H, s), 4.06 (2H, t), 6.20 (1H, dd), 6.29 (1H, d) and 6.75 (1H, d); MS (AP +ve): ***m*/*z*** 237 [M+H]⁺.
4-Phenoxy-phthalic acid [J. Org. Chem. (1977), 42(21), 3425-31] (893mg, 3.5mmol) was dissolved in DMF (30ml) and polymer bound *N*-cyclohexylcarbodiimide [Argonaut Technologies](1.69 mol. eq./g, 4.1 g) was added, followed by a solution of 3-methoxy-4-(2-pyrrolidin-1-yl-ethoxy)-phenylamine (817mg, 3.5mmol) in DMF (5ml) and a catalytic amount of DMAP. The mixture was stirred for 16 h at ambient temperature. The resin was filtered off over filter-aid, washing with dichloromethane. The filtrate was evaporated *in vacuo* and purified by chromatography using a Biotage Flash40M cartridge (eluting with dichloromethane - methanol - aqueous ammonia). The resulting yellow oil was dissolved in CHCl₃ and treated with ethereal HCl (1 M, 2ml) to give a yellow solid. Recrystallisation from methanol/diethyl ether gave the title compound as an off-white solid (328mg, 0.7mmol);
¹H NMR (CDCl₃): δ 12.82 (1H, br), 7.88 (1H, dd), 7.45-7.27 (5H, m), 7.13-6.94 (5H, m), 4.57 (2H, t), 3.91 (2H, br), 3.86 (3H, s), 3.50 (2H, br), 3.07 (2H, br), 2.26-2.07 (4H, m, br).

### Example A5

### 2-[3-Methoxy-4-(2-pyrrolidin-1-yl-ethoxy)-phenyl]-5-phenylamino-isoindole-1,3-dione

3-Methoxy-4-(2-pyrrolidin-1-yl-ethoxy)-phenylamine [**Example A4**] was used in the same manner as 4-(2-diisopropylamino-ethoxy)-3-methoxy-phenylamine in the procedure of **Example A1** to form 5-bromo-2-[3-methoxy-4-(2-pyrrolidin-1-yl-ethoxy)-phenyl]-isoindole-1,3-dione;
¹H NMR (CDCl₃): δ 2.05 (4H, m), 3.15 (4H, bs), 3.32 (2H, t), 3.87 (3H, s), 4.43 (2H, t), 6.92-7.06 (3H, m), 7.80 (1H, d), 7.92 (1H, dd), 8.07 (1H, d).
This material was used in the procedure of **Example A3** in place of 5-bromo-2-[4-(2-diisopropylamino-ethoxy)-3-methoxy-phenyl]-isoindole-1,3-dione to afford the title compound as a trifluoroacetate salt;
¹H NMR (CDCl₃): δ 2.12 (4H, bd), 2.5 (1H, bs), 3.08 (2H, bm), 3.55 (2H, m), 3.85 (3H, s), 3.88 (2H, m), 4.12 (2H, t), 6.96 (3H, m), 7.17-7.26 (4H, m), 7.39-7.44 (3H, m), 7.73 (1H, d) 12.5 (1H, bs).

### Example A6

### 5-Benzyl-2-[3-methoxy-4-(2-pyrrolidin-1-yl-ethoxy)-phenyl]-isoindole-1,3-dione 5-Bromo-2-[3-methoxy-4-(2-pyrrolidin-1-yl-ethoxy)-phenyl]-isoindole-1,3-dione

**[Example A5]** was used in the same manner as 5-bromo-2-[4-(2-diisopropylamino-ethoxy)-3-methoxy-phenyl]-isoindole-1,3-dione in the procedure of **Example A2** to afford the title compound as the trifluoroacetate salt;
¹H NMR (CDCl₃): δ 2.14 (4H, bm), 3.10 (2H, m), 3.59 (2H, bt), 3.84 (3H, s), 3.91 (2H, m), 4.15 (2H, d), 4.43 (2H, t), 6.2 (1H, bs), 6.94-6.99 (3H, m), 7.18-7.74 (7H, m), 7.85 (1H, d).

### Example A7

### 2-[3-Methoxy-4-(2-pyrrolidin-1-yl-ethoxy)-phenyl]-5-(phenylthio)-isoindole-1,3-dione

4-Fluorophthalic anhydride [Acros] (0.84g, 5mmol) was dissolved in dichloromethane (30ml) and polymer bound N-cyclohexylcarbodiimide [Argonaut Technologies](1.69 mol. eq./g, 3g) was added, followed by a solution of 3-methoxy-4-(2-pyrrolidin-1-yl-ethoxy)-phenylamine **[Example A4]** (1.18g, 5.0mmol) and a catalytic amount of 4-dimethylaminopyridine. The mixture was stirred for 16 h at ambient temperature. The resin was filtered off over filter-aid, and washed with dichloromethane. The filtrate was evaporated *in vacuo* and purified by chromatography using a Biotage Flash40M cartridge, eluting with dichloromethane - methanol to give the 5-fluoro-2-[3-methoxy-4-(2-pyrrolidin-1-yl-ethoxy)-phenyl]-isoindole-1,3-dione;
¹H NMR (CDCl₃): δ 2.12 (4H, m), 3.32 (4H, bs), 3.44 (2H, t), 3.86 (3H, s), 4.48 (2H, t), 6.93-7.03 (3H, m), 7.45 (1H, dt), 7.60 (1H, dd), 7.90 (1H, dd); *m*/*z* [AP+] 458 (M+H⁺, 100%).
A portion of this material (96mg, 0.25mmol) was added to a stirred mixture of thiophenol (55mg, 0.5mmol) and sodium hydride (60% oil dispersion, 16mg / 0.4mmol) in DMF (2ml) and the mixture warmed to 50°C. The solution was cooled, evaporated and the residue purified by reverse phase chromatography (acetonitrile - water - TFA) to give the title compound as the trifluoroacetate salt, an off white solid; ¹H NMR (CDCl₃): δ 1.85 (4H, m), 2.74 (4H, m), 3.02 (2H, t), 3.85 (3H, s), 4.22 (2H, t), 6.89- 6.97 (3H, m), 7.44-7.60 (7H, m), 7.76 (1H, d).

### Example A8

### 5-[(4-chlorophenyl)thio]-2-[3-methoxy-4-(2-pyrrolidin-1-ylethoxy)phenyl]-1H-isoindole-1,3(2H)-dione

Using the method of **Example A7** but utilising 4-chlorothiophenol [Aldrich] in place of thiophenol to give the title compound as the trifluoroacetate salt.

### Example A9

### 5-[(3-methoxyphenyl)thio]-2-[3-methoxy-4-(2-pyrrolidin-1-ylethoxy)phenyl]-1H-isoindole-1,3(2H)-dione

Using the method of **Example A7** but utilising 3-methoxythiophenol [Aldrich] in place of thiophenol to give the title compound as the trifluoroacetate salt.

### Example A10

### 5-[(4-hydroxypheayl)thio]-2-[3-methoxy-4-(2-pyrrolidin-1-ylethoxy)phenyl]-1H-isoindole-1,3(2H)-dione

Using the method of **Example A7** but utilising 4-hydroxythiophenol [Aldrich] in place of thiophenol to give the title compound as the trifluoroacetate salt.

### Example A11

### 5-[(4-fluorophenyl)thio]-2-[3-methoxy-4-(2-pyrrolidin-1-ylethoxy)phenyl]-1H-isoindole-1,3(2H)-dione

Using the method of **Example A7** but utilising 4-fluorothiophenol [Aldrich] in place of thiophenol to give the title compound as the trifluoroacetate salt.

### Example A12

### 5-[(4-aminophenyl)thio]-2-[3-methoxy-4-(2-pyrrolidin-1-ylethoxy)phenyl]-1H-isoindole-1,3(2H)-dione

Using the method of **Example A7** but utilising 4-aminothiophenol [Aldrich] in place of thiophenol to give the title compound as the trifluoroacetate salt.

### Example A13

### 2-[3-methoxy-4-(2-pyrrolidin-1-ylethoxy)phenyl]-5-{[4-(trifluoromethyl)phenyl]thio}-1H-isoindole-1,3(2H)-dione

Using the method of **Example A7** but utilising 4-trifluoromethylthiophenol [Aldrich] in place of thiophenol to give the title compound as the trifluoroacetate salt.

### Example A14

### 5-[(4-methoxyphenyl)thio]-2-[3-methoxy-4-(2-pyrrolidin-1-ylethoxy)phenyl]-1H-isoindole-1,3(2H)-dione

Using the method of **Example A7** but utilising 4-methoxythiophenol [Aldrich] in place of thiophenol to give the title compound as the trifluoroacetate salt.

### Example A15

### 5-(4-Chloro-phenoxy)-2-[3-methoxy-4-(2-pyrrolidin-1-yl-ethoxy)-phenyl]-isoindole-1,3-dione

4-Fluorophthalic anhydride (42mg, 0.25mmol) in DMF (0.5ml) was treated with 4-chlorophenol (32mg, 0.25mmol) and sodium hydride (60% oil disp. 10mg, 0.25mmol). The mixture was heated to 140°C for 15 minutes then cooled, dichloromethane (5ml) added, 3-methoxy-4-(2-pyrrolidin-1-yl-ethoxy)-phenylamine **[Example A4]** (59mg, 0.25mmol) introduced, polymer bound N-cyclohexylcarbodiimide (1.69 mol. eq./g, 0.5g) added, polymer bound diethylisopropylamine resin [Argonaut Technologies] (3.49 mol. eq./g, 0.2g) added and the mixture stirred at RT for 16hours. The solution was filtered through filter-aid, washing with dichloromethane, the combined organic phases evaporated and purified by reverse phase chromatography (acetonitrile - water- TFA) to give the title compound as its trifluoroacetate salt;
¹H NMR (CDCl₃): δ 2.1 (2H, bs), 2.25 (2H, bs), 3.05 (2H, bs), 3.55 (2H, bs), 3.83 (3H, s), 3.89 (2H, bs), 4.55 (2H, bs) 6.97- 7.19 (5H, m), 7.39 (1H, d), 7.47 (1H, s), 7.70 (2H, d), 7.93 (1H, d), 12.7 (1H, bs).

### Example A16

### 5-(4-fluorophenoxy)-2-[3-methoxy-4-(2-pyrrolidin-1-ylethoxy)phenyl]-1H-isoindole-1,3(2H)-dione

5-Nitro-2-phenylisoindole-1,3-dione (5.9 g, 22mmol) was dissolved in DMF (40ml) and sodium 4-fluorophenolate (3.1 g, 23.1mmol) was added. The resulting mixture was stirred at 100 °C for 2 hours under an argon atmosphere. The mixture was allowed to cool to RT then poured into 4 % aqueous HCl. The yellow precipitate which formed was filtered off, washed with water and recrystallised (DCM/TBME) to yield (4-fluorophenoxy)-2-phenylisoindol-1,3-dione (2.4 g, 41 %);
MS [APCI⁺] 334 (100%, M+H⁺).
This phthalimide (1.4 g, 4.2mmol), ethyleneglycol (20 mL) and 25 % aqueous NaOH (4 mL) were heated at 140 °C for 16hours. The resulting mixture was poured into 1M aqueous HCl to give an orange precipitate. This was washed with 0.1 M aqueous HCl and water then dried to afford 4-(4-fluorophenoxy)phthalic acid in 65 % yield;
*m*/*z* [APCI⁻] 275 (20%, M-H⁻), 231 (100%).
Using the procedure of **Example A4** but utilising 4-(4-fluorophenoxy)phthalic acid in place of 4-phenoxyphthalic acid gave the title compound which crystallised from ethereal hydrochloric acid to give a white solid.
Also by the method of **Example A15** but using 4-fluorophenol in place of 4-chlorophenol to give the title compound as the trifluoroacetate salt.

### Example A17

### 5-(4-Trifluoromethyl-phenoxy)-2-[3-methoxy-4-(2-pyrrolidin-1-yl-ethoxy)-phenyl]-isoindole-1,3-dione

Using the method of **Example A15** but utilising 4-trifluoromethylphenol [Aldrich] in place of 4-chlorophenol to give the title compound as the trifluoroacetate salt.

### Example A18

### 5-(4-Fluoromethyl-phenoxy)-2-[3-methoxy-4-(2-pyrrolidin-1-yl-ethoxy)-phenyl]-isoindole-1,3-dione

Using the method of **Example A15** but utilising 4-fluoro-3-chlorophenol [Aldrich] in place of 4-chlorophenol to give the title compound as the trifluoroacetate salt.

### Example A19

### 5-(3-Fluoro-phenoxy)-2-[3-methoxy-4-(2-pyrrolidin-1-yl-ethoxy)-phenyl]-isoindole-1,3-dione

Using the method of **Example A15** but utilising 3-fluorophenol [Aldrich] in place of 4-chlorophenol to give the title compound as the trifluoroacetate salt.

### Example A20

### 5-(3-Chloro-phenoxy)-2-[3-methoxy-4-(2-pyrrolidin-1-yl-ethoxy)-phenyl]-isoindole-1,3-dione

Using the method of **Example A15** but utilising 3-chlorophenol [Aldrich] in place of 4-chlorophenol to give the title compound as the trifluoroacetate salt.

### Example A21

### 5-(3-tert-Butyl-phenoxy)-2-[3-methoxy-4-(2-pyrrolidin-1-yl-ethoxy)-phenyl]-isoindole-1,3-dione

Using the method of **Example A15** but utilising 3-*tert*-butylphenol [Aldrich] in place of 4-chlorophenol to give the title compound as the trifluoroacetate salt.

### Example A22

### 5-(3-Methoxy-phenoxy)-2-[3-methoxy-4-(2-pyrrolidin-1-yl-ethoxy)-phenyl]-isoindole-1,3-dione

Using the method of **Example A15** but utilising 3-methoxyphenol [Aldrich] in place of 4-chlorophenol to give the title compound as the trifluoroacetate salt.

### Example A23

### 5-(3-Methyl-phenoxy)-2-[3-methoxy-4-(2-pyrrolidin-1-yl-ethoxy)-phenyl]-isoindole-1,3-dione

Using the method of **Example A15** but utilising 3-methylphenol [Aldrich] in place of 4-chlorophenol to give the title compound as the trifluoroacetate salt.

### Example A24

### 5-(4-Methyl-phenoxy)-2-[3-methoxy-4-(2-pyrrolidin-1-yl-ethoxy)-phenyl]-isoindole-1,3-dione

Using the method of **Example A15** but utilising 4-methylphenol [Aldrich] in place of 4-chlorophenol to give the title compound as the trifluoroacetate salt.

### Example A25

### 5-(4-Cyano-phenoxy)-2-[3-methoxy-4-(2-pyrrolidin-1-yl-ethoxy)-phenyl]-isoindole-1,3-dione

Using the method of **Example A15** but utilising 4-cyanophenol [Aldrich] in place of 4-chlorophenol to give the title compound as the trifluoroacetate salt.

### Example A26

### 5-(2-Cyano-phenoxy)-2-[3-methoxy-4-(2-pyrrolidin-1-yl-ethoxy)-phenyl]-isoindole-1,3-dione

Using the method of **Example A15** but utilising 2-cyanophenol [Aldrich] in place of 4-chlorophenol to give the title compound as the trifluoroacetate salt.

### Example A27

### 2-[3-methoxy-4-(2-pyrrolidin-1-ylethoxy)phenyl]-5-[2-(methylthio)phenoxy]-1H-isoindole-1,3(2H)-dione

Using the method of **Example A15** but utilising 2-(methylthio)phenol [Aldrich] in place of 4-chlorophenol to give the title compound as the trifluoroacetate salt.

### Example A28

### 5-(2-Bromo-phenoxy)-2-[3-methoxy-4-(2-pyrrolidin-1-yl-ethoxy)-phenyl]-isoindole-1,3-dione

Using the method of **Example A15** but utilising 2-bromo-phenol [Aldrich] in place of 4-chlorophenol to give the title compound as the trifluoroacetate salt.

### Example A29

### 5-(2-Chloro-phenoxy)-2-[3-methoxy-4-(2-pyrrolidin-1-yl-ethoxy)-phenyl]-isoindole-1,3-dione

Using the method of **Example A15** but utilising 2-chloro-phenol [Aldrich] in place of 4-chlorophenol to give the title compound as the trifluoroacetate salt.

### Example A30

### 5-(2-Fluoro-phenoxy)-2-[3-methoxy-4-(2-pyrrolidin-1-yl-ethoxy)-phenyl]-isoindole-1,3-dione

Using the method of **Example A15** but utilising 2-fluoro-phenol [Aldrich] in place of 4-chlorophenol to give the title compound as the trifluoroacetate salt.

### Example A31

### 5-(2-Ethyl-phenoxy)-2-[3-methoxy-4-(2-pyrrolidin-1-yl-ethoxy)-phenyl]-isoindole-1,3-dione

Using the method of **Example A15** but utilising 2-ethyl-phenol [Aldrich] in place of 4-chlorophenol to give the title compound as the trifluoroacetate salt.

### Example A32

### 5-(2-,5-Dinuorophenoxy)-2-[3-methoxy-4-(2-pyrrolidin-1-yl-etboxy)-phenyl]-isoindole-1,3-dione

Using the method of **Example A15** but utilising 2,5-difluorophenol [Aldrich] in place of 4-chlorophenol to give the title compound as the trifluoroacetate salt.

### Example A33

### 5-(2-Tritluoromethylphenoxy)-2-[3-methoxy-4-(2-pyrrolidin-1-yl-ethoxy)-phenyl]-isoindole-1,3-dione

Using the method of **Example A15** but utilising 2-trifluoromethylphenol [Aldrich] in place of 4-chlorophenol to give the title compound as the trifluoroacetate salt.

### Example A34

### 5-(2,4-Difluorophenoxy)-2-[3-methoxy-4-(2-pyrrolidin-1-yl-ethoxy)-phenyl]-isoindole-1,3-dione

Using the method of **Example A15** but utilising 2,4-difluorophenol [Aldrich] in place of 4-chlorophenol to give the title compound as the trifluoroacetate salt.

### Example A35

### 5-(4-Fluoro-2-methoxyphenoxy)-2-[3-methoxy-4-(2-pyrrolidin-1-yl-ethoxy)-phenyl]-isoindole-1,3-dione

Using the method of **Example A15** but utilising 4-fluoro-2-methoxyphenol [Aldrich] in place of 4-chlorophenol to give the title compound as the trifluoroacetate salt.

### Example A36

### 5-(2-Methoxyphenoxy)-2-[3-methoxy-4-(2-pyrrolidin-1-yl-ethoxy)-phenyl]-isoindole-1,3-dione

Using the method of **Example A15** but utilising 2-methoxyphenol [Aldrich] in place of 4-chlorophenol to give the title compound as the trifluoroacetate salt.

### Example A37

### 5-(Cyclohex-2-enyloxy)-2-[3-methoxy-4-(2-pyrrolidin-1-ylethoxy)phenyl]isoindol-1,3-dione

Using the procedure of **Example A39** but utilising racemic 2-cyclohexenol [Aldrich] in place of 3-methyl-2-butenol to give first 4-(cyclohex-2-enyloxy)-phthalic acid dimethyl ester;
¹H NMR (CDCl₃): δ 1.60-2.20 (m, 6H), 3.86 (s, 3H), 3.90 (s, 3H), 4.85-4.90 (m, 1H), 5.81-5.85 (m, 1H), 5.98-6.02 (m, 1H), 6.99 (d, *J* 8.8, 1H), 7.07 (s, 1H), 7.79 (d, *J* 8.8, 1H).
This was progressed in the same manner as 4-(3-methylbut-2-enyloxy)phthalic acid dimethyl ester in the same example to give the title compound as the hydrochloride salt, a racemate.

### Example A38

### 2-[3-Methoxy-4-(2-pyrrolidin-1-ylethoxy)phenyl]-5-(pent-2-enyloxy)isoindol-1,3-dione

Using the procedure of **Example A39** but utilising a 2.7:1 mixture of Z and E-pent-2-enol [Aldrich] in place of 3-methyl-2-butenol to give the title compound as a 2.7:1 mixture of Z and E isomers as their hydrochloride salts

### Example A39

### 2-[3-Methoxy-4-(2-pyrrolidin-1-ylethoxy)phenyl]-5-(3-methylbut-2-enyloxy)isoindol-1,3-dione

3-Methyl-2-butenol [Aldrich] was used in place of allyl alcohol in the procedure described in *Angew. Chem.,* **1992,** *104,* 198-200, to prepare 4-allyloxyphthalic acid dimethyl ester. This gave 4-(3-methylbut-2-enyloxy)phthalic acid dimethyl ester; ¹H NMR (CDCl₃): δ 1.74 (s, 3H), 1.80 (s, 3H), 3.86 (s, 3H), 3.91 (s, 3H), 4.56 (d, *J* 6.7, 2H), 5.44-5.48 (m, 1H), 6.98 (d, *J* 8.6, 1H), 7.07 (s, 1H), 7.79 (d, *J* 8.6, 1H). This material was treated by the same procedure used in *Angew. Chem.,* **1992,** *104,* 198-200, to prepare 4-allyloxyphthalic acid from 4-allyloxyphthalic acid dimethyl ester, and afforded 4-(3-methylbut-2-enyloxy)phthalic acid;
¹H NMR (CD₃OD): δ 1.76 (s, 3H), 1.79 (s, 3H), 4.62 (d, *J* 6.8, 2H), 5.44-5.47 (m, 1H), 7.05 (d, *J* 8.8, 1H), 7.09 (s, 1H), 7.81 (d, *J* 8.8, 1H).
A mixture of this acid (67mg, 0.27mmol), 3-methoxy-4-(2-pyrrolidin-1-yl-ethoxy)-phenylamine **[Example A4]** (67mg, 0.28mmol) and *N*-cyclohexylcarbodiimide resin (1.69 mol. eq./g, 0.46 g, 0.78mmol) was stirred in a 50:50 mixture of DMF and dichloromethane (1.5ml) at room temperature under argon for 16h. The mixture was filtered through filter-aid and the resin washed several times with dichloromethane. The filtrate was evaporated and the crude residue purified by flash chromatography on silica gel eluting with methanol in dichloromethane to yield the title compound as a brown oil;
¹H NMR (CDCl₃): δ 1.78 (s, 3H), 1.82-1.87 (m, 7H), 2.62-2.80 (m, 4H), 3.01 (t, *J* 6.0, 2H), 3.86 (s, 3H), 4.22 (t, *J* 6.4, 2H), 4.66 (d, *J* 6.8, 2H), 5.47-5.51 (m, 1H), 6.92-7.01 (m, 3H), 7.21 (d, *J* 8.4, 1H), 7.40 (s, 1H), 7.82 (d, *J* 8.4, 1H),
which was converted to the hydrochloride salt by treatment with ethereal hydrogen chloride (1M).

### Example A40

### 2-[3-Methoxy-4-(2-pyrrolidin-1-ylethoxy)phenyl]-5-(2-methylallyloxy)isoindol-1,3-dione

Using the procedure of **Example A39** but utilising 2-methyl-propenol [Aldrich] in place of 3-methyl-2-butenol to give the title compound as the hydrochloride salt.

### Example A41

### 5-(Cyclopent-2-enyloxy)-2-[3-methoxy-4-(2-pyrrolidin-1-yl-ethoxy)-phenyl]-isoindole-1,3-dione

Using the procedure of **Example A39** but utilising racemic 2-cyclopentenol [Wiley] in place of 3-methyl-2-butenol to give the title compound as the hydrochloride salt, a racemate.

### Example A42

### 5-Cyclohexyloxy-2-[3-methoxy-4-(2-pyrrolidin-1-ylethoxy)phenyl] isoindol-1,3-dione

4-(Cyclohex-2-enyloxy)-phthalic acid dimethyl ester [**Example A37**] (67mg, 0.23mmol) was hydrogenated under an atmosphere of hydrogen at room temperature and pressure with 10% wet Pd/C (ca. 200mg) in THF (5mL) for 24h. The catalyst was removed by filtration and the solvent was evaporated under vacuum. The crude material was purified by flash chromatography on silica gel eluting with 0-20% MeOH in DCM to yield 4-cyclohexyloxy-phthalic acid dimethyl ester as an oil.
*m*/*z* (APCI+) 293 (15%, [M+H]⁺), 179 (100), 261 (65).
This was used in the procedure of **Example A39** in place of 4-(3-methylbut-2-enyloxy)phthalic acid dimethyl ester to give the title compound;
¹H NMR (CDCl₃): δ 1.30-2.03 (m, 14H), 2.68-2.80 (m, 4H), 3.01 (t, 2H), 3.86 (s, 3H), 4.22 (t, 2H), 4.40-4.46 (m, 1H), 6.92-7.00 (m, 3H), 7.19 (d, 1H), 7.38 (s, 1H), 7.81 (d, 1H); *m*/*z* (APCI-) 463 (20%, [M-H]⁻), 381 (100%).

### Example A43

### 5-(Cyclopentyloxy)-2-[3-methoxy-4-(2-pyrrolidin-1-yl-ethoxy)-phenyl]-isoindole-1,3-dione

Using the procedure of **Example A42** but utilising racemic 4-(cyclopent-2-enyloxy)-phthalic acid dimethyl ester **[Example A41]**in place of4-(cyclohex-2-enyloxy)-phthalic acid dimethyl ester to give the title compound as the hydrochloride salt, a racemate

### Examples B1, B2

### 2-[4-(2-Diisopropylamino-ethoxy)-3-methoxy-phenyl]-5-phenyl-2,3-dihydro-isoindol-1-one

### 2-[4-(2-Diisopropylamino-ethoxy)-3-methoxy-phenyl]-6-phenyl-2,3-dihydro-isoindol-1-one

A solution of 5-bromo-2-[4-(2-diisopropylamino-ethoxy)-3-methoxy-phenyl]-isoindole-1,3-dione **[Example A1]** (66mg, 0.14mmol) was dissolved in THF (ml) and treated with sodium borohydride (5.2mg, 0.14mmol). After stirring for 2 hours, the mixture was treated with isopropanol, stirred for a further 1h then treated with acetic acid (2 drops) and stirred for 16h at RT. The solvent was removed and the residue purified by flash chromatography to give an approximately 1:1 mixture of the 5- and 6- bromo substituted isomers of 2-[4-(2-diisopropylamino-ethoxy)-3-methoxyphenyl]-3-hydroxy-2,3-dihydro-isoindol-1-one (37mg). This material was dissolved in dichloromethane (2ml) and treated with triethylsilane (1ml) and trifluoroacetic acid (0.5ml). The mixture was stirred at RT for 16 hours, the solvents were removed *in vacuo* and the residue purified by flash chromatography on silica gel (eluting with methanol - dichloromethane - aq. ammonia) to obtain 2-[4-(2-diisopropylamino-ethoxy)-3-methoxy-phenyl]-2,3-dihydro-isoindol-1-one as an approximately 1:1 mixture of 5- and 6-bromo isomers (33mg). A solution of this material in benzene (5ml) was treated with aqueous sodium carbonate (2M, 1ml), ethanol (1ml), phenylboronic acid (9mg, 0.07mmol) and tetrakis(triphenylphosphine) palladium[0] (10mg) and the mixture heated to reflux for 16h. The less dense phase was decanted, filtered through filter-aid (washing with dichloromethane), the filtrate evaporated and purified by flash chromatography on silica gel (eluting with dichloromethane - methanol - aq. ammonia) and reverse phase preparative chromatography (aq. MeCN, TFA) to give the title compounds as an approximately 1:1 mixture of isomers as their TFA salts;
¹H NMR (CDCl₃): δ 1.5 (bd, 12H), 3.47 (d, 2H), 3.82 (m, 2H), 3.93 (s. 3H), 4.5 (t, 2H), 4.88, 4.89 (2s, 2H), 6.94-7.05 (2H, m), 7.25-7.75 (7H, m), 7.84 (0.5H, dd), 7.96 (0.5H, dd), 8.08-8.17 (1H, m), 12.5 (1H, bs) (m/z AP+) 459.

### Example B3

### 2-[3-Methoxy-4-(2-pyrrolidin-1-yl-ethoxy)-phenyl]-5-phenoxy-2,3-dihydro-isoindol-1-one

Utilising the procedure outlined in **Example B1/B2** but with 2-[3-methoxy-4-(2-pyrrolidin-1-yl-ethoxy)-phenyl]-5-phenoxy-isoindole-1,3-dione **[Example A4]** in place of 5-bromo-2-[4-(2-diisopropylamino-ethoxy)-3-methoxy-phenyl]-isoindole-1,3-dione gave the title compound in place of 5/6-bromo-2-[4-(2-diisopropylamino-ethoxy)-3-methoxy-phenyl]-2,3-dihydro-isoindol-1-one, but as the single isomer, as a TFA salt:
¹H NMR (CDCl₃): δ 2.1 (bd, 4H), 3.1 (2H, bm), 3.5 (2H, bs), 3.89 (s, 3H), 3.9 (bs, 2H), 4.43 (2H, bs), 4.7 (s, 2H), 6.90-7.44 (m, 9H), 7.93 (d, 1H), 7.94 (d, 1H), 12.5 (bs 1H).

### Example C1

### 3-[3-Methoxy-4-(2-pyrrolidin-1-yl-ethoxy)-phenyl]-7-phenoxy-3H-benzo[d][1,2,3]triazin-4-one

A solution of 2,4-dinitrobenzoic acid [Aldrich](1.06 g, 5mmol) in dichloromethane (20ml) was treated with oxalyl chloride (2ml) and 1 drop DMF. Vigorous evolution of gas occurred which ceased after 1 hour. The solution was evaporated to the yellow acid chloride. A solution of phenol (1.5g, 15.5mmol) in DMF (20ml) was introduced into a second flask charged with sodium hydride (480mg, 60% oil dispersion, 12mmol). When all the hydride had dissolved this solution was treated with the acid chloride, portionwise over five minutes. The solution was then heated to 60°C for 12h, cooled, the solvents removed by evaporation *in vacuo,* and the residue treated with a solution of 40% sodium hydroxide (50ml) and ethanol (50ml). The mixture was stirred at RT for 12 hours then acidified to pH 2 with (2M aq HCl) and extracted into ethyl acetate (3 x 100ml). The combined organic phase was washed with saturated brine (50ml), dried (MgSO₄), filtered, and evaporated to a yellow solid. This was purified by flash chromatography (ethyl acetate - hexane) to give 2-nitro-4-phenoxy-benzoic acid (894mg, 69%);
¹H NMR (CDCl₃): δ 4.8 (1H, bs), 7.10 (1H, dd), 7.16 (1H, dd), 7.21 (1H, d), 7.26-7.31 (2H, m), 7.46 (2H, dd), 7.94, (1H, d).
This acid (445mg, 1.72mmol) was dissolved in dichloromethane (10ml) and treated with oxalyl chloride (2ml) and 1 drop of DMF. Vigorous evolution of gas occurred which ceased after 1 hour. The solution was evaporated to the yellow acid chloride. This was dissolved in dichloromethane (10ml) and treated with 3-methoxy-4-(2-pyrrolidin-1-yl-ethoxy)-phenylamine **[Example A4]** and diethylisopropylamine polystyrene resin beads (3.49 mol. eq./g, 0.6g). The mixture was shaken for 12 hours then filtered, evaporated and purified by flash chromatography on silica gel (eluting with ammonia - methanol - dichloromethane) to give N-[3-methoxy-4-(2-pyrrolidin-1-yl-ethoxy)-phenyl]-2-nitro-4-phenoxy-benzamide as a yellow foam (600mg, 73%); ¹H NMR (CDCl₃): δ 1.81 (4H, m), 2.64 (4H, m), 2.94 (2H, t), 3.84 (3H, s), 4.14 (2H, t), 6.84 (1H, d), 6.93 (1H, dd), 7.08 (2H, d), 7.22-7.47 (5H, m), 7.56-7.60 (2H, m), 7.80 (1H, bs); m/z [AP+] 478 (M+H⁺, 100%).
This amine (400mg, 0.83mmol) was dissolved in ethanol (40ml) and treated with 10% palladium on carbon (250mg), then the mixture was hydrogenated at atmospheric pressure for 18 hours. The mixture was filtered and the solvent removed *in vacuo* to give 2-amino-*N*-[3-methoxy-4-(2-pyrrolidin-1-yl-ethoxy)-phenyl]-4-phenoxy-benzamide as a cream solid (380mg, 100%);
¹H NMR (CDCl₃): δ 1.99 (4H, bs), 3.14 (4H, bs), 3.24 (2H, t), 3.82 (3H, s), 4.30 (2H, t), 5.58 (2H, bs), 6.22 (1H, d), 6.30 (1H, dd), 6.81 (1H, d), 6.98 (1H, dd), 7.04 (2H, d), 7.15 (1H,t), 7.34-7.42 (3H, m), 7.57 (1H, d), 8.21 (1H, bs); m/z [AP+] 459 (M+H⁺, 100%).
This amine (58mg, 0.13mmol) was dissolved in dichloromethane (5ml) and acidified with trifluoroacetic acid (200ul) then treated with butyl nitrite [Aldrich](0.1ml); a brown colour immediately appeared. After 1 minute, the mixture was treated with 1,8-diazabicyclo[5.4.0]undec-7-ene (DBU) [Aldrich] (0.5ml). The colour immediately faded. The solvent was removed and the residue purified by flash chromatography using silica gel (eluting with dichloromethane - methanol - ammonia) to give the title compound;
¹H NMR (CDCl₃): δ 1.83 (4H, m), 2.69 (4H, m), 3.01 (2H, t), 3.90 (3H, m), 4.24 (2H, t), 7.03 (1H, d), 7.14-7.18 (4H, m), 7.30 (1H, t), 7.50-7.54 (4H, m), 8.39 (1H, d) which was crystallized from ether-HCl as the hydrochloride salt, a pale yellow solid

### Example C2

### 3-[3-Methoxy-4-(2-pyrrolidin-1-yl-ethoxy)-phenyl]-7-phenoxy-3H-quinazolin-4-one

2-Amino-*N*-[3-methoxy-4-(2-pyrrolidin-1-yl-ethoxy)-phenyl]-4-phenoxy-benzamide **[Example C1]** (50mg, 0.1 1mmol) was dissolved in 1,2-dichloroethane (5ml) and treated with triethylorthoformate [Aldrich] (2ml) then heated to reflux for 12h. The mixture was cooled and DBU (0.1ml) introduced. The solvent was evaporated then the residue purified by flash chromatography on silica gel (eluting with ammonia - methanol - dichloromethane) to obtain the title compound;
¹H NMR (CDCl₃): δ 1.86 (4H, m), 2.69 (4H, m), 3.02 (2H, t), 3.88 (3H, t), 4.24 (2H, t), 6.89-6.93 (2H, m), 7.01 (1H,d), 7.12-7.26 (5H, m), 7.43 (2H, t), 8.07 (1H, s), 8.31 (1H, dd); which was crystallized from ether-HCl as the hydrochloride salt, a white solid.

### Example C3

### 3-[3-Methoxy-4-(2-pyrrolidin-1-yl-ethoxy)-phenyl]-7-phenoxy-1H-quinazoline-2,4-dione

2-Amino-*N*-[3-methoxy-4-(2-pyrrolidin-1-yl-ethoxy)-phenyl]-4-phenoxy-benzamide **[Example C1]** (62mg, 0.14mmol) was dissolved in dichloromethane (5ml) and treated with carbonyl diimidazole (50mg, 0.31 mmol) and DBU (0.1 ml). The mixture was stirred for 4h at RT then the solvent removed and the residue purified by flash chromatography on silica gel (eluting with ammonia - methanol - dichloromethane) to obtain the title compound;
¹H NMR (CDCl₃): δ 1.79 (4H, m), 2.64 (4H, m), 2.97 (2H, t), 3.82 (3H, s), 4.20 (2H, t), 6.46 (1H, d), 6.76-6.83 (3H, m), 6.98 (1H, d), 7.09 (2H, d), 7.23-7.27 (1H, m), 7.40-7.44 (2H, m), 8.08 (1H, d), 8.8 (1H, bs);
which was crystallized from ether-HCl as the hydrochloride salt, a white solid.

### Example C4

### 3-[3-Methoxy-4-(2-pyrrolidin-1-yl-ethoxy)-phenyl]-2-methyl-7-phenoxy-3H-quinazolin-4-one

2-Amino-*N*-[3-methoxy-4-(2-pyrrolidin-1-yl-ethoxy)-phenyl]-4-phenoxy-benzamide **[Example C1]** (50mg, 0.11mmol) was dissolved in 1,2-dichloroethane (5ml) and treated with triethylorthoacetate [Aldrich] (2ml) then heated to 100°C for 12h. The mixture was cooled and DBU (0.1ml) introduced. The solvent was evaporated then the residue purified by flash chromatography on silica gel (eluting with aq. ammonia - methanol - dichloromethane) to obtain the title compound as a white solid;
¹H NMR (CDCl₃): δ 1.82 (4H, m), 2.23 (3H, s), 2.67 (4H, m), 3.00 (2H, t), 3.86 (3H, s), 4.22 (2H, t), 6.72 (1H, d), 6.77 (1H, dd), 7.00-7.04 (2H, m), 7.12-7.22 (3H, m), 7.26 (1H, t), 7.42 (2H, t), 8.22 (1H, d).

### Example C5

### 3-[3-Methoxy-4-(2-pyrrolidin-1-yl-ethoxy)-phenyl]-7-phenyl-3H-benzo[d][1,2,3] triazin-4-one

3-Nitrobiphenyl (3.0g, 15mmol), was dissolved in DMF (7.5ml) and dry chloroform (16.5mmol). This mixture was added dropwise to a stirred precooled mixture of potassium tert-butoxide (7.2mg, 60mmol) in DMF (20ml) and THF (25ml), at such a rate that the temperature was maintained between -69 and -73°C. When the addition was complete, the mixture was stirred for a further 1 minute then treated with acetic acid (1.5ml) in methanol (5ml) and allowed to warm to room temperature. The mixture was treated with saturated aqueous sodium bicarbonate (50ml) and extracted with dichloromethane (3 x 50ml), the combined organic phase dried (MgSO₄), filtered and evaporated, then the residue purified by flash chromatography (ether - hexane). This gave a yellow material which consisted of between 30 and 50% of dichloromethylsubstituted 3-nitrobiphenyl and starting material. The mixture was dissolved in acetonitrile (10ml) and treated with a solution of silver trifluoromethanesulphonate (15mmol, 3.84g) in water (5ml). The mixture was heated to reflux for 16h in the dark then cooled, the mixture concentrated *in vacuo,* filtered and the filtrate extracted with ether (3 x 50ml). The combined organic phase was evaporated and purified by flash chromatography to give 3-nitrobiphenyl-4-carbaldehyde;
¹H NMR (CDCl₃): δ 7.51-7.58 (3H, m), 7.29 (3H, s), 7.51-7.58 (3H, m), 7.69 (2H, dd), 8.03, 8.08 (2H, 2xd), 8.34 (1H, d), 10.49 (1H, s).
This aldehyde (454mg, 2.0mmol) was dissolved in acetic acid (10ml) and treated with sodium perborate tetrahydrate (385mg, 2.5mmol), the mixture was heated to 50°C for 48h then cooled, concentrated to a paste and washed with water (2 x 50ml). The residue was dried in a desiccator to afford 3-nitrobiphenyl-4-carboxylic acid as a white solid (420mg, 87%);
¹H NMR (CDCl₃): δ 5.5 (1H, bs), 7.48-7.56 (4H, m), 7.30 (1H, bs), 7.50-7.77 (3H, m).
This acid (417g, 1.72mmol) was dissolved in dichloromethane (10ml) and treated with oxalyl chloride (2ml) and 1 drop of DMF. Vigorous evolution of gas occurred which ceased after 1 hour. The solution was evaporated to the yellow acid chloride. This was dissolved in dichloromethene (10ml) and treated with 3-methoxy-4-(2-pyrrolidin-1-yl-ethoxy)-phenylamine **[Example C1]** (472mg, 2mmol) and diethylisopropylamine polystyrene resin beads (1.0g, 3.6 mmol equiv). The mixture was shaken for 12 hours then filtered, evaporated and purified by flash chromatography on silica gel (eluting with ammonia - methanol - dichloromethane) to give *N*-[3-methoxy-4-(2-pyrrolidin-1-yl-ethoxy)-phenyl]-2-nitro-4-phenyl-benzamide as a yellow foam (563mg, 71%);
¹H NMR (CDCl₃): δ 1.78 (4H, bs), 2.61 (4H, bs), 2.91 (2H, t), 3.79 (3H, s), 4.10 (2H, t), 6.80 (1H, d), 6.98 (1H, dd), 7.32 (1H, d), 7.42-7.50 (3H, m), 7.57 (2H, d), 7.66 (1H, d), 7.83 (1H, dd), 8.20 (2H, dd). *m*/*z* [AP+] 462 (M+H⁺, 100%).
This amine (560mg, 1.3mmol) was dissolved in ethanol (20ml) and treated with 10% palladium on carbon (100mg) then the mixture hydrogenated at atmospheric pressure for six hours. The mixture was filtered and the solvent removed *in vacuo* to give 2-amino-*N*-[3-methoxy-4-(2-pyrrolidin-1-yl-ethoxy)-phenyl]-4-phenyl-benzamide as a cream solid (518mg, 99%);
¹H NMR (CDCl₃): δ 1.82 (4H, m), 2.68 (4H, m), 2.96 (2H, t), 3.86 (3H, s), 4.16 (2H, t), 5.6 (2H, bs), 6.86-6.97 (4H, m), 7.35-7.58 (7H, m), 7.89 (1H, bs). ***m*/*z*** [AP+] 431(M+H⁺ 100%).
This amine was treated in the manner of 2-amino-*N*-[3-methoxy-4-(2-pyrrolidin-1-yl-ethoxy)-phenyl]-4-phenoxy-benzamide in **Example C1** to give the title compound; ¹H NMR (CDCl₃): δ 1.86 (4H, m), 2.73 (4H, m), 3.05 (2H, t), 3.91 (3H, t), 4.29 (2H, t), 7.06 (1H, d), 7.18-7.24 (2H, m), 7.47-7.57 (3H, m), 7.76 (2H, d), 8.06 (1H, d), 8.42 (1H, d), 8.49 (1H, d).
which crystallised from Et₂O / HCl as a pale yellow solid.

### Example C6

### 3-[3-Methoxy-4-(2-pyrrolidin-1-yl-ethoxy)-phenyl]-7-phenyl-3H-quinazolin-4-one

Using 2-amino-*N*-[3-methoxy-4-(2-pyrrolidin-1-yl-ethoxy)-phenyl]-4-phenyl-benzamide [**Example C5**] in the manner of 2-amino-*N*-[3-methoxy-4-(2-pyrrolidin-1-yl-ethoxy)-phenyl]-4-phenoxy-benzamide in Example C2, gave the title compound as a cream solid;
¹H NMR (CDCl₃): δ 1.83 (4H, m), 2.69 (4H, m), 3.01 (2H, t), 3.90 (3H, t), 4.24 (2H, t), 6.92-6.96 (2H, m), 7.03 (1H, d), 7.43-7.53 (3H, m), 7.72 (2H, d), 7.79 (1H, dd), 7.98 (1H, d), 8.15 (1H, s), 8.42 (1H,d).

### Example C7

### 3-[3-Methoxy-4-(2-pyrrolidin-1-yl-ethoxy)-phenyl]-7-phenyl-1H-quinazoline-2,4-dione

Using 2-Amino-*N*-[3-methoxy-4-(2-pyrrolidin-1-yl-ethoxy)-phenyl]-4-phenyl-benzamide **[Example C5]** in the manner of 2-amino-*N*-[3-methoxy-4-(2-pyrrolidin-1-yl-ethoxy)-phenyl]-4-phenoxy-benzamide in **Example C3,** gave the title compound; ¹H NMR (CDCl₃): δ 1.82 (4H, m), 2.67 (4H, m), 2.99 (2H, t), 3.83 (3H, s), 4.21 (2H, t), 6.81 (1H, d), 6.85 (1H, dd), 7.00 (1H, d), 7.23 (1H, d), 7.45-7.52 (4H, m), 7.62 (2H, d), 7.66 (1H, s), 8.21 (1H, d);
which crystallised from diethyl ether / HCl as a cream solid.

### Example C8

### 3-[3-Methoxy-4-(2-pyrrolidin-1-yl-ethoxy)-phenyl]-2-methyl-7-phenoxy-3H quinazolin-4-one

Using 2-amino-*N*-[3-methoxy-4-(2-pyrrolidin-1-yl-ethoxy)-phenyl]-4-phenyl-benzamide [**Example C5**] in the manner of 2-amino-*N*-[3-methoxy-4-(2-pyrrolidin-1-yl-ethoxy)-phenyl]-4-phenoxy-benzamide in **Example C4**, gave the title compound; ¹H NMR (CDCl₃): δ 1.85 (4H, m), 2.31 (3H, s), 2.69 (4H, m), 3.02 (2H, t), 3.87 (3H, t), 4.24 (2H, t), 6.76 (1H, d), 6.83 (1H, dd), 7.04 (1H, d), 7.41-7.52 (3H, m), 7.70-7.91 (3H, m), 7.91 (1H, d), 8.32 (1H, d).

### Example C9

### 7-Allyloxy-3-[3-methoxy-4-(2-pyrrolidin-1-ylethoxy)phenyl]-3H-quinazolin-4-one hydrochloride.

4-allyloxy-2-nitro-benzoic acid allyl ester [J. Org. Chem. 1987, 52(18), 4086] (0.21g, 0.8mmol) was dissolved in ethanol (5ml), and 40% sodium hydroxide added (5ml). The mixture was stirred for 16h then acidified to pH 2 with 2M hydrochloric acid. The mixture was extracted with ethyl acetate (3 x 25ml) and the combined organic phases washed with brine (75ml), dried (M_{g}SO₄) and concentrated to yield 4-allyloxy-2-nitrobenzoic acid as brown solid (0.16g);
¹H-NMR (DMSO) δ 4.72-4.74 (m, 2H), 5.29-5.45 (dd, 2H), 5.99-6.07 (m, 1H), 7.28-7.31 (dd, 1H), 7.49-7.50(d, 1H), 7.82-7.87 (d, 1H) 13.45 (bs, 1H).
This acid (0.16g, 0.72mmol) was dissolved in dichloromethane (1ml) and oxalyl chloride (0.2ml) added, followed by 1 drop of DMF. The mixture was stirred under argon for 1hr and concentrated to yield 4-allyloxy-2-nitrobenzoyl chloride, an orange solid (0.173g). This was redissolved in dichloromethane (5ml) and added dropwise to a stirred solution of 3-methoxy-4-(2-pyrrolidin-1-yl-ethoxy)-phenylamine **[Example C1]** (154mg, 0.66mmol) and triethylamine(72.8mg, 0.72mmol) in 5ml dichloromethane. The mixture was stirred for 90 minutes, then the solution washed with saturated sodium hydrogen carbonate (25ml), and brine (25ml). The residue was dried (MgSO₄) and concentrated to yield 4-allyloxy-N-[3-methoxy-4-(2-pyrrolidin-1-ylethoxy)phenyl]-2-nitro-benzamide a brown solid (191.5mg);
¹H-NMR (CDCl₃) δ 1.93 (bs, 4H), 3.04 (bm, 4H), 2.80-2.81 (bm, 2H), 3.74 (s, 3H), 4.21 (bm, 2H), 4.61-4.63 (bd, 2H), 5.30-5.47 (dd, 2H), 5.98-6.08(m, 1H), 6.75-6.77 (d, 1H), 6.96-6.99 (dm, 1H), 7.14-7.17 (dm, 1H), 7.49-7.50 (d, 2H), 7.57-7.59 (d, 1H), 8.59 (s, 1H).
A solution of this material (191.5mg, 0.43mmol) in methanol was added dropwise to a stirred suspension of iron powder (72.6mg, 1.3mmol) and ammonium chloride (115.8mg, 2.17mmol) in water (5ml). The mixture was heated to 80°C for 90mins, then hot filtered through celite. The filter cake was washed with dichloromethane (10ml) and water (10ml). The organic phase was separated and the aqueous layer basified with saturated sodium hydrogen carbonate. The aqueous layer was extracted with dichloromethane (3 x 10ml) and the combined organic layers washed with brine (10ml), then dried (MgSO₄) and concentrated to yield 4-allyloxy-2-amino- N-[3-methoxy-4-(2-pyrrolidin-1-ylethoxy)phenyl]-benzamide as a yellow gum;
¹H-NMR (DMSO) δ1.76-1.82 (bm, 4H), 2.62-2.64 (bm, 4H), 2.92-2.85 (t, 2H), 3.85 (s, 3H), 4.12-4.16 (t, 2H), 4.51-4.53 (m, 2H), 5.28-5.42 (m, 2H), 5.63 (bs, 2H), 5.99-6.06 (m, 1H), 6.18-6.19 (d, 1H), 6.26-6.29 (dd, 1H), 6.85-6.91 (m, 3H), 7.29-7.38 (d, 1H), 7.62 (s, 1H). This material was treated in the same manner as 2-amino-*N*-[3-methoxy-4-(2-pyrrolidin-1-yl-ethoxy)-phenyl]-4-phenoxy-benzamide **[Example C1]** in the method of **Example C2** to afford 7-allyloxy-3-[3-methoxy-4-(2-pyrrolidin-1-ylethoxy)phenyl]-3*H*-quinazolin-4-one which was crystallised from ether - HCl to give the title compound as a yellow solid (21.5mg);
¹H-NMR (CDCl₃) δ 2.11-2.25 (bd, 4H), 3.06 (bs, 2H), 3.54-3.57 (bd, 2H), 3.88 (bs, 5H), 4.62 (bs, 2H), 4.69-4.72 (dm, 2H), 5.35-5.53 (dd, 2H), 6.03-6.10 (m, 1H), 6.96 (bs, 2H), 7.07-7.10 (d, 1H), 7.17-7.21 (dd, 1H), 7.31-7.32 (d, 1H), 8.23-8.27 (d, 1H), 8.36 (s, 1H), 12.85 (bs, 1H).

### Example D1

### 3-[3-Methoxy-4-(2-pyrrolidin-1-yl-ethoxy)-phenyl]-6-phenoxy-3H-benzo[d][1,2,3]triazin-4-one

5-Chloro-2-nitrobenzoic acid [Aldrich] (600mg, 3mmol) was suspended in dichloromethane (5ml) and treated with oxalyl chloride (2ml) and DMF (1 drop). On stirring, an immediate effervescence occurred which ceased after 1 hour. The solvent was removed *in vacuo* and the residue dissolved in dichloromethane (10ml) and treated successively with DIEA resin (3.1mmol/g, 1.95g) and 3-methoxy-4-(2-pyrrolidin-1-yl-ethoxy)-phenylamine (650mg, 3mmol). The mixture was stirred at RT for 16 hours then filtered, evaporated, and the residue purified by flash chromatography (methanol - dichloromethane - aqueous ammonia) to afford 5-chloro-*N*-[3-methoxy-4-(2-pyrrolidin-1-yl-ethoxy)-phenyl]-2-nitro-benzamide as a yellow foam;
¹H NMR (CDCl₃): δ 1.86 (4H, m), 2.68 (4H, m), 2.98 (2H, t), 3.87 (3H, s), 4.16 (2H, t), 6.87 (1H, d), 6.94 (1H, dd), 7.34 (1H, d), 7.56-7.64 (3H, m), 8.09 (1H, d); *m*/*z* [AP+] 420 (100%, (M+H⁺), 422.
A solution of this material (210mg, 0.5mmol) in DMF (2ml) was treated with phenol (94mg, 1mmol) and sodium hydride (60% oil dispersion, 36mg, 0.9mmol) then the mixture heated to 60°C for 48h. The solution was cooled, evaporated and the residue suspended in dichloromethane and filtered. The filtrate was evaporated and purified by flash chromatography on silica gel (eluting with methanol - dichloromethane - aqueous ammonia) to afford *N*-[3-methoxy-4-(2-pyrrolidin-1-yl-ethoxy)-phenyl]-2-nitro-5-phenoxy-benzamide (200mg, 84%) as a yellow wax;
¹H NMR (CDCl₃): δ 1.80 (4H, m), 2.62 (4H, m), 2.92 (2H, t), 3.82 (3H, s), 4.12 (2H, t), 6.83 (1H, d), 6.93 (1H, dd), 7.03-7.11 (4H, m), 7.26-7.41 (2H, m), 7.43 (2H, m), 7.77 (1H, m), 8.11 (1H, d) *m*/*z* [AP+] 420 (100% M+H⁺).
A solution of this amine (100mg, 0.24mmol) in ethanol (10ml) was treated with 10% palladium on carbon (50% wet, 100mg), and hydrogenated at RT for 12 hours. The mixture was filtered, and the solvent removed *in vacuo* to give 2-amino-*N*-[3-methoxy-4-(2-pyrrolidin-1-yl-ethoxy)-phenyl]-5-phenoxy-benzamide as a white waxy solid, a portion of which was crystallised from Et₂O / HCl to give the **hydrochloride salt**
¹H NMR (CDCl₃): δ 1.99 - 2.11 (6H, bm), 2.97 (2H, bs), 3.39 (2H, bs), 3.77 (3H, s), 4.38 (2H, bs), 5.5 (2H, bs), 6.66 (1H, d), 6.78-6.98 (6H, m), 7.20-7.24 (1H, m), 7.36 (1H, s), 8.06 (1H, brs), 12.5 (1H, brs) *m*/*z* [AP+] 448 (100%, M+H⁺).
A solution of the free base of this amine (58mg, 0.13mmol) in dichloromethane (2ml) was acidified with 5 drops of TFA then treated with butyl nitrite (100ul). A deep orange colour formed immediately. After 1 minute, DBU was introduced dropwise until basic. The solvent was removed *in vacuo* and the residue purified by flash chromatography on silica gel (eluting with methanol - dichloromethane - aqueous ammonia) to give the title compound which crystallised as a pale yellow HCl salt on treatment with 1M HCl in ether;
¹H NMR (CDCl₃): δ 2.11, 2.25 (4H, 2xm), 3.07 (2H, m), 3.53 (2H, m), 3.88 (3H, s), 3.92 (2H, m), 4.61 (2H, t), 7.08 (1H, d), 7.12 (2H, d), 7.16-7.27 (2H, m), 7.45 (2H, t), 7.64 (1H, dd), 7.76 (1H, d), 8.20 (1H,d), 12.9 (1H, bs).

### Example D2

### 3-[3-Methoxy-4-(2-pyrrolidin-1-yl-ethoxy)-phenyl]-6-phenoxy-3H-quinazolin-4-one

A solution of 2-amino-*N*-[3-methoxy-4-(2-pyrrolidin-1-yl-ethoxy)-phenyl]-5-phenoxy-benzamide **[Example D1]** (25mg, 0.055mmol) in 1,2-dichloroethane (1ml) was treated with triethylorthoformate (2ml) and the mixture heated to 80°C for 16 hours. The mixture was cooled, 1 drop of DBU added and the mixture evaporated. The residue was subjected to flash chromatography on silica gel eluting with (methanol - dichloromethane - aqueous ammonia) to give the title compound;
¹H NMR (CDCl₃): δ 1.83 (4H, m), 2.67 (4H, m), 2.99 (2H, t), 3.88 (3H, s), 4.22 (2H, t), 6.89-6.92 (2H, m), 7.01 (1H, d), 7.08 (2H, d), 7.17 (1H, t), 7.38 (2H, t), 7.53 (1H, dd), 7.76 (1H, d), 7.84 (1H, d), 8.05 (1H, s); which crystallised as a pale yellow HCl salt on treatment with 1M HCl in ether.

### Example D3

### 3-[3-Methoxy-4-(2-pyrrolidin-1-yl-ethoxy)-phenyl]-6-phenoxy-1H-quinazoline-2,4-dione

A solution of 2-amino-*N*-[3-methoxy-4-(2-pyrrolidin-1-yl-ethoxy)-phenyl]-5-phenoxy-benzamide **[Example D1]** (25mg, 0.055mmol) in dichloromethane (1ml) was treated with carbonyl diimidazole (16mg, 0.1mmol), and DBU (1 drop). The mixture was stirred at RT for 4 hours then evaporated and the residue purified by flash chromatography on silica gel (eluting with methanol - dichloromethane - aqueous ammonia) to give the title compound;
¹H NMR (CDCl₃): δ 1.84 (4H, m), 2.0 (1H, brs), 2.74 (4H, m), 3.03 (2H, t), 3.82 (3H, s), 4.23 (2H, t), 6.77-6.84 (2H, m), 6.98-7.03 (4H, m), 7.12 (1H, t), 7.31-7.36 (3H, m), 7.71 (1H, d); which crystallised as a white HCl salt on treatment with 1M HCl in ether.

### Example D4

### 3-[3-Methoxy-4-(2-pyrrolidin-1-yl-ethoxy)-phenyl]-2-methyl-6-phenoxy-3H-quinazolin-4-one

A solution of 2-amino-*N*-[3-methoxy-4-(2-pyrrolidin-1-yl-ethoxy)-phenyl]-5-phenoxy-benzamide **[Example D1]** (25mg, 0.055mmol) in 1,2-dichloroethane (1ml) was treated with triethylorthoacetate (2ml) and the mixture heated to 80°C for 2 hours. The mixture was cooled, 1 drop of DBU added and the mixture evaporated. The residue was subjected to flash chromatography on silica gel eluting with (methanol - dichloromethane - aqueous ammonia) to give the title compound;
¹H NMR (CDCl₃): δ 1.83 (4H, m), 2.27 (3H, s), 2.69 (4H, m), 3.01 (2H, t), 3.86 (3H, s), 4.23 (2H, t), 6.72 (1H, d), 6.77 (1H, dd), 7.00-7.06 (3H, m), 7.14 (1H, t), 7.36 (2H, dd), 7.48 (1H, dd), 7.67 (1H, d), 7.77 (1H, d); which crystallised as a white HCl salt on treatment with 1M HCl in ether.

### Example D5

### 6-(4-Chloro-phenyl)-3-[3-methoxy-4-(2-pyrrolidin-1-yl-ethoxy)-phenyl]-3H-quinazolin-4-one

4'-Chloro-4-nitro-biphenyl [J. Amer. Chem. Soc; 68; 1946; 404] (2.74g, 11.7mmol) was dissolved in DMF (20ml) and THF (6ml), then potassium t-butoxide 5.26g (47mmol) was added. The stirred mixture was cooled to -73°C then a solution of chloroform in THF (6ml) was slowly introduced, maintaining this temperature. After a further 1 minute, the reaction was quenched with acetic acid (5ml). The mixture was poured into water (200ml) and extracted with dichloromethane. The crude product was purified by flash chromatography on silica (using dichloromethane - hexane as eluents) to give 4'-chloro-3-(1,1-dichloro-methyl)-4-nitro-biphenyl in 63% yield;
¹H NMR (CDCl₃): δ 8.33 (s) 1H; 8.10 (d) 1H; 7.7 (d) 1H; 7.68 (s) 1H; 7.55 (d) 2H; 7.50 (d) 2H.
4'-Chloro-3-(1,1-dichloromethyl)-4-nitrobiphenyl (0.4 g, 1.26mmol) was dissolved in methanolic sodium methoxide (15 mL; 0.5 M). The mixture was refluxed for 12 hours under argon. The solvent was evaporated and the residue diluted in 1,4-dioxane (15ml) and water (1ml), then a few drops of concentrated HCl were added. The reaction mixture was stirred at 100 °C for 4 hours. The solvent was then evaporated and the crude mixture purified by flash chromatography on silica gel (eluting with dichloromethane in petroleum ether) to yield the 4'-chloro-4-nitrobiphenyl-3-carbaldehyde (0.32 g, 1.26mmol); *m*/*z* (APCI-) 260 (40%, [M-H]⁻), 231 (100%).
This aldehyde (0.32 g, 1.26mmol) was dissolved in acetic acid (5 mL) and sodium perborate (0.23 g, 1.5mmol) was added. The resulting mixture was stirred at 50 °C for 16h. The mixture was cooled, the solvent was evaporated, ethyl acetate was added and the organic layer washed with water, dried over MgSO₄ and evaporated to yield the 4'-chloro-4-nitrobiphenyl-3-carboxylic acid (0.14 g, 41 %); *m*/*z* [APCI-] 276 (100%, [M-H⁻], 247 (60%).
This acid (0.14 g, 0.5mmol) was dissolved in dichloromethane (2ml) and oxalyl chloride added (87µl, 1mmol). The mixture was stirred for 60 min at room temperature under argon. The solvent was evaporated and the residue added to a solution of 3-methoxy-4-(2-pyrrolidin-1-ylethoxy)phenylamine [**Example A4**] (0.10g, 0.44mmol) and triethylamine (70µl, 0.5mmol) in dichloromethane (4ml). The mixture was stirred for 90 mins at room temperature under argon and then washed with a saturated aqueous solution of NaHCO₃ (5ml) and brine (5ml). The organic layer was dried over MgSO₄, the solvent evaporated and the residue purified by flash chromatography on silica gel (eluting with methanol - dichloromethane - aq. ammonia). 4'-Chloro-4-nitrobiphenyl-3-carboxylic acid [3-methoxy-4-(2-pyrrolidin-1-ylethoxy)phenyl]amide was obtained in 54% yield; *m*/*z* [APCI⁺] 496 (100%, M+H⁺). Iron powder (45mg, 0.81mmol) and ammonium chloride (72mg, 1.35mmol) were mixed in water (2ml) and 4'-chloro-4-nitrobiphenyl-3-carboxylic acid [3-methoxy-4-(2-pyrrolidin-1-ylethoxy)phenyl]amide (0.13 g, 0.27mmol) added, dissolved in MeOH (2ml). The mixture was heated at 80 °C for 90 min and then filtered while hot. Dichloromethane (30ml) was added to the cooled filtrate. The organic layer was washed with water (10ml), saturated aqueous NaHCO₃ (10ml) and brine (10ml), then dried and the solvent evaporated to give a yellow gum. This was dissolved in 1,2-dichloroethane (10ml) and triethylorthoformate (4ml). The mixture was refluxed for 16h under argon, then after cooling DBU (0.2ml) was added and the mixture was stirred for 10 mins at room temperature. The solvent was evaporated and the residue purified by flash chromatography on silica gel (eluting with methanol - dichloromethane - aqueous ammonia) to yield the title compound as an orange gum;
¹H NMR (CDCl₃): δ 1.78-1.86 (m, 4H), 2.65-2.78 (m, 4H), 2.99 (t, 2H), 3.89 (s, 3H), 4.22 (t, 2H), 6.92-6.95 (m, 2H), 7.03 (d, 1H), 7.45 (d, 2H), 7.63 (d, 2H), 7.83 (d, 1H), 8.00 (d, 1H), 8.13 (s, 1H), 8.54 (s, 1H);
which was converted to the hydrochloride salt by treatment with a solution of HCl in ether.

### Example E1

### 3-[3-Methoxy-4-(2-pyrrolidin-yl-ethoxy)-phenyl]-6-phenyl-3H-thieno[2,3-d]ltriazine-4-one

6-Phenyl-3*H*-thieno[2,3-d][1,2,3]-triazinone (Indian Journal of Chemistry, 9, 1971, 1209), (100mg, 0.44mmol) and 3-methoxy(4-pyrrolidin-1-ylethoxy)phenylamine (103mg, 0.44 mMol) **[Example A4]** were refluxed in xylene (5ml) for 16h. The solvent was evaporated and the residue purified by chromatography on deactivated neutral alumina (eluting with ethyl acetate - methanol) to give 2-amino-5-phenyl-thiophene-3-carboxylic acid [3-methoxy-4-(2-pyrrolidin-1-yl-ethoxy)-phenyl]-amide in 38% yield;
¹H NMR (CDCl₃): δ 10.7 (bs, 1H), 9.4 (s, 1H), 7.90 (s, 1H), 7.62 (s, 2H), 7.51 (d, 1H), 7.48 (d, 2H), 7.39 (t, 2H), 7.3 (dd, 1H), 7.18 (t, 1H), 7.01 (d, 1H), 4.29 (t, 2H), 3.80 (s, 3H), 3.51 (m, 4H), 1.98 (m, 4H); *m*/*z* [ES+], 438, [ES-] 436.
This material (50mg, 0.114mmol) was treated with butyl nitrite (1ml) in dichlomethane (5ml) at room temperature. The crude product was purified by flash chromatography on silica gel (eluting with dichloromethane - methanol - aq. ammonia) to give the title compound;
¹H NMR (CDCl₃): δ 7.87 (s, 1H), 7.75 (d, 2H), 7.49 (m, 3H), 7.18 (dd, 1H), 7.15 (dd, 1H), 7.05 (d,1H), 4.26 (t, 2H), 3.91 (s, 3H), 3.01 (t, 2H), 3.67 (m, 4H), 1.82 4H.

### Example E2

### 3-[3-Methoxy-4-(2-pyrrolidin-yl-ethoxy)-phenyl]-6-phenyl-3-H-thieno-[2,3-d]pyrimidin-4-one

2-Amino-5-phenyl-thiophene-3-carboxylic acid [3-methoxy-4-(2-pyrrolidin-1-yl-ethoxy)phenyl]-amide **[Example E1]** (28mg, 0.064mmol) was stirred at 95°C in triethyl orthoformate (5ml) for 24h. The mixture was cooled, 4 drops of DBU added and the volatiles removed. The crude product was purified by chromatography on silica gel (eluting with dichloromethane - methanol - aq. ammonia) to give the title compound.
¹H NMR (CDCl₃): δ 8.06 (s, 1H), 7.73 (s, 1H) 7.68 (d, 2H) 7.42 (t, 3H), 7.35 (t, 1H), 7.01 (d, 1H), 6.92 (s, 1H) 4.23 (t, 2H), 3.92 (s, 3H), 3.00 (t, 2H), 2.68 (m, 4H), 1.84 (m, 4H).

### Example F1

### 3-[3-Methoxy-4-(2-pyrrolidin-1-yl-ethoxy)-phenyl]-6-phenyl-3H-thieno[3,2-d]pyrimidin-4-one

A solution of 3-[3-methoxy-4-(2-pyrrolidin-1-ylethoxy)phenyl]-2-(methylthio)-6-phenylthieno[3,2-*d*]pyrimidin-4(3*H*)-one **[Example F2]** 80mg (0.16mmol) in ethanol (10ml) was treated with Raney Nickel [W10, 50mg] with stirring at room temperature for 1h. The solvent was evaporated and the residue purified by reverse phase preparative chromatography (using acetonitrile - water - TFA) to give the title compound as a trifluoroacetate salt;
¹H NMR (CDCl₃): δ 8.25 (bs, 1H), 7.76 (d, 2H), 7.61 (m, 2H), 7.50 (m, 3H), 7.05 (d,1H), 6.98 (d,1H), 6.94 (dd, 1H), 4.46 (t, 2H), 4.00 (m, 2H), 3.89 (s, 3H), 3.64 (t, 2H), 3.12 (m, 2H) 2.18 (m, 4H) 448

### Example F2

### 3-[3-methoxy-4-(2-pyrrolidin-1-ylethoxy)phenyl]-2-(methylthio)-6-phenylthieno[3,2-d]pyrimidin-4(3H)-one

5-Phenyl 3-amino thiophenecarboxylic acid methyl ester [Lancaster] (2g, 8.54mMol) and 2,2' Bis pyridyl thionate [Aldrich] (2g, 8.54mmol) were dissolved in dichloromethane (20ml) then a solution of 3-methoxy(4-pyrrolidin-1-ylethoxy)phenylamine **[Example A4]** in dichloromethane (20ml) introduced. The mixture was stirred for 2 hours at room temperature, then the solvent removed and the product purified by chromatography on silica gel (eluting with methanol - dichloromethane), to give 3-{3-[3-methoxy-4-(2-pyrrolidin-1-yl-ethoxy)-phenyl]-thioureido}-5-phenyl-thiophene-2-carboxylic acid methyl ester in 52% yield;
¹H NMR (CDCl₃): δ 10.60 (s, 1H), 9.14 (s, 1H), 7.90 (s, 1H), 7.65 (d, 2H), 7.40 (m, 3H), 6.97 (d, 1H), 6.88 (m, 2H), 4.20 (t, 2H), 3.89 (s, 3H), 3.73 (s, 3H), 2.98 (t, 2H), 2.66 (m, 4H), 1.83 (m, 4H). *m*/*z* 512 (ES+) 510 (ES-).
This ester (300mg, 0.56mmol) and potassium carbonate (162mg, 1.17mmol) were suspended in dry DMF (5ml) and treated with a solution of methyl iodide (83mg, 0.59mmol) in DMF (1ml). When the addition was complete, the mixture was evaporated. The residue was purified by chromatography on silica gel (eluting with dichloromethane - methanol) giving the title compound in 50% yield;
¹H NMR (CDCl₃): δ 7.72 (d, 2H), 7.45 (m, 4H), 7.05 (d, 1H), 6.90 (dd, 1H), 6.81 (d, 1H), 4.52 (m,1H), 4.41 (m, 1H), 3.87 (s, 3H), 3.37 (m, 2H), 3.21 (m, 4H), 3.52 (s, 3H), 2.08 (m, 4H).

### Example F3

### 3-[3-Methoxy-4-(pyrrotidin-1-yl-ethoxy)phenyl-6-phenyl-2-thioxo-2,3-dihydro-1H-thieno[3,2-d]pyrimidin-4-one

3-{3-[3-Methoxy-4-(2-pyrrolidin-1-yl-ethoxy)-phenyl]-thioureido}-5-phenyl-thiophene-2-carboxylic acid methyl ester **[Example F2]** (100mg, 0.2mmol) was refluxed in toluene (10ml) for 16 hours. The product was collected from the cooled solution by filtration in 42% yield;
¹H NMR (CDCl₃): δ 7.80 (m, 2H), 7.50 (m, 4H), 7.03 (d, 1H), 6.90 (d, 1H), 6.75 (dd, 1H), 4.14 (t, 2H), 3.73 (s, 3H), 2.98 (t, 2H), 2.70 (m, 4H), 1.76 (m, 4H); *m*/*z* [ES+] 480; (ES-) 478.

### Example G1

### 2-[3-Methoxy-4-(3-pyrrolidin-1-yl-propyl)-phenyl]-5-phenoxy-isoindole-1,3-dione

6-Nitroindanone [J.Med.Chem., 19, 1976, 472-475] (900mg, 5.0mmol) was suspended in dichloromethane (25ml) and trifluoromethanesulphonic acid (0.05ml) added. The resulting solution was cooled on ice. In a second flask, m-chloroperoxybenzoic acid [55%, Aldrich] (10g) was suspended in dichloromethane and stirred for several minutes. The insoluble material was removed by filtration through a hydrophobic membrane and the filtrate evaporated to give a white powder. A portion of this material (2.6g) was added portionwise to the indanone and the resultant suspension stirred for 72h at room temperature. The reaction mixture was diluted with dichloromethane (10ml) and aqueous sodium disulphite (20%; 10ml). The aqueous layer was extracted (30ml x 3) with dichloromethane and the combined organic layers washed with saturated aqueous sodium bicarbonate (20ml), brine (10ml), and dried (MgSO₄) to yield crude 7-nitro-chroman-2-one as a brown solid (1.1g, ~70% pure);
¹H NMR (CDCl₃): δ 8.01 (dd, 1H,), 7.91 (dd, 1H), 7.40 (d, 1H,), 3.14 (d, 2H,), 2.86 (d, 2H,).
The crude 7-nitro-chroman-2-one (1.1g, approx. 4.2mmol) was dissolved in tetrahydrofuran (15ml). Pyrrolidine (0.35ml) was added and the reaction mixture stirred for 1 hour. The solution was adsorbed onto silica and eluted using methanol in dichloromethane. 3-(2-hydroxy-4-nitrophenyl)-1-pyrrolidin-1-yl-propan-1-one was obtained as a brown solid (900mg, 3.40mmol);
¹H NMR (CDCl₃): δ 10.62 (br, 1H); 7.74 (d, 1H); 7.66 (dd, 1H); 7.17 (d, 1H); 3.47 (m, 2H); 3.36 (m, 2H); 3.00 (m, 2H); 2.70 (m, 2H); 1.92 (m, 4H); MS (ES+) 265.2 (M+H⁺; 100%).
This amide (900mg, 3.4mmol) was dissolved in dimethylformamide (100ml) and potassium carbonate (516mg) added, followed by iodomethane (0.6ml, 3.7mmol). The solution was stirred for 5 hours, then concentrated *in vacuo.* The residual oil was partitioned between ethyl acetate (50ml) and H₂O (50ml), the aqueous layer extracted with ethyl acetate (3 x 50ml), the combined organic phases washed with brine (20ml), dried (MgSO₄) and evaporated to yield 3-(2-methoxy-4-nitrophenyl)-1-pyrrolidin-1-yl-propan-1-one as a black oil (900mg, 3.2mmol);
¹H NMR (CDCl₃): δ 7.78 (dd, 1H, J=8.2 Hz, 2.2 Hz); 7.68 (d, 1H, J=2.2 Hz); 7.36 (d, 1H, J=8.2 Hz); 3.93 (s, 3H); 3.46 (t, 4H, J=6.6 Hz); 3.34 (t, 2H, J=7.7 Hz); 2.55 (t, 2H, J=7.7 Hz); 1.89 (m, 4H)
This amide (900mg, 3.2mmol) was dissolved in tetrahydrofuran (150ml) and treated with borane:tetrahydrofuran (1M, 10ml). The reaction mixture was heated to 50°C for 4 hours, followed by a further addition of borane:THF solution (10ml). After heating for a further 16 hours, a third portion (10ml) was added, and heating maintained for a further 3 hours. The reaction mixture was cooled and methanol (10ml) added, followed by conc. hydrochloric acid (0.5ml). The mixture was heated to 80°C for 1 hour, then evaporated *in vacuo.* The residue was partitioned between ethyl acetate (50ml) and H₂O (50ml), the aqueous phases extracted with ethyl acetate (3 x 50ml) and the combined organics washed with brine (20ml), dried (MgSO₄) and evaporated. The resultant crude brown oil was purified by chromatography on silica gel using methanol in dichloromethane as eluent. 1-[3-(2-methoxy-4-nitrophenyl)-propyl]-pyrrolidine was obtained as a clear oil (186mg, 22%);
¹H NMR (CDCl₃): δ 7.80 (dd, 1H,); 7.78 (d, 1H); 7.29 (d, 1H); 3.93 (s, 3H); 3.20 (m, 2H), 2.82 (m, 2H); 2.68 (m, 4H); 2.16 (m, 4H); 1.86 (m, 2H).
This amine (186mg, 0.7mmol) was dissolved in 4:1 mixture of ethanol:THF (20ml), and palladium on carbon (10% wet paste; 100mg) added. The mixture was stirred under an atmosphere of hydrogen for 16h, then filtered over filter-aid and evaporated to yield 3-methoxy-4-(3-pyrrolidin-1-yl-propyl)-phenylamine as a colourless oil (148mg, 90%);
¹H NMR (CDCl₃):δ 6.88 (m, 1H); 6.21 (m, 2H); 3.74 (s, 3H); 3.61 (br, 2H); 2.49 (m, 8H); 1.77 (m, 6H).
This amine was used in place of 3-methoxy-4-(3-pyrrolidin-1-yl-propyl)-phenylamine in using the procedure of **Example A4** to give 2-[3-methoxy-4-(3-pyrrolidin-1-yl-propyl)-phenyl]-5-phenoxy-isoindole-1,3-dione.

### Example G2

### 4-(1,3-Dioxo-5-phenoxy-1,3-dihydro-isoindol-2-yl)-2-methoxymethoxy-N-(2-pyrrolidin-1-yl-ethyl)-benzamide

A solution of 2-chloro-4-nitrophenol [Specs] (345mg, 2mmol) in DMF (5ml) was treated with sodium hydride (60% oil dispersion, 140mg, 3.5mmol), then chloromethylmethyl ether (193mg, 182ul, 2.4mmol). The mixture was stirred for 24 h then methanol (5ml) added, the mixture stirred for a further 1h then evaporated, the residue dissolved in dichloromethane, filtered, evaporated and the residue purified by flash chromatography (diethyl ether - hexane) to afford 1-chloro-2-methoxymethoxy-4-nitro-benzene as a clear oil;
¹H NMR (CDCl₃): δ 3.55 (3H, s), 5.35 (2H, s), 7.53 (1H, d), 7.84 (1H, dd), 8.05 (1H, d).
This material (445mg, 2.05mmol) was dissolved in DMF (5ml) and treated with *N*-2-hydroxyethylpyrrolidine (288mg, 2.5mmol), and sodium hydride (60% oil dispersion, 90mg, 2.3mmol), added. The mixture was heated to 80°C for 8 hours then cooled and stirred at RT for 30h. The solvent was removed *in vacuo,* and the residue subjected to flash chromatography (aq. ammonia - methanol - dichloromethane) to afford 1-[2-(2-methoxymethoxy-4-nitro-phenoxy)-ethyl]-pyrrolidine as a waxy solid (449mg, 1.5mmol).
¹H NMR (CDCl₃): δ 1.81 (4H, m), 2.66 (4H, m), 2.97 (2H, t), 3.53 (3H, s), 4.25 (2H, t), 5.26 (2H, s), 6.95 (1H, d), 7.94 (1H, dd), 8.00 (1H, d).
This material (449mg, 1.5mmol) was dissolved in ethanol (30ml) and hydrogenated on 10% Pd on carbon (100mg) for 6 hours at RTP. The catalyst was removed by filtration and the filtrate evaporated to give 3-methoxymethoxy-4-(2-pyrrolidin-1-yl-ethoxy)-phenylamine as a white solid;
¹H NMR (CDCl₃): δ 1.80 (4H, m), 2.64 (4H, m), 2.86 (2H, t), 3.4 (2H, brs), 3.50 (3H, s), 4.07 (2H, t), 5.16 (2H, s), 6.28 (1H, dd), 6.54 (1H, d), 6.76 (1H, d)
This material was treated with 3-phenoxyphthalic anhydride in the same manner as for 4-(2-diisopropylamino-ethoxy)-3-methoxy-phenylamine in **Example A4** to give the title compound, which crystallised from dilute TFA in ether as a trifluoroacetate salt.

### Example G3

### 2-[3-(2-Methoxy-ethoxy)-4-(2-pyrrolidin-1-yl-ethoxy)-phenyl]-5-phenoxy-isoindole-1,3-dione

2-Chloro-4-nitrophenol was treated with 2-methoxyethyl chloride [Aldrich] in place of chloromethyl methyl ether in the procedure of **Example G2** to give the title compound, which crystallised from dilute TFA in ether as a trifluoroacetate salt.

### Example G4

### 2-[3-Methoxymethyl-4-(2-pyrrolidin-1-yl-ethoxy)-phenyl]-5-phenoxy-isoindole-1,3-dione

1-Chloro-2-methoxymethyl-4-nitro-benzene [Patent: US 5084449 A] was utilised in place of 1-chloro-2-methoxymethoxy-4-nitro-benzene in the procedure of **Example G2** to afford the title compound as a white solid as the trifluoroacetate salt.

### Example G5

### 2-[3-Hydroxy-4-(2-pyrrolidin-1-yl-ethoxy)-phenyl]-5-phenoxy-isoindole-1,3-dione

4-(1,3-Dioxo-5-phenoxy-1,3-dihydro-isoindol-2-yl)-2-methoxymethoxy-*N*-(2-pyrrolidin-1-yl-ethyl)-benzamide **[Example G2]** was treated with trifluoroacetic acid (1ml, 1M in dichloromethane). After 30 minutes, the solvent was removed to afford the title compound as a trifluoroacetate salt.

### Example G6

### 2-{4-[2-(2,5-Dimethyl-pyrrolidin-1-yl)-ethoxy]-3-methoxy-phenyl}-5-phenoxy-isoindole-1,3-dione

2,5-dimethyl-pyrrolidine was treated in the same manner as azetidine in **Example G11** to give first 1-(2,5-dimethyl-pyrrolidin-1-yl)-2-(2-methoxy-4-nitro-phenoxy)-ethanone; ¹H NMR (CDCl₃): δ 7.86 (dd, 1H), 7.76 (d, 1H), 6.93 (d, 1H), 4.85 (bm, 2H), 4.12 (bm, 2H), 3.96 (s, 3H), 2.11 (bm, 1H), 1.99 (bm, 1H), 1.70 (bm, 2H), 1.30 (m, 6H). This was treated in the same manner as 1-azetidin-1-yl-2-(2-methoxy-4-nitro-phenoxy)-ethanone **[Example 19]** to give 1-[2-(2-methoxy-4-nitro-phenoxy)-ethyl]-2,5-dimethyl-pyrrolidine;
¹H NMR (CDCl₃): δ 7.89 (dd, 1H), 7.74 (d, 1H), 6.92 (m, 1H), 4.16 (t, 2H), 3.94 (s, 3H), 2.71 (m, 2H), 1.87 (m, 2H), 1.39 (m, 2H), 1.15 & 1.05 (d, 6H).
This material was treated in the same manner as 1-[2-(2-methoxy-4-nitro-phenoxy)-ethyl]-azetidine in **Example G11** to give the title compound (as a mixture of meso isomers and enantiomers).

### Example G7

### 2-{4-[2-(2-methyl-pyrrolidin-1-yl)-ethoxy]-3-methoxy-phenyl}-5-phenoxy-isoindole-1,3-dione

2-methylpyrrolidine was treated in the same manner as azetidine in **Example G11** to give first 2-(2-methoxy-4-nitro-phenoxy)-1-(2-methyl-pyrrolidin-1-yl)-ethanone; ¹H NMR (CDCl₃): δ 7.84 (dd, 1H), 7.75 (d, 1H), 6.94 (d, 1H), 4.79 (s, 2H), 4.23 (bm, 3H), 3.96 (s, 3H), 3.53 (bm, 2H), 1.96 (bm, 4H), 1.20 (d, 3H). This was treated in the same manner as 1-azetidin-1-yl-2-(2-methoxy-4-nitro-phenoxy)-ethanone **[Example 19]** to give 1-[2-(2-methoxy-4-nitro-phenoxy)-ethyl]-2-methyl-pyrrolidine;
¹H NMR (CDCl₃): δ 7.89 (dd, 1H), 7.74 (d, 1H), 6.92 (d, 1H), 4.24 (t, 2H), 3.94 (s, 3H), 3.28 (m, 2H), 2.66 (q, 1H, J=6.5 Hz), 2.4 (m, 2H), 1.95 (m, 1H), 1.79 (m, 2H), 1.46 (m, 1H), 1.14 (d, 3H, J=6.07 Hz). This material was treated in the same manner as 1-[2-(2-methoxy-4-nitro-phenoxy)-ethyl]-azetidine in **Example G11** to give the title compound.

### Example G8

### 2-{4-[2-(2-methyl-piperidin-1-yl)-ethoxy]-3-methoxy-phenyl}-5-phenoxy-isoindole-1,3-dione

2-Methylpiperidine was treated in the same manner as azetidine in **Example G11** to give first 2-(2-methoxy-4-nitro-phenoxy)-1-(2-methyl-piperidin-1-yl)-ethanone; ¹H NMR (CDCl₃): δ 7.85 (dd, 1H), 7.75 (d, 1H), 6.97 (d, 1H), 4.88 (s, 2H), 3.96 (s, 3H), 1.72-1.65 (bm, 6H), 1.30-1.13 (bm, 6H); *m*/*z* [AP+] 309.2 (NM⁺, 100 %). This was treated in the same manner as 1-azetidin-1-yl-2-(2-methoxy-4-nitro-phenoxy)-ethanone **[Example 19]** to give 1-[2-(2-methoxy-4-nitro-phenoxy)-ethyl]-2-methylpiperidine.
¹H NMR (CDCl₃): δ 7.86 (dd, 1H), 7.76 (d, 1H), 6.93 (d, 1H), 4.91 (bm, 2H), 3.96 (s, 3H), 1.85 (bm, 2H), 1.63 (bm, 4H), 1.56-1.50 (bm, 2H), 1.32-1.21 (bm, 6H); *m*/*z* [AP+] 323.2 (MH⁺, 100 %). This material was treated in the same manner as 1-[2-(2-methoxy-4-nitro-phenoxy)-ethyl]-azetidine in **Example G11** to give the title compound.

### Example G9

### 2-{4-[2-((cis)-2,6-dimethyl-piperidin-1-yl)-ethoxy]-3-methoxy-phenyl}-5-phenoxy-isoindole-1,3-dione

2,5-(*cis*)-Dimethylpiperidine was treated in the same manner as azetidine in **Example G11** to give first 1-[2-(2-methoxy-4-nitro-phenoxy)-ethyl]-(*cis*)-2,6-dimethyl-piperidine; ¹H NMR (CDCl₃): δ 7.89 (dd, 1H), 7.74 (d, 1H), 6.94 (d, 1H), 4.21 (t, 2H), 3.94 (s, 3H), 3.17 (q, 1H), 2.95 (m, 1H), 2.84 (q, 1H), 2.39 (m, 2H), 1.65 (m, 4H), 1.33 (m, 2H), 1.12 (d, 3H).

This was treated in the same manner as 1-azetidin-1-yl-2-(2-methoxy-4-nitro-phenoxy)-ethanone **[Example 19]** to give 1-[2-(2-methoxy-4-nitro-phenoxy)-ethyl]-(*cis*)-2,6-dimethyl-piperidine;
¹H NMR (CDCl₃): δ 7.89 (dd, 1H), 7.74 (d, 1H), 6.91 (d, 1H), 4.09 (t, 2H), 3.93 (s, 3H), 3.11 (t, 2H), 2.56 (m, 2H), 1.69 (m, 1H), 1.55 (bm, 2H), 1.32 (m, 3H), 1.18 (d, 6H). This material was treated in the same manner as 1-[2-(2-methoxy-4-nitro-phenoxy)-ethyl]-azetidine in **Example G11** to give the title compound.

### Example G10

### 2-{4-[2-(2,2,6,6-tetramethyl-piperidin-1-yl)-ethoxy]-3-methoxy-phenyl}-5-phenoxy-isoindole-1,3-dione.

2,2,6,6-Tetramethylpiperidine was treated in the same manner as azetidine in **Example G11** to give first 2-(2-methoxy-4-nitro-phenoxy)-1-(2,2,6,6-tetramethyl-piperidin-1-yl)-ethanone;
¹H NMR (CDCl₃): δ 7.84 (dd, 1H), 7.75 (d, 1H), 6.86 (d, 1H), 4.84 (s, 2H), 3.96 (s, 3H), 1.77 (bm, 6H), 1.49 (s, 12H).
This was treated in the same manner as 1-azetidin-1-yl-2-(2-methoxy-4-nitro-phenoxy)-ethanone **[Example 19]** to give 1-[2-(2-methoxy-4-nitro-phenoxy)-ethyl]-2,2,6,6-tetramethyl-piperidine;
¹H NMR (CDCl₃): δ 7.89 (dd, 1H), 7.74 (d, 1H), 6.92 (d, 1H), 3.96 (m, 5H), 2.99 (t, 2H), 1.55 (bm, 2H), 1.08 (s, 12H).
This material was treated in the same manner as 1-[2-(2-methoxy-4-nitro-phenoxy)-ethyl]-azetidine in **Example G11** to give the title compound.

### Example G11

### 2-[4-(2-Azetidin-1-yl-ethoxy)-3-methoxy-phenyl]-5-phenoxy-isoindole-1,3-dione

A suspension of (2-methoxy-4-nitro-phenoxy)acetic acid [FEBS Lett. (1983), 153(2), 431], (3.12g 15.9mmol) in dichloromethane (100ml) was cooled down to 0°C in an ice bath before oxalyl chloride (5.26g, 41.7mmol) was added. The reaction mixture was stirred for 45 minutes, giving a clear solution. The solvents were removed *in vacuo* to give (2-methoxy-4-nitro-phenoxy)-acetyl chloride as a pale yellow solid. (3.41 g, 100%);
¹H NMR (CDCl₃): δ 7.87 (dd, 1H,), 7.81 (d, 1H), 6.92 (d, 1H), 5.09 (s, 2H), 3.98 (s, 3H).
A solution of this acid chloride (300mg, 1.22mmol) was dissolved in dichloromethane (12ml) and treated with azetidine (76mg, 1.34mmol) followed by triethylamine (370mg, 3.66mmol). The reaction mixture was stirred at RT for 16h under an argon atmosphere. The solvent was removed *in vacuo* to give a dark brown oil which was purified by flash chromatography on silica gel (eluting with dichloromethane - methanol - aqueous ammonia) to give 1-azetidin-1-yl-2-(2-methoxy-4-nitro-phenoxy)-ethanone as a yellow oil (150mg, 46 %);
¹H NMR (CDCl₃): 7.88 (dd, 1H), 7.77 (d, 1H), 6.91 (d, 1H), 4.70 (s, 2H), 4.37 (t, 2H), 4.11 (t, 2H), 3.96 (s, 3H), 2.34 (q, 2H).
This amide was dissolved in THF (15ml) then borane (5.6ml, 1M solution in THF) was introduced. The reaction mixture was stirred for 18 hours at 50°C under argon. Excess borane was quenched by the dropwise addition of MeOH then aq HCl (2M, 200ul). The solvent was removed *in vacuo* and the residual oil partitioned between ethyl acetate and a saturated solution of aqueous sodium bicarbonate (20ml). The aqueous phase was extracted with ethyl acetate (3 x 50ml). The combined ethyl acetate layers were washed with brine and dried over MgSO₄. The solvent was removed *in vacuo* to yield 1-[2-(2-methoxy-4-nitro-phenoxy)-ethyl]-azetidine as a pale red oil (53mg, 38 %);
¹H NMR (CDCl₃): 7.89 (dd, 1H, J=8.9), 7.73 (d, 1H), 6.89 (d, 1H), 4.09 (t, 2H), 3.93 (s, 3H), 3.35 (t, 4H), 2.91 (t, 2H), 2.13 (t, 2H).
1-[2-(2-methoxy-4-nitro-phenoxy)-ethyl]-azetidine (53.5mg, 0.21mmol) was dissolved in a 1:1 mixture of THF and ethanol (7ml) and 10% palladium on carbon paste (150mg) introduced. The reaction mixture was stirred under an atmosphere of hydrogen for 50 hours at RT. The catalyst was filtered off over filter-aid and the filtrate evaporated to give 4-[2-azetidin-1-yl-ethoxy]-3-methoxy-phenylamine as a brown oil (47mg, 100%). This material was treated with 3-phenoxyphthalic anhydride in the same manner as for 4-(2-diisopropylamino-ethoxy)-3-methoxy-phenylamine in **Example A4** to give the title compound.

### Example G12

### 2-{4-[2-(7-Aza-bicyclo[2.2.1]hept-7-yl)-ethoxy]-3-methoxy-phenyl}-5-phenoxy-isoindole-1,3-dione

7-Aza-bicyclo[2.2.1]heptane [J. Am. Chem. Soc. (1989), 111(5), 1776-81], was treated in the same manner as azetidine in **Example G11** to give the title compound.

### Example G13

### 2-{4-[2-(2-Aza-bicyclo[2.2.2]oct-2-yl)-ethoxy]-3-methoxy-phenyl}-5-phenoxy-isoindole-1,3-dione

2-Aza-bicyclo[2.2.2]octane [J. Am. Chem. Soc. (1989), 111(5), 1776-81], was treated in the same manner as azetidine in **Example G11** to give the title compound.

### Example G14

### 2-{3-Methoxy-4-[2-(4-phenyl-piperidin-1-yl)-ethoxy]-phenyl}-5-phenoxy-isoindole-1,3-dione

4-Phenylpiperidine [AstaTech] was treated in the same manner as azetidine in **Example G11** to give the title compound.

### Example G15

### 5-Phenoxy-2-(2-pyrrolidin-1-ylmethyl-2,3-dihydro-benzo[1,4]dioxin-6-yl)-isoindole-1,3-dione

2-Pyrrolidin-1-ylmethyl-2,3-dihydro-benzo[1,4]dioxin-6-ylamine [WO 0121577 A2] was treated with 3-phenoxyphthalic anhydride in the same manner as for 4-(2-diisopropylamino-ethoxy)-3-methoxy-phenylamine in **Example A4** to give the title compound.

### Example G16

### Dimethyl-[7-(5-phenoxy-1,3-dihydro-isoindol-2-yl)-chroman-3-ylmethyl]-amine

3-Dimethylaminomethyl-chroman-7-ylamine[WO 0121577 A2] was treated with 3-phenoxyphthalic anhydride in the same manner as for 4-(2-diisopropylamino-ethoxy)-3-methoxy-phenylamine in **Example A4** to give the title compound.

### Example G17

### 5-Phenoxy-2-(6-pyrrolidin-1-ylmethyl-5,6,7,8-tetrahydro-naphthalen-2-yl)-isoindole-1,3-dione

6-Pyrrolidin-1-ylmethyl-5,6,7,8-tetrahydro-naphthalen-2-ylamine [WO 0121577 A2] was treated with 3-phenoxyphthalic anhydride in the same manner as for 4-(2-diisopropylamino-ethoxy)-3-methoxy-phenylamine in Example A4 to give the title compound.

### Example G18

### 5-Phenoxy-2-(3-Pyrrolidin-1-ylmethyl-2H-chromen-7-yl)-isoindole-1,3-dione

3-Pyrrolidin-1-ylmethyl-2*H*-chromen-7-ylamine [WO 0121577 A2] was treated with 3-phenoxyphthalic anhydride in the same manner as for 4-(2-diisopropylamino-ethoxy)-3-methoxy-phenylamine in **Example A4** to give the title compound.

### Example G19

### 5-Phenoxy-2-(6-pyrrolidin-1-ylmethyl-7,8-dihydro-naphthalen-2-yl)-isoindole-1,3-dione

6-Pyrrolidin-1-ylmethyl-7,8-dihydro-naphthalen-2-ylamine [WO 0121577 A2] was treated with 3-phenoxyphthalic anhydride in the same manner as for 4-(2-diisopropylamino-ethoxy)-3-methoxy-phenylamine in **Example A4** to give the title compound.

### Example G20

### 2-{3-Methoxy-4-[3-(4-phenyl-piperidin-1-yl)-propoxy]-phenyl}-5-phenoxy-isoindole-1,3-dione

4-Phenyl-piperidine [Aldrich] was treated in the same manner as pyrrolidine in the procedure of **Example G21** to give the title compound.

### Example G21

### 2-[3-Methoxy-4-(3-pyrrolidin-1-yl-propoxy)-phenyl]-5-phenoxy-isoindole-1,3-dione

A solution of 3-bromo-1-propanol (0.685 g, 4.93mmol), 4-nitroguaiacol (1 g, 5.91mmol) and tributylphosphine (1.49 g, 7.36mmol) in 20ml tetrahydrofuran was treated with 1,1'-(azodicarbonyl)dipiperidine (1.49g,mmol), added portionwise over 5 mins, and the resulting mixture stirred for 16 hrs. The solvent was removed *in vacuo* and the residues partitioned between ethyl acetate (25ml) and 0.5 M HCl (25ml). The organic phase was washed with 0.5 M NaOH (4 x 25ml), water (25ml) and saturated brine (25ml), then dried and concentrated to a pale yellow solid. This was purified by flash chromatography on silica gel (eluting with ethyl acetate - hexane) to give 1-(3-bromo-propoxy)-2-methoxy-4-nitro-benzene as a white solid (300mg, 21 %);
¹H NMR (DMSO) δ 2.26-2.33 (m, 2H), 3.64-3.67 (t, 2H), 3.89 (s, 3H), 4.21-4.24 (t, 2H), 7.20-7.23 (d, 1H), 7.74-7.75 (d, 1H), 7.88-7.91 (dd, 1H).
This material (100mg, 0.34mmol) was treated with pyrrolidine (27mg, 0.37mmol) and potassium carbonate (96.7mg, 0.68mmol) in DMF (10ml). The mixture was heated to 70°C for 16 hrs and the resulting solution partitioned between water (25ml) and ethyl acetate (25ml). The organic phase was extracted and washed with water (10ml) and brine (5ml), then dried (MgSO₄) and concentrated to yield 1-[3-(2-methoxy-4-nitro-phenoxy)-propyl]-pyrrolidine as a yellow oil (90mg, 94%);
¹H-NMR (DMSO) δ1.66-1.70 (m, 4H), 1.89-1.96 (m, 2H), 2.44-2.55 (m 6H), 3.88(s, 3H), 4.14-4.18 (t, 2H), 7.17-7.19 (d, 1H), 7.73-7.75 (d, 1H), 7.87-7.9 (dd, 2H).
This amine (90mg, 0.32mmol), was dissolved in ethanol (5ml) and treated with 10% palladium on carbon (40mg) then the mixture stirred under a hydrogen atmosphere for 16 hrs. The mixture was filtered through a plug of celite and the filtrate concentrated to yield 3-methoxy-4-(3-pyrrolidin-1-yl-propoxy)-phenylamine as a brown oil (75mg, 93 %);
¹H-NMR (DMSO) δ 1.66-1.69(m, 4H), 1.76-1.79(m, 2H), 2.44(bm, 6H), 3.66(s, 3H), 3.79-3.82(t, 2H), 4,64(bs, 2H), 6.02-6.05(dd, 1H), 6.24-6.25(d, 1H), 6.61-6.63(d, 1H). This aniline was treated with 3-phenoxyphthalic anhydride in the same manner as for 4-(2-diisopropylamino-ethoxy)-3-methoxy-phenylamine in **Example A4** to give the title compound.

### Example G22

### 2-{3-Methoxy-4-[3-(4-methyl-piperazin-1-yl)-propoxy]-phenyl}-5-phenoxy-isoindole-1,3-dione

N-methyl-piperazine [Aldrich] was treated in the same manner as pyrrolidine in the procedure of **Example G21** to give the title compound.

### Example G23

### 2-[4-(3-Azepan-1-yl-propoxy)-3-methoxy-phenyl]-5-phenoxy-isoindole-1,3-dione

Hexamethyleneamine [Aldrich] was treated in the same manner as pyrrolidine in the procedure of **Example G21** to give the title compound.

### Example G24

### 2-[3-Methoxy-4-(3-morpholin-4-yl-propoxy)-phenyl]-5-phenoxy-isoindole-1,3-dione

Morpholine [Aldrich] was treated in the same manner as pyrrolidine in the procedure of **Example G21** to give the title compound.

The following tables give examples which illustrate but do not limit the invention in any way.

### Table A

Encompassing compounds of general formula (A), a subset of formula (I) where QY = a phthalimide group, R⁶ = OMe and ML is an oxyethyl group

| | | | | |
|---|---|---|---|---|
| 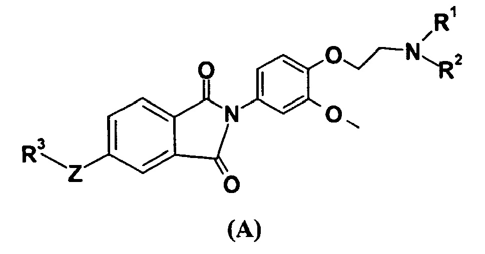 | | | | |

| Example No. | 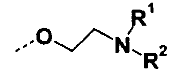 | Z | R3 | [M+H] + (AP+) |
|---|---|---|---|---|
| A1 | 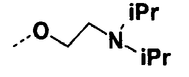 | bond | Ph | 473 |
| A2 | 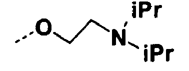 | CH₂ | Ph | 487 |
| A3 | 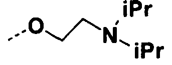 | NH | Ph | 488 |
| A4 | 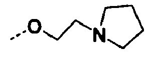 | O | Ph | 459 |
| A5 | 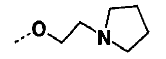 | NH | Ph | 458 |
| A6 | 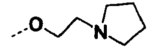 | CH₂ | Ph | 457 |
| A7 | 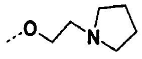 | S | Ph | 475 |
| A8 | 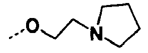 | S | p-Cl-Ph | 509, 511 |
| A9 | 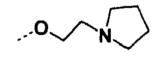 | S | 3-MeO-Ph | 505 |
| A10 | 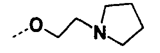 | S | 4-HO-Ph | 491 |
| A11 | 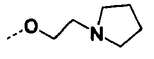 | S | 4-F-Ph | 493 |
| A12 | 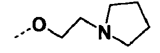 | S | 4-H₂N-Ph | 490 |
| A13 | 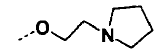 | S | 4-F₃C-Ph | 543 |
| A14 | 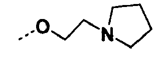 | S | 4-MeO-Ph | 505 |
| A15 | 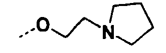 | O | 4-Cl-Ph | 493, 495 |
| A16 | 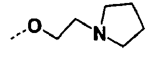 | O | 4-F-Ph | 477 |
| A17 | 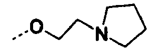 | O | 4-F₃C-Ph | 527 |
| A18 | 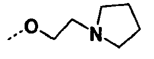 | O | 4-F-3-Cl-Ph | 561,563 |
| A19 | 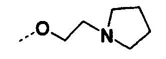 | O | 3-Fl-Ph | 477 |
| A20 | 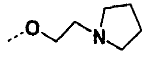 | O | 3-Cl-Ph | 493, 495 |
| A21 | 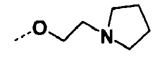 | O | 3-^{t}Bu-Ph | 515 |
| A22 | 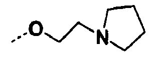 | O | 3-MeO-Ph | 489 |
| A23 | 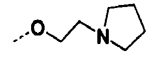 | O | 3-Me-Ph | 473 |
| A24 | 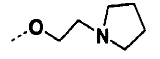 | O | 4-Me-Ph | 473 |
| A25 | 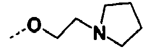 | O | 4-NC-Ph | 484 |
| A26 | 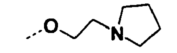 | O | 2-NC-Ph | 484 |
| A27 | 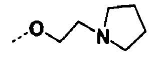 | O | 2-MeS-Ph | 505 |
| A28 | 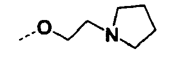 | O | 2-Br-Ph | 537, 539 |
| A29 | 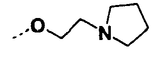 | O | 2-Cl-Ph | 493, 495 |
| A30 | 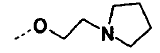 | O | 2-F-Ph | 477 |
| A31 | 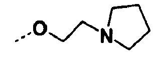 | O | 2-Et-Ph | 487 |
| A32 | 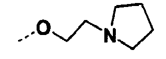 | O | 2,5-diF-Ph | 495 |
| A33 | 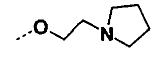 | O | 2-F₃C-Ph | 527 |
| A34 | 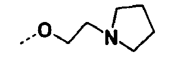 | O | 2,4-diF-Ph | 495 |
| A35 | 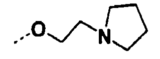 | O | 4-F-2-MeO-Ph | 507 |
| A36 | 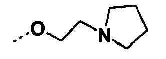 | O | 2-MeO-Ph | 489 |
| A37 | 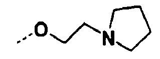 | O | 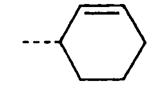 | 463 |
| A38 | 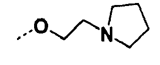 | O | 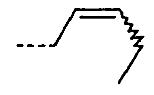 | 451 |
| A39 | 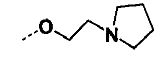 | O | 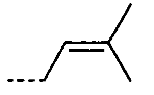 | 451 |
| A40 | 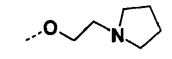 | O | 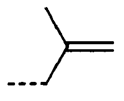 | 437 |
| A41 | 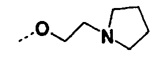 | O | 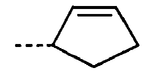 | 449 |
| A42 | 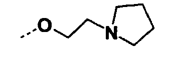 | O | 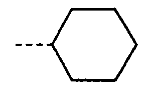 | 465 |
| A43 | 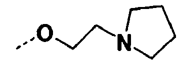 | O | 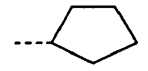 | 451 |

### Table B

Encompassing compounds of general formula **B,** a subset of formula (I) where QY = a benzene ring fused onto a 5-membered heterocycle composed of groups J, J' and J" and linked via J"; R⁶ = OMe, ML is an oxyethyl group and R³ = Ph

| | | | | | | |
|---|---|---|---|---|---|---|
| 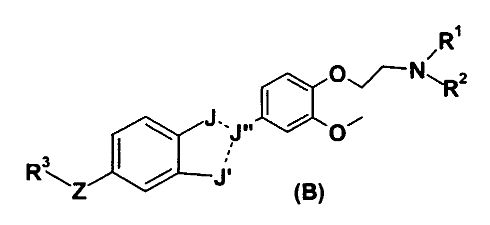 | | | | | | |

| Example No. | 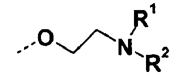 | Z | J | J' | J" | [M+H]⁺ [AP+, 100%] |
|---|---|---|---|---|---|---|
| B1 | 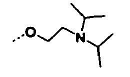 | bond | C=O | CH₂ | N | 459 |
| B2 | 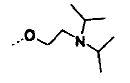 | bond | CH₂ | C=O | N | 459 |
| B3 | 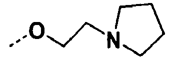 | O | C=O | CH₂ | N | 445 |

### Table C

Encompassing compounds of general formula (**C**), a subset of formula (I) where QY = a benzene ring fused onto a 6-membered heterocycle composed of groups J, J', J" and J''' and linked via J'''; ML is an oxyethyl group and NR¹R² form a pyrrolidine ring.

| | | | | | | | |
|---|---|---|---|---|---|---|---|
| 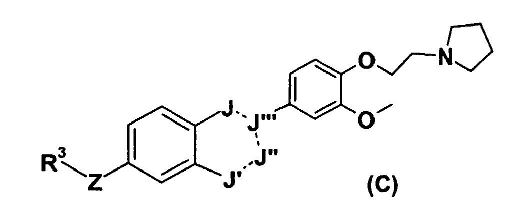 | | | | | | | |

| Example No. | R³ | Z | J | J' | J" | J"' | [M+H]⁺ [AP+, 100%] |
|---|---|---|---|---|---|---|---|
| C1 | Ph | O | C=O | N= | =N- | N | 459 |
| C2 | Ph | O | C=O | N= | =CH- | N | 458 |
| C3 | Ph | O | C=O | NH- | C=O | N | 474 |
| C4 | Ph | O | C=O | N= | =CMe- | N | 472 |
| C5 | Ph | bond | C=O | N= | =N- | N | 443 |
| C6 | Ph | bond | C=O | N= | =CH- | N | 442 |
| C7 | Ph | bond | C=O | NH- | C=O | N | 458 |
| C8 | Ph | bond | C=O | -N= | =CMe- | N | 456 |
| C9 | Allyl | O | C=O | N= | =CH- | N | 422 |

### Table D

Encompassing compounds of general formula (**D**), a subset of formula (I) where QY = a benzene ring fused onto a 6-membered heterocycle composed of groups J, J', J" and J''' and linked via J'"; ML is an oxyethyl group, R⁶ = OMe

| | | | | | | | | |
|---|---|---|---|---|---|---|---|---|
| 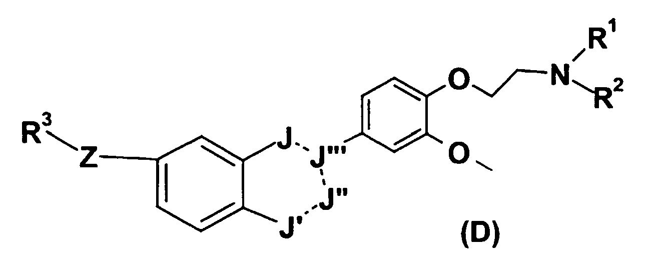 | | | | | | | | |

| Example No. | 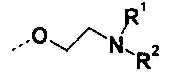 | R³ | Z | J | J' | J'' | J''' | [M+H]⁺ [AP+] |
|---|---|---|---|---|---|---|---|---|
| D1 | 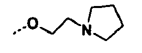 | Ph | O | C=O | N= | =N- | N | 459 |
| D2 | 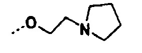 | Ph | O | C=O | N= | =CH- | N | 458 |
| D3 | 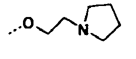 | Ph | O | C=O | NH- | C=O | N | 474 |
| D4 | 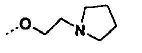 | Ph | O | C=O | N= | =CMe- | N | 472 |
| D5 | 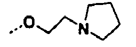 | 4-ClPh | bond | C=O | N= | =CH- | N | 476,478 |

### Table E

Encompassing compounds of general formula (**E**), a subset of formula (I) where QY = a thiophene ring fused onto a 6-membered heterocycle composed of groups J, J', J" and J''' and linked via J'''; ML is an oxyethyl group, R³ = Ph; R⁶ = OMe, Z = a bond

| | | | | | | |
|---|---|---|---|---|---|---|
| 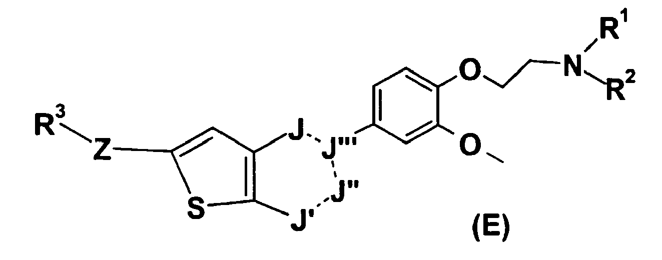 | | | | | | |

| Example No. | 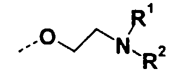 | A | A' | A" | A''' | [M+H]⁺ [AP+, 100%] |
|---|---|---|---|---|---|---|
| E1 | 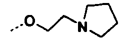 | C=O | N= | =N- | N | 449 |
| E2 | 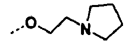 | C=O | N= | =CH- | N | 448 |

### Table F

Encompassing compounds of general formula (**F**), a subset of formula (I) where QY = a thiophene ring fused onto a 6-membered heterocycle composed of groups J, J', J" and J''' and linked via J'''; ML is an oxyethyl group, R³ = Ph; R⁶ = OMe; Z = a bond

| | | | | | | |
|---|---|---|---|---|---|---|
| 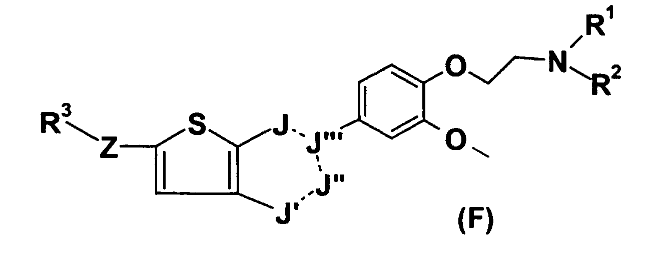 | | | | | | |

| Example No. | 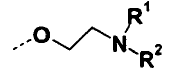 | J | J' | J" | J'" | [M+H]⁺ [AP+, 100%] |
|---|---|---|---|---|---|---|
| F1 | 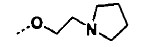 | C=O | N= | =CH- | N | 448 |
| F2 | 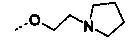 | C=O | N= | =C(SMe)- | N | 494 |
| F3 | 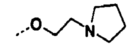 | C=O | NH | C=S | N | 480 |

### Table G

Encompassing compounds of general formula (**G**), a subset of formula (I) where QY = a phthalimide group and Z is O and R³ = Ph (=phenyl).

| | | | | |
|---|---|---|---|---|
| 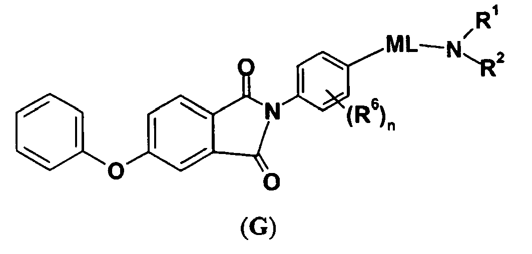 | | | | |

| Example No. | 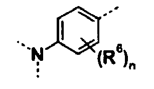 | ML | R²-N-R¹ | [M+H]⁺ AP+ |
|---|---|---|---|---|
| G1 | 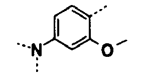 | -(CH2)3- | 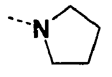 | 457 |
| G2 | 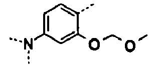 | -O-(CH₂)₂- | 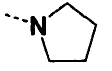 | 489 |
| G3 | 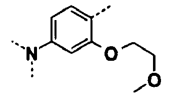 | -O-(CH₂)₂- | 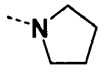 | 503 |
| G4 | 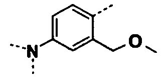 | -O-(CH₂)₂- | 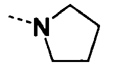 | 473 |
| G5 | 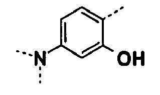 | -O-(CH₂)₂- | 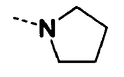 | 445 |
| G6 | 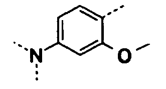 | -O-(CH₂)₂- | 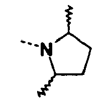 | 487 |
| G7 | 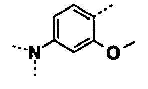 | -O-(CH₂)₂- | 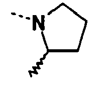 | 473 |
| G8 | 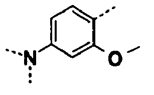 | -O-(CH₂)₂- | 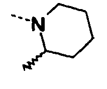 | 487 |
| G9 | 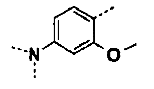 | -O-(CH₂)₂- | 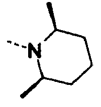 | 501 |
| G10 | 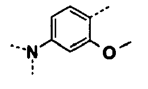 | -O-(CH₂)₂- | 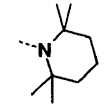 | 529 |
| G11 | 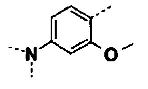 | -O-(CH₂)₂- | 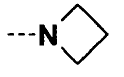 | 445 |
| G12 | 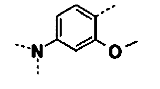 | -O-(CH₂)₂- | 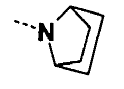 | 485 |
| G13 | 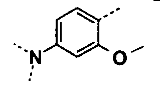 | -O-(CH₂)₂- | 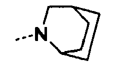 | 499 |
| G14 | 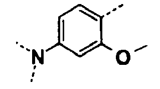 | -O-(CH₂)₂- | 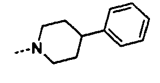 | 549 |
| G15 | 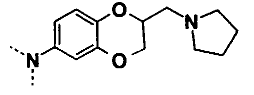 | | | 457 |
| G16 | 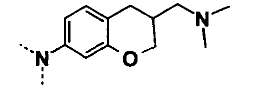 | | | 455 |
| G17 | 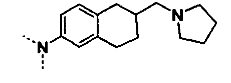 | | | 453 |
| G18 | 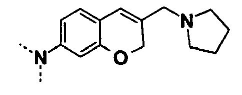 | | | 453 |
| G19 | 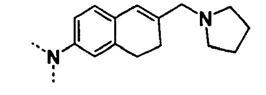 | | | 451 |
| G20 | 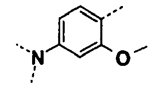 | -O-(CH₂)₃- | 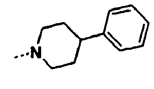 | 563 |
| G21 | 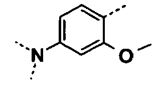 | -O-(CH₂)₃- | 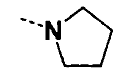 | 473 |
| G22 | 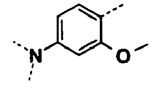 | -O-(CH₂)₃- | 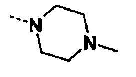 | 502 |
| G23 | 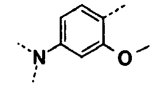 | -O-(CH₂)₃- | 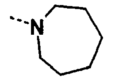 | 501 |
| G24 | 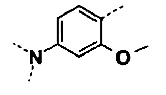 | -O-(CH₂)₃- | 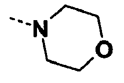 | 489 |

Examples given show a pKi in binding to the 353 form of the 11CBy receptor of>6; the most potent examples would have a pKi in the range 7.5-8, for example A5, C5, F1.

The activity of the compounds used in this invention has been assessed by competitive binding assays to 11 CBy receptors, as follows:

### Radioligand Binding Studies

Radioligand binding assays were carried out on well washed membranes from HEK293 cells stably expressing 11CBy receptors. Membranes (5-15 mg protein) were incubated with [¹²⁵I]-Melanin Concentrating Hormone (0.22 nM)(obtained from NEN) in the presence and absence of competing test compounds for 45 min at 37°C in a buffer (pH7.4), containing 50mM Tris and 0.2% BSA. Non-specific binding was defined using 0.1 mM Melanin Concentrating Hormone (obtained from Bachem). The test compounds were added at concentrations between 10M and 10pM in 10 concentration steps. Following incubation, the reaction was stopped by filtration through GF/B filters and washed with 4 x 1ml of ice-cold 50mM Tris buffer. Microscint 20 (Packard) was added to the filters and the radioactivity measured using a Packard TopCount.

Bound cpm in the presence of test compound was expressed as a fraction of the bound cpm in the absence of test compound and plotted against the concentration of compound. From this an IC50 was determined from which the pKi was calculated.

The compounds described in Examples have a pKi value of greater than 6. For example, the compounds of examples A5, C5 and F1 have a pKi in the range 7.5-8.

### Study of Effects of 11CBy Antagonists on Plasma Glucagon and Blood Glucose Levels

In chronically femoral artery and vein cannulated conscious CD rats, MCH @ 50 ug/kg (iv) produced 48.7% and 21 % increases in plasma glucagon from the pretreatment value after 5 and 15 minutes respectively. Glucagonotropic effects of MCH produced a maximum rise in blood glucose concentration after 15 min (101%).

Intravenous administration of the title compound from Example A4 (pKi = 7.7) at a level of 15 mg/kg five minutes prior to iv bolus injection in the rat glucagon secretion model reduced the rise in plasma glucagon to 2.7% after 15 min with no rise after 5 minutes, while blood glucose increased by only 44%

**Table 1. Plasma glucagon (pg/ml) and blood glucose levels (mmol/l) in conscious CD rats**

| Plasma glucagon | | | |
|---|---|---|---|
| Groups | 0 | 5 min | 15min |
| Vehicle (1ml/kg) | 26.3±1.6 (6) | 29.35±3.7(7) | 25.74±1.3(7) |
| MCH (50ug/kg) | 23.6±1.2(6) | 35.16±2.5(7) | 28.67±1.61(7) |
| Example A4 (15 mg/kg) + MCH | 23.45±1.16(7) | 30.85±2.01(7) | 22.87±0.56(5) |
| | | | |

| Blood glucose | | | |
|---|---|---|---|
| Groups | 0 | 5 min | 15min |
| Vehicle (1ml/kg) | 6.06±0.11 (7) | 6.14±0.23 (7) | 6.3±0.25(7) |
| MCH (50ug/kg) | 5.87±0.22 (7) | 6.5±0.33 (7) | 11.81±1.27(7) |
| Example A4 (15 mg/kg) + MCH | 5.35±0.23 (7) | 6.46±0.46(7) | 7.71±0.43 (7) |

| | | | |
|---|---|---|---|
| Parenthesis = animal numbers. Example A4 given alone produced no change in either plasma glucagon and blood glucose levels. Plasma glucagon determined by commercial RIA kit. | | | |

## Claims

1. A compound of formula (I) comprising: 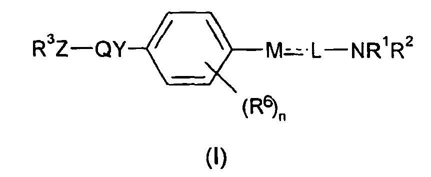 a salt or solvate thereof , wherein
M is O or CH₂;
L is a 2- or 3- membered alkylene chain which may be linked to the phenyl ring of formula (I) via an R⁶ substituent located in the ortho position relative to the group M to form a bicyclic structure;
wherein together M-L may be optionally substituted by at least one moiety selected from the group consisting of methyl, ethyl, hydroxy and C₁₋₃ alkoxy;
(i) R¹ and R² are each independently selected from the group consisting of hydrogen, C₁₋₆ straight or branched alkyl which may be optionally substituted by phenyl, and C₃₋₆ cycloalkyl optionally substituted by one or more C₁₋₆ alkyl groups;
or (ii) R¹ and R² together with the nitrogen atom to which they are bonded form a 4-8 membered heterocyclic ring or a 7-10 membered bicyclic heterocyclic ring containing 1, 2" 3, or 4 heteroatoms selected from N, S, and O, wherein said 4 to 8 membered heterocyclic ring and said 7 to 10 membered bicyclic heterocyclic ring are optionally substituted with a substituent selected from the group consisting of phenyl and from one to four C₁₋₃ alkyl;
each R⁶ is the same or different and is independently selected from the group consisting of hydroxy, C₁₋₂alkyl, C₁₋₃alkoxy, halo, C₂₋₃alkenyl, benzyl, and -C(R^{a})NOR^{b}, wherein R^{a} and R^{b} are each independently selected from the group consisting of hydrogen, methyl, methoxymethyl, methoxymethoxy, and methoxyethoxy and n is 1, 2, 3, or 4;
QY is a bicyclic fused heterocyclic ring wherein Q and Y are each one ring of said bicyclic fused heterocyclic group, wherein said Y ring contains from 1 to 3 nitrogens and is bound to the phenyl ring of formula (I) via a nitrogen atom, and said Q ring is a 5- or 6- membered aryl or heterocyclic ring having a group ZR³; Z is bound to the Q ring;
Z is selected from the group consisting of a direct bond, NH, NCH₃, O, S, and CH₂; and
R³ is a group selected from the group consisting of aryl, alk-2-en-1-yl, cycloalkyl and cycloalk-2-en-1-yl and wherein said aryl, alk-2-en-1-yl, cycloalkyl, and cycloalk-2-en-1-yl are optionally substituted from 1 to 3 times with a subsituent selected from the group consisting of C₁₋₃ alkyl, halo, amino, alkylamino, dialkylamino, hydroxy, C₁₋₃ alkoxy, cyano, trifluoromethyl, and methylthio groups.

2. The compound of claim 1 wherein M is O.

3. The compound of claims 1 - 2 wherein L is (CH₂)₂.

4. The compound of claims 1 - 3 wherein L is linked to the phenyl ring of formula (I) via an R⁶ substituent located in the ortho position relative to the group M to form a bicyclic structure.

5. The compound of claim 4 wherein said bicyclic structure is selected from the group consisting of 1,4-benzodioxan, benzopyran, 1,2-dihydrobenzopyran, 1,2,3,4-tetrahydronaphthalene, and 1,2-dihydronaphthalene.

6. The compound of claims 1 - 5 wherein (i) R¹ and R² independently are a C₁₋₆ alkyl.

7. The compound of claim 6 wherein R¹ and R² independently are methyl and isopropyl.

8. The compound of claims 1 - 5 wherein (ii) when R¹ and R² together with the nitrogen atom to which they are bonded form a 4- to 8-membered heterocyclic ring.

9. The compound of claim 8 wherein NR¹R² represents a 5- or 6- membered heterocyclic group optionally substituted by 1 to 4 methyl groups or a phenyl group.

10. The compound of claim 8 or 9 wherein NR¹R² represents a pyrrolidinyl group.

11. The compound of claims 1 - 10 wherein QY is a 5,5; 5,6; 6,5; or 6,6 bicyclic fused heterocyclic ring.

12. The compound of claim 12 wherein QY is a group selected from isoindole-1,3-dione, 2,3-dihydro-isoindole-1-one, 3-H-benzo[d](1,2,3)triazin-4-one, 3-H-quinazolin-4-one, 1-H-quinazoline-2,4-dione, 3-H-thieno[2,3-d]-1-triazine-4-one, 3-H-thieno[2,3d]pyrimidin-4-one, 3-H-thieno[3,2-d]pyrimidin-4-one, 2-thioxo-2,3-dihydro-1-H-thieno[3,2-d]pyrimidine-4-one, 2-methylthio-3H-thieno[3,2-d] pyrimidin-4-one.

13. The compound of claims 1 - 12 wherein at least one R⁶ is in the ortho position with respect to the group M.

14. The compound of claims 1 - 13 wherein R⁶ is methoxy.

15. The compound of claims 1 - 14 wherein Z is a bond or an oxygen atom.

16. The compound of claims 1 - 15 wherein R³ is selected from the group consisting of (i) phenyl which is optionally substituted by halogen, hydroxy, C₁₋₃ alkoxy, C₁₋₄ alkyl, cyano or trifluoromethyl, (ii) C₅₋₇ cycloakyl, (iii) C₅₋₇ 2-cycloakenyl, and (iv) C₃₋₆ alk-2-en-1-yl.

17. The compound of claims 1 - 16 wherein R³ represents phenyl.

18. 3-[3-Methoxy-4-(2-pyrrolidin-1-yl-ethyl)-phenyl]-7-phenyl-3H-benzo-[d][1,2,3]triazin-4-one and physiologically acceptable acid addition salts thereof.

19. 3-[3-Methoxy-4-(2-pyrrolidin-1-yl-ethoxy)-phenyl]-6-phenyl-3H-thieno-[3,2-d]pyrimidin-4-one and physiologically acceptable acid addition salts thereof.

20. A pharmaceutical composition comprising a compound of claims 1 -19 or a physiologically acceptable salt or solvate thereof and one or more pharmaceutically acceptable carriers or excipients.

21. A compound of claims 1 -19 or a physiologically acceptable salt or solvate thereof for use in therapy.

22. The use of a compound of claims 1-19 in the manufacture of a therapeutic agent for the treatment of one or more of bacterial, fungal, protozoan and viral infections, infection caused by HIV-1 or HIV-2; pain; cancers; diabetes; obesity; feeding abnormalities, such as anorexia and bulima; drinking abnormalities; asthma; Parkinson's disease; both acute and congestive heart failure; hypertension; urinary retention; osteoporosis; angina pectoris; myocardial infarction; ulcers; allergies; benign prostatic hypertrophy; psychotic and neurological disorders, including anxiety, schizophrenia, depression (major), manic depression bi-polar depression), delirium, dementia, and/or severe mental retardation; and dyskinesias (such as Huntington's disease or Gilles de la Tourette's syndrome).

23. Use according to claim 22 for the treatment of obesity, diabetes, major depression, schizophrenia, sleep disorders and anxiety.

24. A process for preparing a compound according to claim 1 wherein Y is a 5 membered ring containing two carbonyl groups comprising reacting an aniline of formula (II) 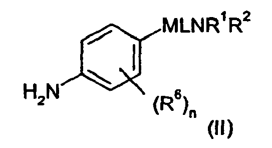 or a protected derivative thereof with an anhydride of formula (III) 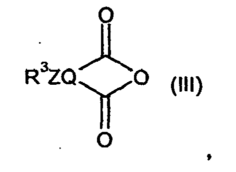 an ortrhodicarboxylic acid of formula (IV) 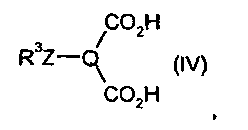 or an imide of formula (V) 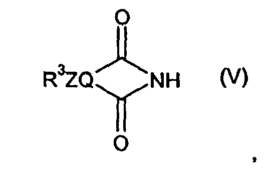 wherein R¹, R², R³, R⁶, Z, Q, Y, M, and L are as defined in formula (I).

25. A process for preparing a compound according to claim 1 wherein Y is a 5-membered ring containing a single carbonyl group comprising reducing a compound of formula (I) in which Y is a 5-membered ring containing 2 carbonyl groups.

26. A process for preparing a compound according to claim 1 wherein Y is a 6-membered heterocycle comprising cyclisation of a compound of formula (VI) 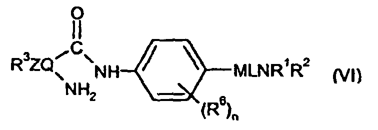 wherein R³, Z, Q, R⁶, n, M, L, R¹ and R² have the meanings defined in formula (I).

27. A process for preparing a compound according to claim 1 wherein Y is a pyrimidine-2,4-dione comprising reacting an amine of formula (VI) 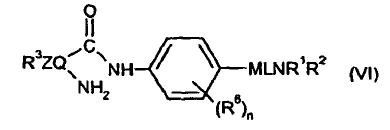 with a carboxylating agent and a base in an aprotic solvent and wherein R³, Z, Q, R⁶, n, M, L, R¹ and R² have the meanings defined in formula (I).

28. A process for preparing a compound according to claim 1 wherein Y is 1,2,3-triazin-4-one comprising diazotisaton of a compound of formula (VI) 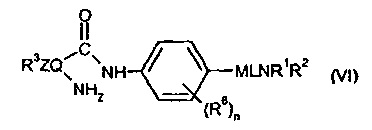 followed by treatment with a base and wherein R³ Z, Q, R⁶, n, M, L, R¹ and R² have the meanings defined in formula (I).

29. A process for preparing a compound according to claim 1 wherein Y is a pyrimidinone ring containing a thiocarbonyl group at the 2 position, comprising reacting a thiourea of formula (VII) 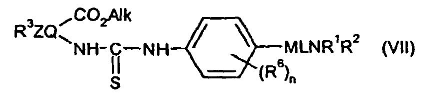 or a protected derivative thereof by heating said thiourea in a high boiling solvent and wherein R³, Z, Q, R⁶, n, M, L, R¹ and R² have the meanings defined in formula (I), and Alk is C₁₋₄alkyl.

30. A process for preparing a compound according to claim 1 wherein Y is a pyrimidinone having an alkylthio group in the 2 position comprising reacting a thiourea of formula (VII) 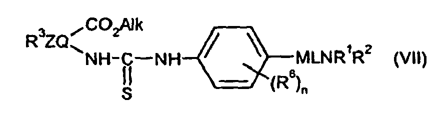 with an alkyl halide in a protic polar solvent and wherein R³, Z, Q, R⁶, n, M, L, R¹ and R² have the meanings defined in formula (I) or are protected derivatives thereof and Alk is C₁₋₄alkyl.

31. A process for preparing a compound according to claim 1 wherein Y is a pyridimidinone derivative, unsubstituted at the 2 position comprising reacting a corresponding pyrimidinone having an alkylthio group at the 2 position with Raney nickel in a solvent.

32. A process for preparing a compound according to claim 1 comprising reacting a compound of formula (VIII), 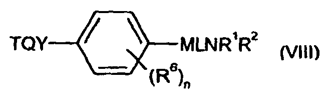 in which T is a leaving group, with a compound capable of introducing the group R³Z, and wherein Q, Y, R⁶, n, M, L, R¹ and R² have the meanings defined in formula (I).

33. A process for preparing a compound according to claim 1 wherein Z is a bond and R³ is an aryl group comprising reacting a compound of formula (VIII) in which T is a leaving group 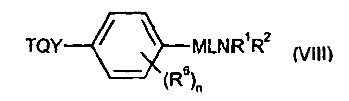 with (i) an arylboronic acid in a Suzuki coupling reaction or (ii) with an aryl-tin reagent in a Stille displacement reaction wherein Q, Y, R6, N, M, L, R2, and R2 have the meanings defined in formula (I).

34. A process for preparing a compound according to claim 1 wherein Z is a methylene group comprising treating a compound of formula (VIII) in which T is a leaving group 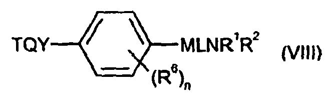 with an organo zinc or an organo mangesium compound in the presence of a palladium catalyst, and wherein Q, Y, R⁶, n, M, L, R¹ and R² have the meanings defined in formula (I).

35. A process for preparing a compound according to claim 1 where Z is NH or NCH₃ comprising reacting a compound of formula (VIII) in which T is a leaving group 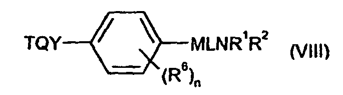 with an amine in the presence of a palladium catalyst under standard Buchwald conditions, and wherein Q, Y, R⁶, n, M, L, R¹ and R² have the meanings defined in formula (I).

36. A process for preparing a compound according to claim 1 wherein Z is sulfur comprising reacting a compound of formula (VIII) in which T is a leaving group 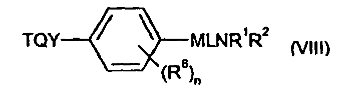 with a thiol in the presence of a base, and wherein Q, Y, R⁶, n, M, L, R¹ and R² have the meanings defined in formula (I).

37. A process for preparing a compound according to claim 1 wherein Z is oxygen comprising reacting a compound of formula (VIII) in which T is a leaving group 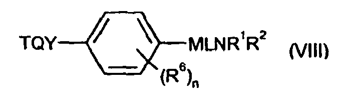 with an alcohol in the presence of an alkali metal hydride and an aprotic solvent, and wherein Q, Y, R⁶, n, M, L, R¹ and R² have the meanings defined in formula (I).

38. The use of a compound of claim 1 for the manufacture of a medicament to inhibit MCH-induced hyperglycemia by reducing MCH-induced hyperglucagonemia.

## Patentansprüche

1. Verbindung der Formel (I), umfassend: 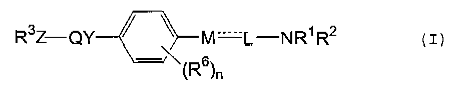 ein Salz oder Solvat davon, worin:
M O oder CH₂ ist;
L eine 2- oder 3-gliedrige Alkylenkette ist, die an den Phenylring der Formel (I) über einen R⁶-Substituenten gebunden sein kann, der sich in der ortho-Stellung relativ zur Gruppe M befindet, unter Bildung einer bicyclischen Struktur;
worin M-L zusammen gegebenenfalls mit wenigstens einer Einheit substituiert sein können, die aus der Gruppe ausgewählt ist, die aus Methyl, Ethyl, Hydroxy und C₁₋₃-Alkoxy besteht;
(i) R¹ und R² jeweils unabhängig aus der Gruppe ausgewählt sind, die aus Wasserstoff, linearem oder verzweigtem C₁₋₆-Alkyl, das gegebenenfalls mit Phenyl substituiert sein kann, und gegebenenfalls mit einer oder mehreren C₁₋₆-Alkylgruppen substituiertem C₃₋₆-Cycloalkyl besteht;
oder (ii) R¹ und R² zusammen mit dem Stickstoffatom, an das sie gebunden sind, einen 4- bis 8-gliedrigen heterocyclischen Ring oder einen 7- bis 10-gliedrigen bicyclischen heterocyclischen Ring bilden, der 1, 2, 3 oder 4 Heteroatome enthält, die aus N, S und O ausgewählt sind, worin der 4- bis 8-gliedrige heterocyclische Ring und der 7- bis 10-gliedrige bicyclische heterocyclische Ring gegebenenfalls mit einem Substituenten substituiert sind, der aus der Gruppe, die aus Phenyl besteht, und aus einem bis vier C₁₋₃-Alkyl ausgewählt ist;
R⁶ jeweils gleich oder verschieden ist und unabhängig aus der Gruppe ausgewählt ist, die aus Hydroxy, C₁₋₂-Alkyl, C₁₋₃-Alkoxy, Halogen, C₂₋₃-Alkenyl, Benzyl und -C(R^{a})NOR^{b} besteht, worin R^{a} und R^{b} jeweils unabhängig aus der Gruppe ausgewählt sind, die aus Wasserstoff, Methyl, Methoxymethyl, Methoxymethoxy und Methoxyethoxy besteht, und n 1, 2, 3 oder 4 ist;
QY ein bicyclischer kondensierter heterocyclischer Ring ist, worin Q und Y jeweils ein Ring der bicyclischen kondensierten heterocyclischen Gruppe sind, worin der Y-Ring 1 bis 3 Stickstoffe enthält und an den Phenylring der Formel (I) über ein Stickstoffatom gebunden ist und der Q-Ring ein 5- oder 6-gliedriger Aryl- oder heterocyclischer Ring mit einer Gruppe ZR³ ist; Z an den Q-Ring gebunden ist;
Z aus der Gruppe ausgewählt ist, die aus einer direkten Bindung, NH, NCH₃, O, S und CH₂ besteht; und
R³ eine Gruppe ist, die aus der Gruppe ausgewählt ist, die aus Aryl, Alk-2-en-1-yl, Cycloalkyl und Cycloalk-2-en-1-yl besteht, worin das Aryl, Alk-2-en-1-yl, Cycloalkyl und Cycloalk-2-en-1-yl gegebenenfalls 1- bis 3-mal mit einem Substituenten substituiert sind, der aus der Gruppe ausgewählt ist, die aus C₁₋₃-Alkyl-, Halogen-, Amino-, Alkylamino-, Dialkylamino-, Hydroxy-, C₁₋₃-Alkoxy-, Cyano-, Trifluormethyl- und Methylthiogruppen besteht.

2. Verbindung gemäß Anspruch 1, worin M O ist.

3. Verbindung gemäß Ansprüchen 1 bis 2, worin L (CH₂)₂ ist.

4. Verbindung gemäß Ansprüchen 1 bis 3, worin L an den Phenylring der Formel (I) über einen R⁶-Substituenten gebunden ist, der sich in der ortho-Stellung relativ zur Gruppe M befindet, unter Bildung einer bicyclischen Struktur.

5. Verbindung gemäß Anspruch 4, worin die bicyclische Struktur aus der Gruppe ausgewählt ist, die aus 1,4-Henzodioxan, Benzopyran, 1,2-Dihydrobenzopyran, 1,2,3,4-Tetrahydronaphthalin und 1,2-Dihydronaphthalin besteht.

6. Verbindung gemäß Ansprüchen 1 bis 5, worin (i) R¹ und R² unabhängig C₁₋₆-Alkyl sind.

7. Verbindung gemäß Anspruch 6, worin R¹ und R² unabhängig Methyl und Isopropyl sind.

8. Verbindung gemäß Ansprüchen 1 bis 5, worin (ii) R¹ und R² zusammen mit dem Stickstoffatom, an das sie gebunden sind, einen 4- bis 8-gliedrigen heterocyclischen Ring bilden.

9. Verbindung gemäß Anspruch 8, worin NR¹R² eine 5- oder 6-gliedrige heterocyclische Gruppe darstellt, die gegebenenfalls mit 1 bis 4 Methylgruppen oder einer Phenylgruppe substituiert ist.

10. Verbindung gemäß Anspruch 8 oder 9, worin NR¹R² eine Pyrrolidinylgruppe darstellt.

11. Verbindung gemäß Ansprüchen 1 bis 10, worin QY ein 5,5-, 5,6-, 6,5- oder 6,6-bicyclischer kondensierter heterocyclischer Ring ist.

12. Verbindung gemäß Anspruch 11, worin QY eine Gruppe ist, aus Isoindol-1,3-dion, 2,3-Dihydro-isoindol-1-on, 3-H-Benzo[d](1,2,3)triazin-4-on, 3-H-Chinazolin-4-on, 1-H-Chinazolin-2,4-dion, 3-H-Thierio[2,3-d]-1-triazin-4-on, 3-H-Thieno[2,3-d]pyrimidin-4-on, 3-H-Thieno[3,2-d]pyrimidin-4-on, 2-Thioxo-2,3-dihydro-1-H-thieno[3,2-d]pyrimidin-4-on und 2-Methylthio-3H-thieno[3,2-d]pyrimidin-4-on ausgewählt ist.

13. Verbindung gemäß Ansprüchen 1 bis 12, worin wenigstens ein R⁶ in der ortho-Stellung in Bezug auf die Gruppe M ist.

14. Verbindung gemäß Ansprüchen 1 bis 13, worin R⁶ Methoxy ist.

15. Verbindung gemäß Ansprüchen 1 bis 14, worin z eine Bindung oder ein Sauerstoffatom ist.

16. Verbindung gemäß Ansprüchen 1 bis 15, worin R³ aus der Gruppe ausgewählt ist, die aus (i) Phenyl, das gegebenenfalls mit Halogen, Hydroxy, C₁₋₃-Alkoxy, C₁₋₄-Alkyl, Cyano oder Trifluormethyl substituiert ist, (ii) C₅₋₇-Cycloalkyl, (iii) C₅₋₇-Cycloalkenyl und (iv) C₃₋₆-Alk-2-en-1-yl besteht.

17. Verbindung gemäß Ansprüchen 1 bis 16, worin R³ Phenyl darstellt.

18. 3-[3-Methoxy-4-(2-pyrrolidin-1-yl-ethyl)phenyl]-7-phenyl-3H-benzo-[d][1,2,3]triazin-4-on und physiologisch akzeptable Säureadditionssalze davon.

19. 3-[3-Methoxy-4-(2-pyrrolidin-l-yl-ethoxy)phenyl]-6-phenyl-3H-thieno-[3,2-d]pyrimidin-4-on und physiologisch akzeptable Säureadditionssalze davon.

20. Pharmazeutische Zusammensetzung, die eine Verbindung gemäß Ansprüchen 1 bis 19 oder ein physiologisch akzeptables Salz oder Solvat davon und einen oder mehrere pharmazeutisch akzeptable Träger oder Exzipienten umfaßt.

21. Verbindung gemäß Ansprüchen 1 bis 19 oder physiologisch akzeptables Salz oder Solvat davon zur Verwendung in der Therapie.

22. Verwendung einer Verbindung gemäß Ansprüchen 1 bis 19 in der Herstellung eines Therapeutikums zur Behandlung von einem oder mehreren aus bakteriellen, pilzlichen, Protozoen- und viralen Infektionen; durch HIV-1 oder HIV-2 verursachter Infektion; Schmerz; Krebs; Diabetes; Fettsucht; Ernährungsabnormalitäten wie Anorexie und Bulimie; Trinkabnormalitäten; Asthma; Parkinson-Krankheit; sowohl akuter als auch Stauungs-Herzinsuffizienz; Hypertonie; Harnverhaltung; Osteoporose; Angina pectoris; Myokardinfarkt; Geschwüren; Allergien; gutartiger Prostatahypertrophie; psychotischen und neurologischen Störungen, einschließlich Angstzustand, Schizophrenie, Depression (major), manische Depression bipolare Depression, Delirium, Demenz und/oder schwere mentale Retardierung; und Dyskinesien (wie Huntington-Krankheit und Gilles de la Tourette-Syndrom).

23. Verwendung gemäß Anspruch 22 zur Behandlung von Fettsucht, Diabetes, majorer Depression, Schizophrenie, Schlafstörungen und Angstzustand.

24. Verfahren zur Herstellung einer Verbindung gemäß Anspruch 1, worin Y ein 5-gliedriger Ring ist, der zwei Carbonylgruppen enthält, umfassend das Umsetzen eines Anilins der Formel (II): 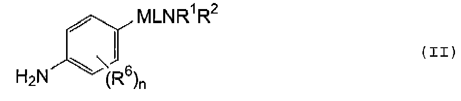 oder eines geschützten Derivats davon mit einem Anhydrid der Formel (III): 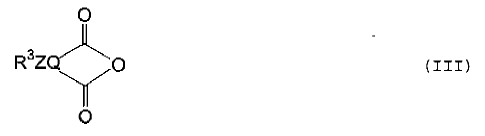 einer Orthodicarbonsäure der Formel (IV): 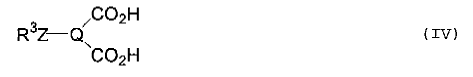 oder einem Imid der Formel (V): 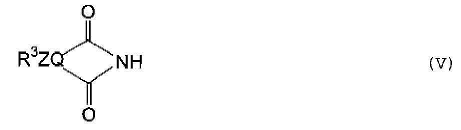 worin R¹, R², R³, R⁶, z, Q, Y, M und L wie in Formel (I) definiert sind.

25. Verfahren zur Herstellung einer Verbindung gemäß Anspruch 1, worin Y ein 5-gliedriger Ring ist, der eine einzelne Carbonylgruppe enthält, umfassend das Reduzieren einer Verbindung der Formel (I), worin Y ein 5-gliedriger Ring ist, der 2 Carbonylgruppen enthält.

26. Verfahren zur Herstellung einer Verbindung gemäß Anspruch 1, worin Y ein 6-gliedriger Heterocyclus ist, umfassend die Cyclisierung einer Verbindung der Formel (VI) : 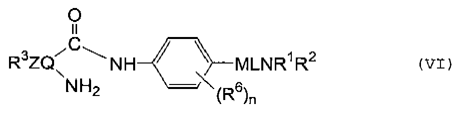 worin R³, Z, Q, R⁶, n, M, L, R¹ und R² die in Formel (I) definierten Bedeutungen haben.

27. Verfahren zur Herstellung einer Verbindung gemäß Anspruch 1, worin Y ein Pyrimidin-2,4-dion ist, umfassend das Umsetzen eines Amins der Formel (VI): 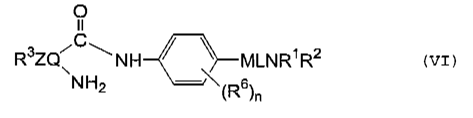 mit einem Carboxylierungsmittel und einer Base in einem aprotischen Lösungsmittel, worin R³, Z, Q, R⁶, n, M, L, R¹ und R² die in Formel (I) definierten Bedeutungen haben.

28. Verfahren zur Herstellung einer Verbindung gemäß Anspruch 1, worin Y 1,2,3-Triazin-4-on ist, umfassend die Diazotierung einer Verbindung der Formel (VI): 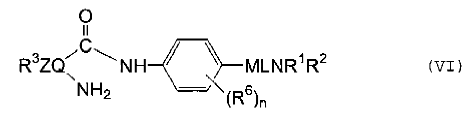 gefolgt von der Behandlung mit einer Base, worin R³, Z, Q, R⁶, n, M, L, R¹ und R² die in Formel (I) definierten Bedeutungen haben.

29. Verfahren zur Herstellung einer Verbindung gemäß Anspruch 1, worin Y ein Pyrimidinonring ist, der eine Thiocarbonylgruppe in der 2-Stellung enthält, umfassend das Umsetzen eines Thioharnstoffs der Formel (VII) 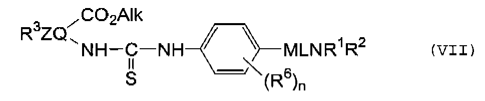 oder eines geschützten Derivats davon durch Erwärmen des Thioharnstoffs in einem hochsiedenden Lösungsmittel, worin R³, Z, Q, R⁶, n, M, L, R¹ und R² die in Formel (I) definierten Bedeutungen haben und Alk C₁₋₄-Alkyl ist.

30. Verfahren zur Herstellung einer Verbindung gemäß Anspruch 1, worin Y ein Pyrimidinon mit einer Alkylthiogruppe in der 2-Stellung ist, umfassend das Umsetzen eines Thioharnstoffs der Formel (VII) 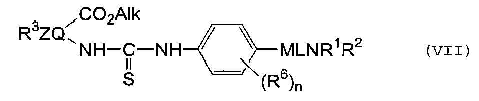 mit einem Alkylhalogenid in einem protischen polaren Lösungsmittel, worin R³, Z, Q, R⁶, n, M, L, R¹ und R² die in Formel (I) definierten Bedeutungen haben oder geschützte Derivate davon sind und Alk C₁₋₄-Alkyl ist.

31. Verfahren zur Herstellung einer Verbindung gemäß Anspruch 1, worin Y ein Pyrimidinonderivat ist, das in der 2-Stellung unsubstituiert ist, umfassend das Umsetzen eines entsprechenden Pyrimidinons mit einer Alkylthiogruppe in der 2-Stellung mit Raney-Nickel in einem Lösungsmittel.

32. Verfahren zur Herstellung einer Verbindung gemäß Anspruch 1, umfassend das Umsetzen einer Verbindung der Formel (VIII): 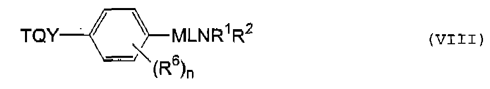 worin T eine Abgangstruppe ist, mit einer Verbindung, die die Gruppe R³Z einführen kann, worin Q, Y, R⁶, n, M, L, R¹ und R² die in Formel (I) definierten Bedeutungen haben.

33. Verfahren zur Herstellung einer Verbindung gemäß Anspruch 1, worin Z eine Bindung ist und R³ eine Arylgruppe ist, umfassend das Umsetzen einer Verbindung der Formel (VIII), worin T eine Abgangsgruppe ist: 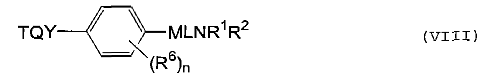 mit (i) einer Arylboronsäure in einer Suzuki-Kupplungsreaktion oder (ii) mit einem Arylzinn-Reagens in einer Stille-Substitutionsreaktion, worin Q, Y, R⁶, n, M, L, R¹ und R² die in Formel (I) definierten Bedeutungen haben.

34. Verfahren zur Herstellung einer Verbindung gemäß Anspruch 1, worin Z eine Methylengruppe ist, umfassend das Behandeln einer Verbindung der Formel (VIII), worin T eine Abgangsgruppe ist: 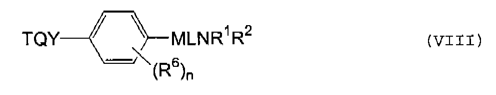 mit einer Organozink- oder Organomagnesium-Verbindung in Gegenwart eines Palladiumkatalysators, worin Q, Y, R⁶, n, M, L, R¹ und R² die in Formel (I) definierten Bedeutungen haben.

35. Verfahren zur Herstellung einer Verbindung gemäß Anspruch 1, worin Z NH oder NCH₃ ist, umfassend das Umsetzen einer Verbindung der Formel (VIII), worin T eine Abgangsgruppe ist: 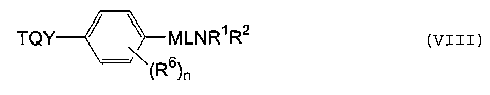 mit einem Amin in Gegenwart eines Palladiumkatalysators unter Standard-Buchwald-Bedingungen, worin Q, Y, R⁶, n, M, L, R¹ und R² die in Formel (I) definierten Bedeutungen haben.

36. Verfahren zur Herstellung einer Verbindung gemäß Anspruch 1, worin Z Schwefel ist, umfassend das Umsetzen einer Verbindung der Formel (VIII), worin T eine Abgangsgruppe ist: 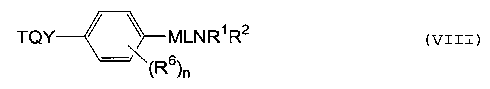 mit einem Thiol in Gegenwart einer Base, worin Q, Y, R⁶, n, M, L, R¹ und R² die in Formel (I) definierten Bedeutungen haben.

37. Verfahren zur Herstellung einer Verbindung gemäß Anspruch 1, worin Z Sauerstoff ist, umfassend das Umsetzen einer Verbindung der Formel (VIII), worin T eine Abgangsgruppe ist: 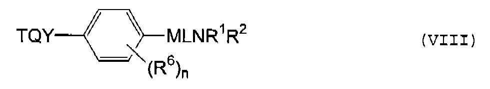 mit einem Alkohol in Gegenwart eines Alkalimetallhydrids und eines aprotischen Lösungsmittels, worin Q, Y, R⁶, n, M, L, R¹ und R² die in Formel (I) definierten Bedeutungen haben.

38. Verwendung einer Verbindung gemäß Anspruch 1 zur Herstellung eines Medikaments zur Inhibierung von MCHinduzierter Hyperglykämie durch Reduzierung von MCHinduzierter Hyperglukagonämie.

## Revendications

1. Composé de formule (I) comprenant : 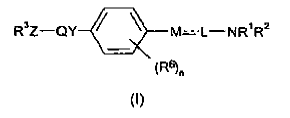 sel ou solvate de ceux-ci,
dans laquelle :
M est O ou CH₂ ;
L est une chaîne alkylène à 2 ou 3 chaînons qui peut être reliée au cycle phényle de la formule (I) par l'intermédiaire d'un substituant R⁶ situé en position ortho par rapport au groupe M pour former une structure bicyclique ;
où M-L ensemble peuvent être éventuellement substitués par au moins une partie choisie dans le groupe consistant en groupes méthyle, éthyle, hydroxy et alcoxy en C₁₋₃ ;
(i) R¹ et R² sont chacun indépendamment choisis dans le groupe consistant en atome d'hydrogène, groupe alkyle en C₁₋₆ à chaîne linéaire ou ramifiée qui peut être éventuellement substitué par un groupe phényle, et cycloalkyle en C₃₋₆ éventuellement substitué par un ou plusieurs groupes alkyles en C₁₋₆ ;
ou bien (ii) R¹ et R² forment ensemble avec l'atome d'azote auquel ils sont liés un noyau hétérocyclique de 4 à 8 chaînons ou un noyau hétérocyclique bicyclique de 7 à 10 chaînons contenant 1, 2, 3 ou 4 hétéroatomes choisis parmi N, S et O, où ledit noyau hétérocyclique de 4 à 8 chaînons et ledit noyau hétérocyclique bicyclique de 7 à 10 chaînons sont éventuellement substitués par un substituant choisi dans le groupe consistant en groupe phényle et de un à quatre groupes alkyles en C₁₋₃ ;
chaque R⁶ est identique ou différent et est indépendamment choisi dans le groupe consistant en groupe hydroxy, alkyle en C₁₋₂, alcoxy en C₁₋₃, halo, alcényle en C₂₋₃, benzyle et -C(R^{a})NOR^{b}, dans lequel R^{a} et R^{b} sont chacun indépendamment choisis dans le groupe consistant en atome d'hydrogène, groupe méthyle, méthoxyméthyle, méthoxyméthoxy et méthoxyéthoxy, et n est 1, 2, 3 ou 4 ;
QY est un noyau hétérocyclique condensé bicyclique dans lequel Q et Y sont chacun un cycle dudit groupe hétérocyclique condensé bicyclique, où ledit cycle Y contient de 1 à 3 atomes d'azote et est relié au noyau phényle de la formule (I) par l'intermédiaire d'un atome d'azote, et ledit cycle Q est un noyau aryle ou hétérocyclique de 5 ou 6 chaînons ayant un groupe ZR³; Z est relié au cycle Q ;
Z est choisi dans le groupe consistant en une liaison directe, NH, NCH₃, O, S et CH₂ ; et
R³ est un groupe choisi dans le groupe consistant en groupes aryle, alc-2-èn-1-yle, cycloalkyle et cycloalc-2-én-1-yle et où lesdits groupes aryle, alc-2-én-1-yle, cycloalkyle et cycloalc-2-én-1-yle sont éventuellement substitués une à trois fois par un substituant choisi dans le groupe consistant en groupes alkyle en C₁₋₃, halo, amino, alkylamino, dialkylamino, hydroxy, alcoxy en C₁₋₃, cyano, trifluorométhyle et méthylthio.

2. Composé selon la revendication 1, dans lequel M est O.

3. Composé selon les revendications 1 à 2, dans lequel L est (CH₂)₂.

4. Composé selon les revendications 1 à 3, dans lequel L est relié au cycle phényle de la formule (I) par l'intermédiaire d'un substituant R⁶ situé en position ortho par rapport au groupe M pour former une structure bicyclique.

5. Composé selon la revendication 4, dans lequel ladite structure bicyclique est choisie dans le groupe consistant en 1,4-benzodioxane, benzopyrane, 1,2-dihydrobenzopyrane, 1,2,3,4-tétrahydronaphtalène et 1,2-dihydronaphtalène.

6. Composé selon les revendications 1 à 5, dans lequel (i) R¹ et R² sont indépendamment un groupe alkyle en C₁₋₆.

7. Composé selon la revendication 6, dans lequel R¹ et R² sont indépendamment un groupe méthyle et isopropyle.

8. Composé selon les revendications 1 à 5, dans lequel (ii) R¹ et R² forment ensemble avec l'atome d'azote auquel ils sont liés un noyau hétérocyclique de 4 à 8 chaînons.

9. Composé selon la revendication 8, dans lequel NR¹R² représente un groupe hétérocyclique de 5 ou 6 chaînons éventuellement substitué par 1 à 4 groupes méthyle ou un groupe phényle.

10. Composé selon la revendication 8 ou 9, dans lequel NR¹R² représente un groupe pyrrolidinyle.

11. Composé selon les revendications 1 à 10, dans lequel QY est un noyau hétérocyclique condensé bicyclique 5,5 ; 5,6 ; 6,5 ; ou 6,6.

12. Composé selon la revendication 11, dans lequel QY est un groupe choisi parmi l'isoindole-1,3-dione, la 2,3-dihydro-isoindole-1-one, la 3-H-benzo[d](1,2,3)triazin-4-one, la 3-H-quinazolin-4-one, la 1-H-quinazoline-2,4-dione, la 3-H-thiéno[2,3-d]-1-triazine-4-one, la 3-H-thiéno[2,3-d]pyrimidin-4-one, la 3-H-thiéno[3,2-d]pyrimidin-4-one, la 2-thioxo-2,3-dihydro-1-H-thiéno[3,2-d]pyrimidine-4-one, la 2-méthylthio-3H-thiéno[3,2-d]pyrimidin-4-one.

13. Composé selon les revendications 1 à 12, dans lequel l'un au moins des R⁶ est en position ortho par rapport au groupe M.

14. Composé selon les revendications 1 à 13, dans lequel R⁶ est un groupe méthoxy.

15. Composé selon les revendications 1 à 14, dans lequel Z est une liaison ou un atome d'oxygène.

16. Composé selon les revendications 1 à 15, dans lequel R³ est choisi dans le groupe consistant en (i) phényle qui est éventuellement substitué par un atome d'halogène, un groupe hydroxy, alcoxy en C₁₋₃, alkyle en C₁₋₄, cyano ou trifluorométhyle, (ii) cycloalkyle en C₅₋₇, (iii) 2-cycloalcényle en C₅₋₇, et (iv) alc-2-én-1-yle en C₃₋₆.

17. Composé selon les revendications 1 à 16, dans lequel R³ représente un groupe phényle.

18. 3-[3-Méthoxy-4-(2-pyrrolidin-1-yl-éthyl)-phényl]-7-phényl-3H-benzo[d][1,2,3]triazin-4-one et ses sels d'addition d'acide acceptables du point de vue physiologique.

19. 3-[3-Méthoxy-4-(2-pyrrolidin-1-yl-éthoxy)-phényl]-6-phényl-3H-thiéno[3,2-d]pyrimidin-4-one et ses sels d'addition d'acide acceptables du point de vue physiologique.

20. Composition pharmaceutique comprenant un composé selon les revendications 1 à 19 ou un sel ou un solvate de ceux-ci acceptables du point de vue physiologique et un ou plusieurs supports ou excipients acceptables du point de vue pharmaceutique,

21. Composé selon les revendications 1 à 19 ou sel ou solvate de ceux-ci acceptables du point de vue physiologique utiles en thérapie.

22. Utilisation d'un composé selon les revendications 1 à 19, dans la fabrication d'un agent thérapeutique destiné au traitement d'une ou de plusieurs des infections bactériennes, fongiques, à protozoaires et virales, d'une infection provoquée par le VIH-1 ou le VIH-2 ; de douleurs ; de cancers ; de diabètes ; de l'obésité ; d'anomalies de l'alimentation comme l'anorexie et la boulimie ; d'anomalies de la consommation de boisson ; d'asthme ; de la maladie de Parkinson ; d'insuffisances cardiaques aiguë et congestive ; d'hypertension ; de rétention urinaire ; d'ostéoporose ; d'angine de poitrine ; d'infarctus du myocarde ; d'ulcères ; d'allergies ; d'hypertrophie prostatique bénigne ; de troubles psychotiques et neurologiques, incluant l'anxiété, la schizophrénie, la dépression (majeure), la dépression bipolaire (maniaco-dépressive), le délire, la démence et/ou le retard mental sévère ; et de dyskinésie (comme la maladie de Huntington ou le syndrome de Gilles de la Tourette).

23. Utilisation selon la revendication 22, pour le traitement de l'obésité, du diabète, d'une dépression majeure, de schizophrénie, de troubles du sommeil et d'anxiété.

24. Procédé de préparation d'un composé selon la revendication 1, dans lequel Y est un cycle à 5 chaînons contenant deux groupes carbonyles comprenant la réaction d'une aniline de formule (II) : 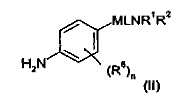 ou d'un dérivé protégé de celle-ci, avec un anhydride de formule (III) : 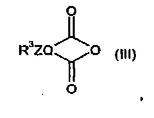 un acide orthodicarboxylique de formule (IV) : 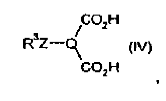 ou un imide de formule (V) : 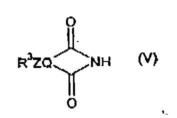 dans lesquelles R¹, R², R³, R⁶, Z, Q, Y, M et L sont tels que définis dans la formule (I).

25. Procédé de préparation d'un composé selon la revendication 1, dans lequel Y est un cycle à 5 chaînons contenant un unique groupe carbonyle, comprenant la réduction d'un composé de formule (I) dans laquelle Y est un cycle à 5 chaînons contenant deux groupes carbonyles.

26. Procédé de préparation d'un composé selon la revendication 1, dans lequel Y est un hétérocycle à 6 chaînons, comprenant la cyclisation d'un composé de formule (VI) : 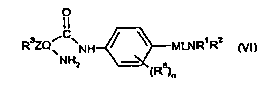 dans laquelle R³, Z, Q, R⁶, n, M, L, R¹ et R² ont les significations définies dans la formule (I).

27. Procédé de préparation d'un composé selon la revendication 1, dans lequel Y est une pyrimidine-2,4-dione, comprenant la réaction d'une amine de formule (VI) : 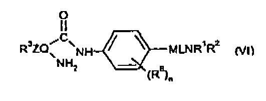 avec un agent de carboxylation et une base dans un solvant aprotique et dans laquelle R³, Z, Q, R⁶, n, M, L, R¹ et R² ont les significations définies dans la formule (I).

28. Procédé de préparation d'un composé selon la revendication 1, dans lequel Y est une 1,2,3-triazin-4-one, comprenant la diazotation d'un composé de formule (VI) : 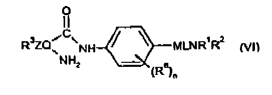 suivie d'un traitement avec une base, et dans laquelle R³, Z, Q, R⁶, n, M, L, R¹ et R² ont les significations définies dans la formule (I).

29. Procédé de préparation d'un composé selon la revendication 1, dans lequel Y est un cycle pyrimidinone contenant un groupe thiocarbonyle en position 2, comprenant la réaction d'une thiourée de formule (VII) : 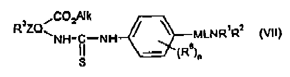 ou d'un dérivé protégé de celle-ci, par chauffage de ladite thiourée dans un solvant à haut point d'ébullition, et dans laquelle R³, Z, Q, R⁶, n, M, L, R¹ et R² ont les significations définies dans la formule (I) et Alk est un groupe alkyle en C₁₋₄.

30. Procédé de préparation d'un composé selon la revendication 1, dans lequel Y est une pyrimidinone ayant un groupe alkylthio en position 2, comprenant la réaction d'une thiourée de formule (VII) : 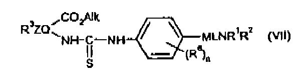 avec un halogénure d'alkyle dans un solvant polaire protique, et dans laquelle R³, Z, Q, R⁶, n, M, L, R¹ et R² ont les significations définies dans la formule (I) ou sont des dérivés protégés de ceux-ci et Alk est un groupe alkyle en C₁₋₄.

31. Procédé de préparation d'un composé selon la revendication 1, dans lequel Y est un dérivé de pyridimidinone non substitué en position 2, comprenant la réaction d'une pyrimidinone correspondante ayant un groupe alkylthio en position 2 avec du nickel Raney dans un solvant.

32. Procédé de préparation d'un composé selon la revendication 1, comprenant la réaction d'un composé de formule (VIII) : 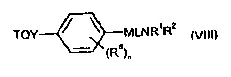 dans laquelle T est un groupe mobile, avec un composé capable d'introduire le groupe R³Z, et dans laquelle Q, Y, R⁶, n, M, L, R¹ et R² ont les significations définies dans la formule (I).

33. Procédé de préparation d'un composé selon la revendication 1, dans lequel Z est une liaison et R³ est un groupe aryle, comprenant la réaction d'un composé de formule (VIII) dans laquelle T est un groupe mobile 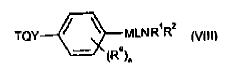 avec (i) un acide arylboronique dans une réaction de couplage de Suzuki, ou avec (ii) un réactif aryl-étain dans une réaction de déplacement de Stille dans laquelle Q, Y, R⁶, n, M, L, R¹ et R² ont les significations définies dans la formule (I).

34. Procédé de préparation d'un composé selon la revendication 1, dans lequel Z est un groupe méthylène, comprenant le traitement d'un composé de formule (VIII) dans laquelle T est un groupe mobile : 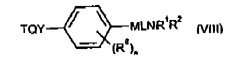 avec un composé organozincique ou organomagnésien en présence d'un catalyseur au palladium, et dans laquelle Q, Y, R⁶, n, M, L, R¹ et R² ont les significations définies dans la formule (I).

35. Procédé de préparation d'un composé selon la revendication 1, dans lequel Z est NH ou NCH₃, comprenant la réaction d'un composé de formule (VIII) dans laquelle T est un groupe mobile : 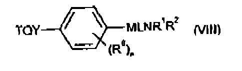 avec une amine en présence d'un catalyseur au palladium dans des conditions de Buchwald standard, et dans laquelle Q, Y, R⁶, n, M, L, R¹ et R² ont les significations définies dans la formule (I).

36. Procédé de préparation d'un composé selon la revendication 1, dans lequel Z est un atome de soufre, comprenant la réaction d'un composé de formule (VIII) dans laquelle T est un groupe mobile : 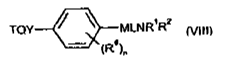 avec un thiol en présence d'une base, et dans laquelle Q, Y, R⁶, n, M, L, R¹ et R² ont les significations définies dans la formule (I).

37. Procédé de préparation d'un composé selon la revendication 1, dans lequel Z est un atome d'oxygène, comprenant la réaction d'un composé de formule (VIII) dans laquelle T est un groupe mobile : 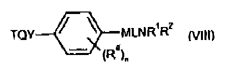 avec un alcool en présence d'un hydrure de métal alcalin et d'un solvant aprotique, et dans laquelle Q, Y, R⁶, n, M, L, R¹ et R² ont les significations définies dans la formule (I).

38. Utilisation d'un composé selon la revendication 1 pour la fabrication d'un médicament destiné à inhiber une hyperglycémie induite par la MCH par réduction de l'hyperglucagonémie induite par la MCH.
